# EUROPÄISCHE PATENTSCHRIFT

(11) **EP 0 629 667 B1**
(45) Veröffentlichungstag und Bekanntmachung des Hinweises auf die Patenterteilung: **25.02.1998**
(21) Anmeldenummer: 94106854.6
(22) Anmeldetag: 02.05.1994
(51) Int. Cl.: C09B 62/503, C09B 62/04, C07C 317/28

(54) **Wasserlöslichse faserreaktive Farbstoffe, Verfahren zu ihrer Herstellung und ihre Verwendung**
Water-soluble fiber-reactive dyes, process for their preparation and the use thereof
Colorants réactifs sur la fibre, solubles dans l'eau, procédé pour leur préparation et leur utilisation

(30) Priorität: 18.05.1993 DE 4316539; 12.08.1993 DE 4327055; 28.01.1994 DE 4402209
(43) Veröffentlichungstag der Anmeldung: 21.12.1994
(73) Patentinhaber: HOECHST AKTIENGESELLSCHAFT, 65929 Frankfurt am Main (DE)
(72) Erfinder: Russ, Hubert Werner, Dr., D-65439 Flörsheim/Main (DE); Tappe, Horst, Dr., D-63128 Dietzenbach (DE); Schumacher, Christian, Dr., D-65929 Frankfurt am Main (DE)

(56) Entgegenhaltungen:
- EP-A- 0 070 806
- EP-A- 0 070 807
- EP-A- 0 298 041
- FR-A- 2 346 418
- GB-A- 2 259 710

## Beschreibung

Die Erfindung liegt auf dem technischen Gebiet der faserreaktiven Farbstoffe.

Die Praxis des Färbens mit faserreaktiven Farbstoffen hat in neuerer Zeit zu erhöhten Anforderungen an die Qualität der Färbungen und die Wirtschaftlichkeit des Färbeprozesses geführt. Infolge dessen besteht weiterhin ein Bedarf nach neuen faserreaktiven Farbstoffen, die verbesserte Eigenschaften, nicht nur in bezug auf die Echtheiten, sondern auch einen hohen Fixiergrad auf dem zu färbenden Material aufweisen. So sind aus den europäischen Patentanmeldungs-Veröffentlichungen Nrs. 0 070 806, 0 070 807, 0 374 758 und 0 499 588 und aus den US-Patentschriften Nrs. 4 908 436 und 5 138 041 faserreaktive Farbstoffe bekannt, die einen Monohalogen-s-triazinylamino-Rest enthalten, der mittels der Aminogruppe und einem aliphatischen Brückenglied mit einer faserreaktiven Gruppe der Vinylsulfonreihe verbunden ist. Diese bekannten Farbstoffe sind wegen der angesprochenen gestiegenen Anforderungen insbesondere hinsichtlich ihres Fixiergrades und der Farbstärke der mit ihnen erhältlichen Färbungen und Drucke verbesserungswürdig. Die EP-A-0 298 041 und GB-A-2 259 710 beschreiben dagegen Farbstoffe mit einem Pyrimidinrest.

Mit der vorliegenden Erfindung wurden neue faserreaktive Farbstoffe der nachstehend definierten allgemeinen Formel (1)

F - Zₙ (1)

gefunden, die sich gegenüber den bekannten Farbstoffen vorteilhaft unterscheiden und Färbungen und Drucke mit hoher Farbstärke liefern.
In der Formel (1) bedeuten:
- F: ist der Rest eines sulfogruppenhaltigen Mono-, Dis- oder Polyazofarbstoffes, wie Trisazofarbstoffes, oder eines Schwermetallkomplex-Mono-, -Dis- oder -Trisazofarbstoffes oder eines Anthrachinon-, Azomethin-, Phenazin-, Stilben-, Triphenylmethan-, Xanthen-, Thioxanthen-, Nitroaryl-, Naphthochinon-, Pyrenchinon-, Perylentetracarbimid-, Formazan-, Kupferformazan-, Phthalocyanin-, Kupferphthalocyanin-, Nickelphthalocyanin- oder Kobaltphthalocyanin-Farbstoffes oder Triphendioxazin-Farbstoffes;
- Z: ist eine Gruppe der allgemeinen Formel (2) 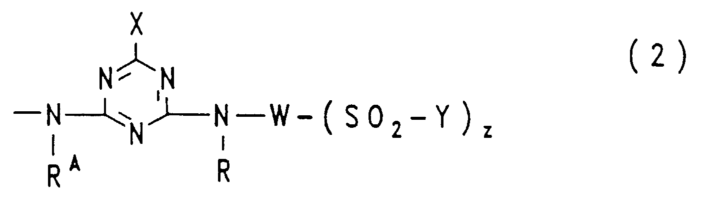 in welcher
R^{A} Wasserstoff oder Alkyl von 1 bis 4 C-Atomen, wie Methyl und Ethyl ist, das durch Halogen, wie Chlor und Brom, Hydroxy, Cyano, Alkoxy von 1 bis 4 C-Atomen, wie Methoxy und Ethoxy, Alkoxycarbonyl von 2 bis 5 C-Atomen, wie Carbethoxy und Carbomethoxy, Carboxy, Sulfamoyl, Sulfo, Sulfato oder Phosphato substituiert sein kann, und bevorzugt Methyl oder Ethyl und insbesondere bevorzugt Wasserstoff ist,
X Halogen, wie Fluor und Chlor, Cyanoamino, Methylsulfonylamino, Ethylsulfonylamino, Carboxy-pyridinyl, Aminocarbonyl-pyridinyl oder ein Rest der Formel -N(R)-W-(SO₂-Y)_{z} mit R, W, Y und z einer der nachstehend angegebenen Bedeutungen ist,
R Phenyl ist, das durch 1, 2 oder 3, bevorzugt 1 oder 2, Substituenten aus der Gruppe Halogen, wie Chlor und Brom, Alkyl von 1 bis 4 C-Atomen, wie Methyl, Alkoxy von 1 bis 4 C-Atomen, wie Methoxy und Ethoxy, Sulfo und Carboxy, bevorzugt durch Methyl, Methoxy oder Sulfo, substituiert sein kann,
W Alkylen von 3 bis 8 C-Atomen, bevorzugt von 3 bis 6 C-Atomen, ist, insbesondere bevorzugt n-Propylen ist, das durch 1 oder 2 Substituenten, bevorzugt 1 Substituenten, aus der Gruppe Alkoxy von 1 bis 6 C-Atomen, bevorzugt 1 bis 4 C-Atomen, Halogen, wie Chlor und Brom, Alkoxycarbonyl von 2 bis 5 C-Atomen, wie Carbomethoxy und Carbethoxy, Carboxy, Sulfo und Sulfato oder durch einen Phenylrest oder einen durch 1 oder 2 Substituenten aus der Gruppe Sulfo, Carboxy, Methoxy, Ethoxy und Methyl substituierten Phenylrest oder durch solch einen gegebenenfalls substituierten Phenylrest und eine Sulfo-, Sulfato- oder Carboxygruppe oder durch einen heterocyclischen Rest substituiert sein kann,
Y Vinyl ist oder β-Sulfatoethyl, β-Thiosulfatoethyl, β-Phosphatoethyl, β-(C₂-C₅-Alkanoyloxy)-ethyl, wie β-Acetyloxyethyl, β-Benzoyloxyethyl, β-(Sulfobenzoyloxy)-ethyl oder β-(p-Toluolsulfonyloxy)-ethyl und bevorzugt Vinyl und insbesondere bevorzugt β-Sulfatoethyl ist und
z die Zahl 1 oder 2 ist, bevorzugt 1 ist;
- n: ist die Zahl 1 oder 2, bevorzugt 1.

Bevorzugt ist F der Rest eines Mono- oder Disazofarbstoffes oder der Rest eines Schwermetallkomplex-Azofarbstoffes, wie eines 1:2-Chromkomplex-, 1:2-Kobaltkomplex- und insbesondere eines o,o'-1:1-Kupferkomplex-Monoazo- oder -Disazofarbstoffes, oder der Rest eines Anthrachinon- oder Kupferformazan- oder eines Nickel- oder Kupferphthalocyaninfarbstoffes oder Triphendioxazinfarbstoffes.

Der Farbstoffrest F besitzt eine oder mehrere, wie 2 bis 6, Sulfogruppen. Der Rest F kann weitere bei organischen Farbstoffen übliche Substituenten enthalten. Solche Substituenten sind beispielsweise: Alkylgruppen von 1 bis 4 C-Atomen, wie Methyl, Ethyl, Propyl, Isopropyl oder Butyl, hiervon bevorzugt Ethyl und insbesondere Methyl; Alkoxygruppen von 1 bis 4 C-Atomen, wie Methoxy, Ethoxy, Propoxy, Isopropoxy und Butoxy, bevorzugt hiervon Ethoxy und insbesondere Methoxy; Alkanoylaminogruppen von 2 bis 5 C-Atomen, wie die Acetylamino- und Propionylaminogruppe; gegebenenfalls durch Sulfo, Carboxy, Methyl, Ethyl, Methoxy, Ethoxy und/oder Chlor substituierte Benzoylaminogruppen; primäre und mono- oder disubstituierte Aminogruppen, wobei die Substituenten beispielsweise Alkylgruppen von 1 bis 4 C-Atomen und/oder Phenylgruppen sind, wie Monoalkylamino- und Dialkylaminogruppen mit 1 bis 4 C-Atomen im Alkylrest, Phenylamino- oder N-(C₁-C₄-Alkyl)-N-phenylamino-Gruppen, wobei die Alkylreste noch substituiert sein können, beispielsweise durch Phenyl, Sulfophenyl, Hydroxy, Sulfato, Sulfo und Carboxy, und die Phenylgruppen noch substituiert sein können, wie durch Chlor, Sulfo, Carboxy, Methyl und/oder Methoxy, so beispielsweise Methylamino-, Ethylamino-, Propylamino-, Isopropylamino-, Butylamino-, N,N-Di-(β-hydroxyethyl)-amino, N,N-Di-(β-sulfatoethyl)-amino-, Sulfobenzylamino-, N,N-Di-(sulfobenzyl)-amino- und Diethylaminogruppen sowie Phenylamino- und Sulfophenylaminogruppen; Alkoxycarbonylgruppen mit einem Alkylrest von 1 bis 4 C-Atomen, wie Methoxycarbonyl und Ethoxycarbonyl; Alkylsulfonylgruppen von 1 bis 4 C-Atomen, wie Methylsulfonyl und Ethylsulfonyl; Trifluormethyl-, Nitro- und Cyanogruppen; Halogenatome, wie Fluor, Chlor und Brom; Carbamoylgruppen, die durch Alkyl von 1 bis 4 C-Atomen mono- und disubstituiert sein können, wobei die Alkylreste wiederum substituiert sein können, beispielsweise durch Hydroxy, Sulfato, Sulfo, Carboxy, Phenyl und Sulfophenyl, wie beispielsweise N-Methyl-carbamoyl und N-Ethyl-carbamoyl; Sulfamoylgruppen, die durch Alkylgruppen von 1 bis 4 C-Atomen mono- oder disubstituiert sein können, und N-Phenyl-N-alkyl-sulfamoylgruppen mit einer Alkylgruppe von 1 bis 4 C-Atomen, wobei diese Alkylgruppen wiederum durch Hydroxy, Sulfato, Sulfo, Carboxy, Phenyl und Sulfophenyl substituiert sein können, wie beispielsweise N-Methyl-sulfamoyl, N-Ethyl-sulfamoyl, N-Propyl-sulfamoyl, N-Isopropyl-sulfamoyl, N-Butyl-sulfamoyl, N-(β-Hydroxyethyl)-sulfamoyl und N,N-Di-(β-hydroxyethyl)-sulfamoyl; N-Phenyl-sulfamoyl-, Ureido-, Hydroxy-, Carboxy-, Sulfomethyl- und Sulfogruppen. Der Farbstoffrest F kann weiterhin eine oder zwei faserreaktive Gruppen der allgemeinen Formel Y-SO₂-W^{o}- besitzen, in welcher W^{o} einen Alkylenrest von 1 bis 4 C-Atomen oder eine Aminogruppe der Formel -N(CH₃)- oder -N(C₂H₅)- oder eine Gruppe der Formel -(C₂-C₄-alkylen)-NH- bedeutet oder für eine direkte kovalente Bindung steht.

In den allgemeinen Formeln (1) und (2) sowie in den nachfolgenden allgemeinen Formeln können die einzelnen Formelglieder, sowohl verschiedener als auch gleicher Bezeichnung innerhalb einer allgemeinen Formel, im Rahmen ihrer Bedeutung zueinander gleiche oder voneinander verschiedene Bedeutungen haben.

Die Gruppen "Sulfo", "Thiosulfato", "Carboxy", "Phosphato" und "Sulfato" schließen sowohl deren Säureform als auch deren Salzform ein. Demgemäß bedeuten Sulfogruppen Gruppen entsprechend der allgemeinen Formel -SO₃M , Thiosulfatogruppen Gruppen entsprechend der allgemeinen Formel -S-SO₃M , Carboxygruppen Gruppen entsprechend der allgemeinen Formel -COOM, Phosphatogruppen Gruppen entsprechend der allgemeinen Formel -OPO₃M₂ und Sulfatogruppen Gruppen entsprechend der allgemeinen Formel -OSO₃M , in welchen
- M: Wasserstoff oder ein Alkalimetall, wie Natrium, Kalium oder Lithium, oder das Moläquivalent eines Erdalkalimetalls, wie des Calciums, und bevorzugt Wasserstoff oder ein Alkalimetall ist.

Wichtige Azofarbstoffe entsprechend der allgemeinen Formel (1) sind solche, in denen F den Rest eines Farbstoffes der Benzol-azo-naphthol-, der Benzol-azo-1-phenyl-5-pyrazolon-, der Benzol-azo-benzol-, der Naphthalin-azo-benzol-, der Benzol-azo-aminonaphthalin-, der Naphthalin-azo-naphthalin-, der Naphthalin-azo-1-phenyl-5-pyrazolon-, der Benzol-azo-pyridon- und der Naphthalin-azo-pyridon-Reihe bedeutet, wobei auch hier die sulfogruppenhaltigen Farbstoffe bevorzugt sind. Von den erfindungsgemäßen 1:1-Kupferkomplex-Azofarbstoffen sind diejenigen der Benzol- und Naphthalinreihe bevorzugt.

Bevorzugte Mono- und Disazofarbstoffe der allgemeinen Formel (1) sind beispielsweise solche der allgemeinen Formeln (3a), (3b) und (3c)

D¹ - N = N - (E - N = N)ᵥ - K² - Z (3a)

Z - D² - N = N - (E - N = N)ᵥ - K¹ (3b)

Z - D² - N = N - (E - N = N)ᵥ - K² - Z (3c)

und die davon abgeleiteten Schwermetallkomplex-Verbindungen, wie 1:1-Kupferkomplex-Verbindungen, in welchen
- D¹: der Rest einer Diazokomponente der Benzol- oder Naphthalinreihe ist,
- D²: der Rest einer Diazokomponente der Diaminobenzol- oder Diaminonaphthalin-Reihe ist,
- E: der Rest einer Mittelkomponente der Benzol- oder Naphthalinreihe bedeutet,
- K¹: der Rest einer Kupplungskomponente der Benzol-, Naphthalin-, Pyrazolon-, 6-Hydroxypyridon(2)- oder Acetessigsäurearylamid-Reihe ist,
- K²: der bivalente Rest einer Kupplungskomponente der Anilin-, Aminonaphthalin-, Acetessigsäure-(aminoaryl)-amid- oder 1-Aminophenyl-pyrazolon-Reihe ist,
wobei D¹, D², E, K¹ und K² einen oder mehrere der für F genannten Substituenten enthalten können, wobei die Reste D¹, D², E, K¹ und K² zusammen mindestens eine, bevorzugt mindestens zwei, Sulfogruppen besitzen,
- v: die Zahl Null oder 1 ist und
- Z: eine Gruppe der allgemeinen Formel (2) obiger Bedeutung ist.

Bevorzugt sind weiterhin Disazofarbstoffe der allgemeinen Formel (3d) und (3e)

D¹ - N = N - K^{o} - N = N - D² - Z (3d)

Z - D² - N = N - K^{o} - N = N - D² - Z (3e)

in welchen Z, D¹ und D² die obengenannten Bedeutungen haben und K^{o} den Rest einer bivalenten Kupplungskomponente der Naphthalinreihe darstellt, wobei D¹, D² und K^{o} die für F genannten Substituenten enthalten können, wobei D¹, D² und K^{o} zusammen mindestens eine Sulfogruppe enthalten.

Azofarbstoffe der allgemeinen Formeln (3d) und (3e) sind insbesondere Farbstoffe der allgemeinen Formel (3f) 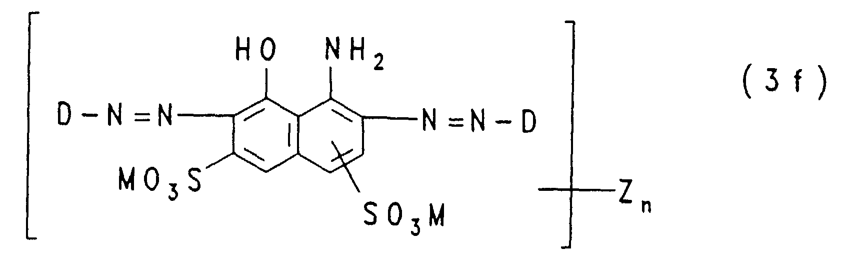 in welcher
- n: die obengenannte Bedeutung besitzt,
- M: für Wasserstoff oder Alkalimetall, wie Natrium, Kalium oder Lithium, oder das Moläquivalent eines Erdalkalimetalls, wie des Calciums, bedeutet,
- Z: für einen Rest der allgemeinen Formel (2) steht, der im Falle von n gleich 1 an einem der Reste D steht und im Falle von n gleich 2 an beide D gebunden ist, und
- D: jedes die Bedeutung von D¹ oder D² in Abhängigkeit davon, ob an D der Rest Z gebunden ist, hat.

Von den 1:1-Kupferkomplex-Azofarbstoffen sind beispielsweise solche der allgemeinen Formel (3g) und (3h) 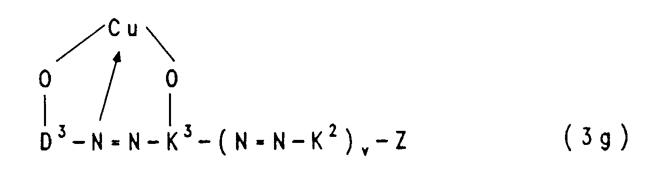 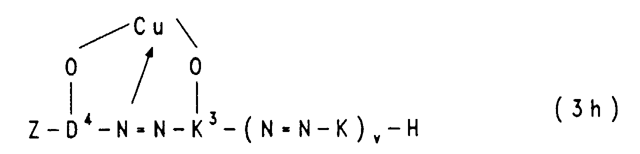 hervorzuheben, in welchen
- Z, K² und v: die obengenannten Bedeutungen haben,
- D⁴: für den Rest einer Diazokomponente steht, die in ortho-Stellung zur Azogruppe die kupferkomplexbindende Oxygruppe enthält, und
- K³: für den Rest einer Kupplungskomponente steht, die in ortho-Stellung oder vicinaler Stellung zur Azogruppe die kupferkomplexbindende Oxygruppe besitzt, und
- K: den bivalenten Rest einer Kupplungskomponente bedeutet.

Aromatische Reste von Diazokomponenten, die keine faserreaktive Gruppe der Formel (2) tragen, wie von solchen, die den allgemeinen Formeln D¹-NH₂ bzw. D³-NH₂ entsprechen, sind beispielsweise solche der allgemeinen Formeln (4a), (4b), (5a) und (5b) 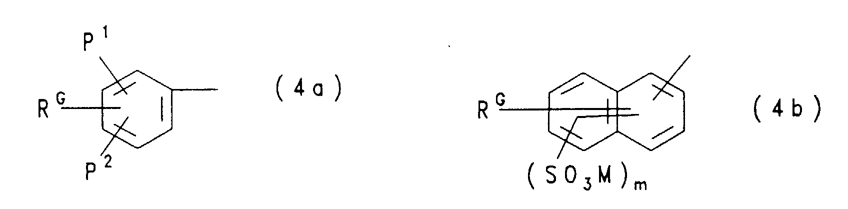 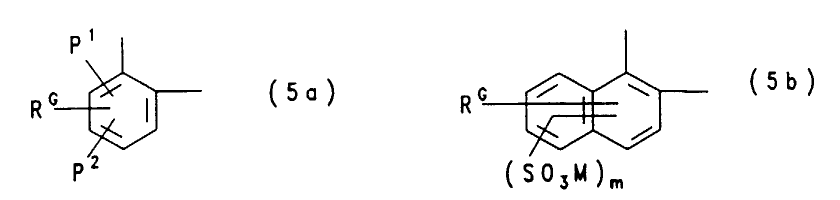 in welchen
- R^{G}: Wasserstoff, Sulfo oder eine Gruppe der Formel Y-SO₂-W^{o}- mit W^{o} und Y der obigen Bedeutung ist,
- P¹: Wasserstoff, Methyl, Ethyl, Methoxy, Ethoxy, Alkanoyl von 2 bis 5 C-Atomen, wie Acetyl und Propionyl, Cyano, Sulfo, Carboxy, Alkoxycarbonyl von 2 bis 5 C-Atomen, wie Methoxycarbonyl und Ethoxycarbonyl, Carbamoyl, N-(C₁-C₄-Alkyl)-carbamoyl, Fluor, Chlor, Brom oder Trifluormethyl ist,
- P²: Wasserstoff, Methyl, Ethyl, Methoxy, Ethoxy, Cyano, Nitro, Carboxy, Sulfo, Chlor, Alkanoylamino von 2 bis 5 C-Atomen, wie Acetylamino, Alkoxycarbonyl von 2 bis 5 C-Atomen, wie Methoxycarbonyl und Ethoxycarbonyl, Carbamoyl, N-(C₁-C₄-Alkyl)-sulfamoyl, Sulfophenylamidocarbonyl, Phenylamidocarbonyl, Alkylsulfonyl von 1 bis 4 C-Atomen, Phenylsulfonyl oder Phenoxy ist,
wobei der Benzolkern in Formel (4a) und (4b) zusätzlich in ortho-Stellung zur freien Bindung, die an die Azogruppe führt, eine Hydroxygruppe enthalten kann,
- m: die Zahl Null, 1 oder 2 bedeutet (wobei diese Gruppe im Falle von m gleich Null ein Wasserstoffatom bedeutet) und
- M: die obengenannte Bedeutung hat.

Bevorzugt ist hiervon P¹ gleich Wasserstoff, Methyl, Methoxy, Brom, Chlor, Sulfo und Carboxy sowie P² gleich Wasserstoff, Methyl, Methoxy, Chlor, Carboxy, Sulfo und Acetylamino.

Gruppen der allgemeinen Formeln (4a) und (4b) sind beispielsweise:
Phenyl, 2-Methyl-phenyl, 3- und 4-Methyl-phenyl, 2-Methoxy-phenyl, 3- und 4-Methoxy-phenyl, 2-Chlor-phenyl, 3- und 4-Chlor-phenyl, 2,5-Dichlor-phenyl, 2,5-Dimethyl-phenyl, 2-Methoxy-5-methyl-phenyl, 2-Methoxy-4-nitro-phenyl, 4-Phenyl-phen-1-yl, 3-Phenoxy-phenyl, 2-Sulfamoyl-phenyl, 3- und 4-Sulfamoyl-phenyl, 2-, 3- und 4-(N-Methyl-sulfamoyl)-, -(N-Ethyl-sulfamoyl)-, -(N,N-Dimethyl-sulfamoyl)- und -(N,N-Diethyl-sulfamoyl)-phenyl, 2-Sulfo-5-trifluormethyl-phenyl, 2-Nitro-phenyl, 3- und 4-Nitro-phenyl, 3-Acetylamino-phenyl, 4-Acetylamino-phenyl, 2-Carboxy-phenyl, 4-Carboxy-phenyl, 3-Carboxy-phenyl, 3-Chlor-6-carboxy-phenyl, 2-Sulfo-phenyl, 3-Sulfo-phenyl, 4-Sulfo-phenyl, 2,5-Disulfo-phenyl, 2,4-Disulfo-phenyl, 3,5-Disulfo-phenyl, 2-Methyl-5-sulfo-phenyl, 2-Methoxy-5-sulfo-phenyl, 2-Methoxy-4-sulfo-phenyl, 2-Sulfo-5-methyl-phenyl, 2-Methyl-4-sulfo-phenyl, 3-Sulfo-4-methoxy-phenyl, 5-Sulfo-2-ethoxy-phenyl, 4-Sulfo-2-ethoxy-phenyl, 2-Carboxy-5-sulfo-phenyl, 2-Carboxy-4-sulfo-phenyl, 2,5-Dimethoxy-4-sulfo-phenyl, 2,4-Dimethoxy-5-sulfo-phenyl, 2-Methoxy-5-methyl-4-sulfo-phenyl, 2-Sulfo-4-methoxy-phenyl, 2-Sulfo-4-methyl-phenyl, 2-Methyl-4-sulfo-phenyl, 2-Chlor-4-sulfo-phenyl, 2-Chlor-5-sulfo-phenyl, 2-Brom-4-sulfo-phenyl, 2,6-Dichlor-4-sulfo-phenyl, 2-Sulfo-4- und -5-chlor-phenyl, 2-Sulfo-4,5-dichlor-phenyl, 2,5-Dichlor-6-sulfo-phenyl, 2,5-Dichlor-4-sulfo-phenyl, 2-Sulfo-5-chlor-4-methyl-phenyl, 2-Sulfo-4-chlor-5-methyl-phenyl, 2-Sulfo-5-methoxy-phenyl, 2,4-Dimethoxy-6-sulfo-phenyl, 2-Sulfo-5-acetylamino-4-methyl-phenyl, 2-Methyl-4,6-disulfo-phenyl, 2,5-Disulfo-4-methoxy-phenyl, 2-Sulfo-5-nitro-phenyl, 2-Sulfo-4-nitro-phenyl, 2,6-Dimethyl-3-sulfo-phenyl, 2,6-Dimethyl-4-sulfo-phenyl, 3-Acetylamino-6-sulfo-phenyl, 4-Acetylamino-2-sulfo-phenyl, 4-Sulfo-naphth-1-yl, 3-Sulfo-naphth-1-yl, 5-Sulfo-naphth-1-yl, 6-Sulfo-naphth-1-yl, 7-Sulfo-naphth-1-yl, 8-Sulfo-naphth-1-yl, 3,6-Disulfo- und 5,7-Disulfo-naphth-1-yl, 3,7-Disulfo-naphth-1-yl, 3,6,8-Trisulfo-naphth-1-yl, 4,6,8-Trisulfo-naphth-1-yl, 5-Sulfo-naphth-2-yl, 6- oder 8-Sulfo-naphth-2-yl, 3,6,8-Trisulfo-naphth-2-yl, 1,5,7-Trisulfo-naphth-2-yl, 1,7-Disulfo-naphth-2-yl, 5,7-Disulfo-naphth-2-yl, 2,5,7-Trisulfo-naphth-1-yl, 4,6,8-Trisulfo-naphth-2-yl, 6,8-Disulfo-naphth-2-yl, 1,6-Disulfo-naphth-2-yl, 1-Sulfo-naphth-2-yl, 1,5-Disulfo-naphth-2-yl, 3,6-Disulfo-naphth-2-yl, 4,8-Disulfo-naphth-2-yl, 2-Hydroxy-5-sulfo-phenyl, 2-Hydroxy-4-sulfo-phenyl, 2-Hydroxy-3,5-disulfo-phenyl, 2-Hydroxy-5-acetylamino-3-sulfo-phenyl, 2-Hydroxy-3-acetylamino-4-sulfo-phenyl, 2-Hydroxy-5-chlor-4-sulfo-phenyl, 2-Hydroxy-5-methylsulfonyl-phenyl, 2-Hydroxy-6-nitro-4-sulfo-naphth-1-yl und 1-Hydroxy-4,8-disulfo-naphth-2-yl, 2-(β-Sulfatoethylsulfonyl)-phenyl, 3-(β-Sulfatoethylsulfonyl)-phenyl, 4-(β-Sulfatoethylsulfonyl)-phenyl, 2-Carboxy-5-(β-sulfatoethylsulfonyl)-phenyl, 2-Chlor-3-(sulfatoethylsulfonyl)-phenyl, 2-Chlor-4-(β-sulfatoethylsulfonyl)-phenyl, 2-Brom-4-(β-sulfatoethylsulfonyl)-phenyl, 4-Methoxy-3-(β-sulfatoethylsulfonyl)-phenyl, 4-Chlor-3-(β-sulfatoethylsulfonyl)-phenyl, 2-Ethoxy-4- oder -5-(β-sulfatoethylsulfonyl)-phenyl, 2-Methyl-4-(β-sulfatoethylsulfonyl)-phenyl, 2-Methoxy-5- oder -4-(β-sulfatoethylsulfonyl)-phenyl, 2,4-Diethoxy-5-(β-sulfatoethylsulfonyl)-phenyl, 2,4-Dimethoxy-5-(β-sulfatoethylsulfonyl)-phenyl, 2,5-Dimethoxy-4-(β-sulfatoethylsulfonyl)-phenyl, 2-Methoxy-5-methyl-4-(β-sulfatoethylsulfonyl)-phenyl, 2- oder 3- oder 4-(β-Thiosulfatoethylsulfonyl)-phenyl, 2-Methoxy-5-(β-thiosulfatoethylsulfonyl)-phenyl, 2-Sulfo-4-(β-phosphatoethylsulfonyl)-phenyl, 2-Sulfo-4-vinylsulfonyl-phenyl, 2-Hydroxy-4- oder -5-(β-sulfatoethylsulfonyl)-phenyl, 2-Chlor-4- oder -5-(β-chlorethylsulfonyl)-phenyl, 2-Hydroxy-5-(β-sulfatoethylsulfonyl)-phenyl, 2-Hydroxy-3-sulfo-5-(β-sulfatoethylsulfonyl)-phenyl, 3- oder 4-(β-Acetoxyethylsulfonyl)-phenyl, 6-Carboxy-1-sulfo-naphth-2-yl, 5-(β-Sulfatoethylsulfonyl)-naphth-2-yl, 6- oder 7- oder 8-(β-Sulfatoethylsulfonyl)-naphth-2-yl, 6-(β-Sulfatoethylsulfonyl)-1-sulfo-naphth-2-yl, 5-(β-Sulfatoethylsulfonyl)-1-sulfo-naphth-2-yl, 8-(β-Sulfatoethylsulfonyl)-6-sulfo-naphth-2-yl, 4-[N-Methyl-N-(β-sulfatoethylsulfonyl)]-amino-phenyl, 3-[N-Methyl-N-(β-sulfatoethylsulfonyl)]-amino-phenyl, 4-[β-(β'-Sulfatoethylsulfonyl)-ethyl]-phenyl, 3- oder 4-[β-(β'-Chlorethylsulfonyl)-ethylamino]-phenyl, 3- oder 4-[β-(β'-Sulfatoethylsulfonyl)-ethylamino]-phenyl, 3- oder 4-[γ-(β'-Chlorethylsulfonyl)-propylamino]-phenyl, 3- oder 4-[γ-(β'-Sulfatoethylsulfonyl)-propylamino]-phenyl, 3- oder 4-[γ-(Vinylsulfonyl)-propylamino]-phenyl, 4-[β-(β'-Sulfatoethylsulfonyl)-ethylamino]-2- oder -3-sulfo-phenyl, 4-[β-(β'-Chlorethylsulfonyl)-ethylamino]-2- oder -3-sulfo-phenyl, 4-[γ-(β'-Sulfatoethylsulfonyl)-propylamino]-2- oder -3-sulfo-phenyl, 4-[γ-(β'-Chlorethylsulfonyl)-propylamino]-2- oder -3-sulfo-phenyl, 4-[β-(β'-Chlorethylsulfonyl)-ethylamino]-2-carboxy-phenyl, 4-[β-(β'-Sulfatoethylsulfonyl)-ethylamino]-2-carboxy-phenyl, 4-[γ-(β'-Chlorethylsulfonyl)-propylamino]-2-carboxy-phenyl und 4-[γ-(β'-Sulfatoethylsulfonyl)-propylamino]-2-carboxy-phenyl.

Gruppen entsprechend den allgemeinen Formelresten D-N = N-E- bzw. D¹-N = N-E- sind beispielsweise 4-(4'-Sulfo-phenyl)-azo-2-sulfo-phenyl, 4-(2',4'-Disulfo-phenyl)-azo-2-methoxy-5-methyl-phenyl, 4-(2',5'-Disulfo-phenyl)-azo-2-methyl-5-methoxy-phenyl, 4-(3',6',8'-Trisulfo-naphth-2'-yl)-azo-3-ureido-phenyl, 4-(4',8'-Disulfo-naphth-2'-yl)-azo-3-acetylamino-phenyl, 7-(1',5'-Disulfo-naphth-2'-yl)-azo-6-sulfo-8-hydroxy-naphth-3-yl und 4-(4'-Sulfo-phenyl)-azo-6-sulfo-naphth-1-yl, 4-[4'-(β-Sulfatoethylsulfonyl)-phenyl]-azo-2-methyl-5-methoxy-phenyl, 4-[3'-(β'-Sulfatoethylsulfonyl)-phenyl]-azo-3-methyl-phenyl, 4-[4'-(β-Sulfatoethylsulfonyl)-phenyl]-azo-3-ureido-phenyl, 4-[6'-(β-Sulfatoethylsulfonyl)-naphth-2'-yl]-azo-3-ureido-phenyl, 7-[2'-Methoxy-5'-(β-sulfatoethylsulfonyl)-phenyl]-azo-8-hydroxy-6-sulfo-naphth-3-yl, 4-(2',5'-Disulfo-phenyl)-azo-6- oder -7-sulfo-naphth-1-yl, 4-(2',4'-Disulfo-phenyl)-azo-6- oder -7-sulfo-naphth-1-yl, 4-(4',8'-Disulfo-naphth-2'-yl)-azo-6- oder -7-sulfo-naphth-1-yl, 4-(3',6',8'-Trisulfo-naphth-2'-yl)-azo-6- oder -7-sulfo-naphth-1-yl und 4-(4',6',8'-Trisulfo-naphth-2'-yl)-azo-6- oder -7-sulfo-naphth-1-yl.

Aromatische Reste Z-D²- von als Diazokomponenten dienenden Verbindungen der allgemeinen Formeln Z-D²-NH₂ oder von deren Diaminobenzol- und Diaminonaphthalin-Verbindungen entsprechend den allgemeinen Formeln H₂N-D²-NH₂ sind bevorzugt Reste der allgemeinen Formeln (6a) und (6b) 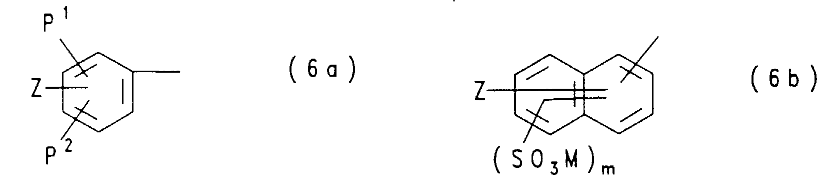 in welchen Z, M, m, P¹ und P² die oben angegebenen, insbesondere bevorzugten Bedeutungen haben, wobei der jeweilige Benzolkern in ortho-Stellung zur freien Bindung, die zur Azogruppe führt, zusätzlich eine Hydroxygruppe enthalten kann.

Aromatische Reste E einer kupplungsfähigen und diazotierbaren Verbindung der allgemeinen Formel H-E-NH₂ sind beispielsweise solche der allgemeinen Formeln (7a), (7b) und (7c) 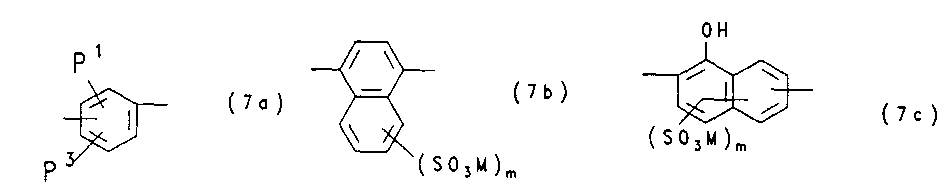 in welchen
- P¹, M und m: die oben angegebenen Bedeutungen haben und
- P³: Wasserstoff, Alkyl von 1 bis 4 C-Atomen, wie Methyl oder Ethyl, Alkoxy von 1 bis 4 C-Atomen, wie Methoxy und Ethoxy, Chlor, Alkanoylamino von 2 bis 5 C-Atomen, wie Acetylamino und Propionylamino, Benzoylamino, Ureido, Phenylureido, Alkylureido mit 1 bis 4 C-Atomen im Alkylrest, Phenylsulfonyl oder Alkylsulfonyl von 1 bis 4 C-Atomen ist.

Reste K¹ von Kupplungskomponenten der allgemeinen Formel H-K¹ , die keine faserreaktive Gruppe der Formel (2) tragen, sind beispielsweise solche der allgemeinen Formeln (8a) bis (8h) 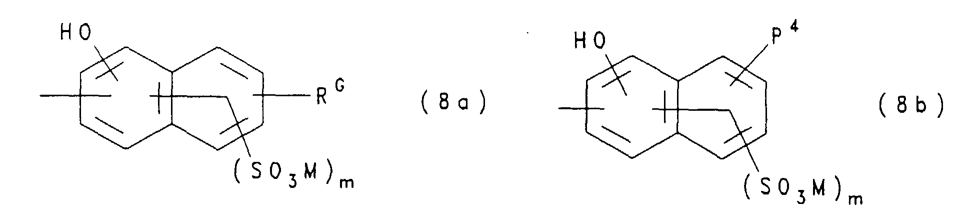 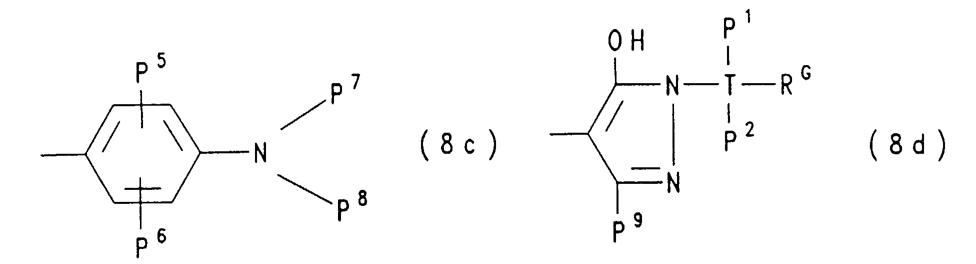 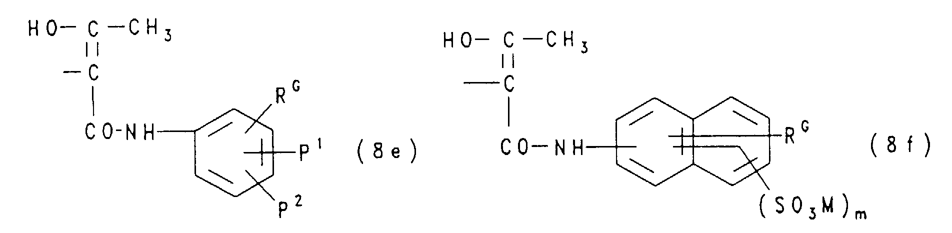 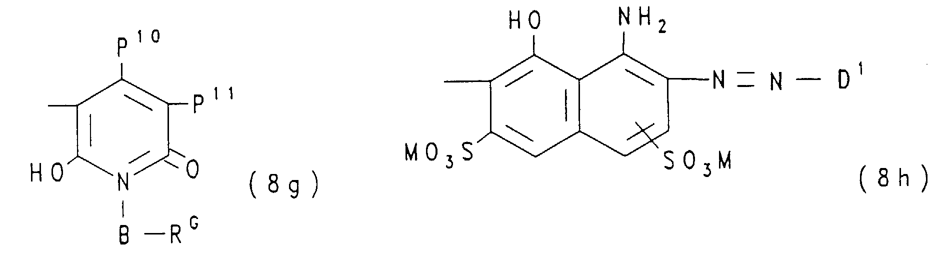 in welchen
- R^{G}, P¹, P², m und M: die obengenannten Bedeutungen haben,
- P⁴: Alkanoylamino von 2 bis 5 C-Atomen, wie Propionylamino und insbesondere Acetylamino, oder Phenylureido ist, das im Phenylrest durch Substituenten aus der Gruppe Chlor, Methyl, Methoxy, Sulfo und Carboxy und/oder durch eine Gruppe -SO₂-Y mit Y einer der obigen Bedeutungen substituiert sein kann, oder Benzoylamino ist, das im Phenylrest durch Substituenten aus der Gruppe Chlor, Methyl, Methoxy, Nitro, Sulfo und Carboxy und/oder durch eine Gruppe -SO₂-Y mit Y einer der obigen Bedeutungen substituiert sein kann,
- P⁵: Wasserstoff, Alkyl von 1 bis 4 C-Atomen, wie Methyl und Ethyl, Alkoxy von 1 bis 4 C-Atomen, wie Methoxy und Ethoxy, Brom, Chlor oder Sulfo ist,
- P⁶: Wasserstoff, Alkyl von 1 bis 4 C-Atomen, wie Methyl und Ethyl, Alkoxy von 1 bis 4 C-Atomen, wie Methoxy und Ethoxy, Chlor, Alkanoylamino von 2 bis 7 C-Atomen, wie Acetylamino und Propionylamino, Ureido oder Phenylureido ist,
- P⁷: Wasserstofff oder Alkyl von 1 bis 4 C-Atomen ist, das durch Hydroxy, Cyano, Carboxy, Sulfo, Sulfato, Methoxycarbonyl, Ethoxycarbonyl oder Acetoxy substituiert sein kann,
- P⁸: Alkyl von 1 bis 4 C-Atomen ist, das durch Hydroxy, Cyano, Carboxy, Sulfo, Sulfato, Methoxycarbonyl, Ethoxycarbonyl oder Acetoxy substituiert sein kann, oder Benzyl oder Phenyl oder durch Alkyl von 1 bis 4 C-Atomen, Alkoxy von 1 bis 4 C-Atomen, Chlor und/oder Sulfo substituiertes Phenyl ist,
- P⁹: Wasserstoff, Alkyl von 1 bis 4 C-Atomen, wie Methyl, Cyano, Carboxy, Carbalkoxy von 2 bis 5 C-Atomen, wie Carbomethoxy und Carbethoxy, Carbamoyl oder Phenyl, bevorzugt Methyl, Carboxy, Methoxycarbonyl, Ethoxycarbonyl oder Phenyl und insbesondere Methyl oder Carboxy, ist
- T: für einen Benzol- oder Naphthalinring, bevorzugt Benzolring, steht,
- P¹⁰: Wasserstoff oder Alkyl von 1 bis 4 C-Atomen, wie Methyl, oder durch Alkoxy von 1 bis 4 C-Atomen, wie Methoxy, oder Cyano substituiertes Alkyl von 1 bis 4 C-Atomen oder Phenyl ist, bevorzugt Alkyl von 1 bis 4 C-Atomen oder Phenyl ist,
- P¹¹: Wasserstoff, Chlor, Brom, Sulfo, Carbamoyl, Methylsulfonyl, Phenylsulfonyl, Cyano oder Sulfoalkyl von 1 bis 4 C-Atomen bedeutet, bevorzugt Wasserstoff, Sulfo, Sulfoalkyl mit einem Alkylrest von 1 bis 4 C-Atomen, wie Sulfomethyl, Cyano oder Carbamoyl ist,
- B: Alkylen von 1 bis 4 C-Atomen, Methylenphenylen, Ethylenphenylen, Phenylenmethylen, Phenylenethylen oder Phenylen oder im Benzolrest durch Fluor, Chlor, Brom, Methyl, Methoxy, Cyano, Sulfo, Carboxy, Acetyl, Nitro, Carbamoyl und/oder Sulfamoyl substituiertes Methylenphenylen, Ethylenphenylen oder Phenylen ist und
- D¹: ein Rest der allgemeinen Formel (4a) oder (4b) ist.

Reste -K²-Z von Kupplungskomponenten der allgemeinen Formel H-K²-Z bzw. H-K²-N(R^{A})H , in die der faserreaktive Rest entsprechend der später angegebenen Gruppierung Z¹ nachträglich eingeführt werden muß, sind beispielsweise Reste der allgemeinen Formeln (9a) bis (9h) 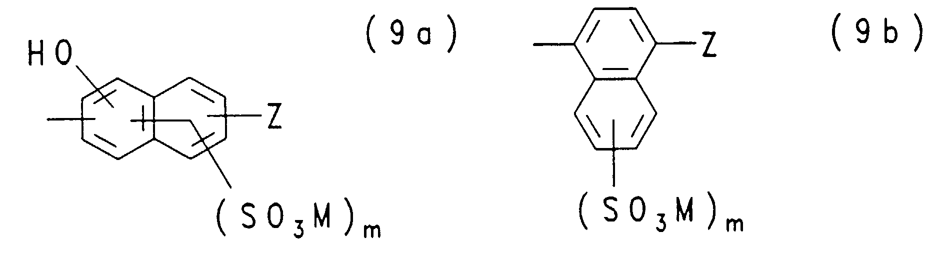 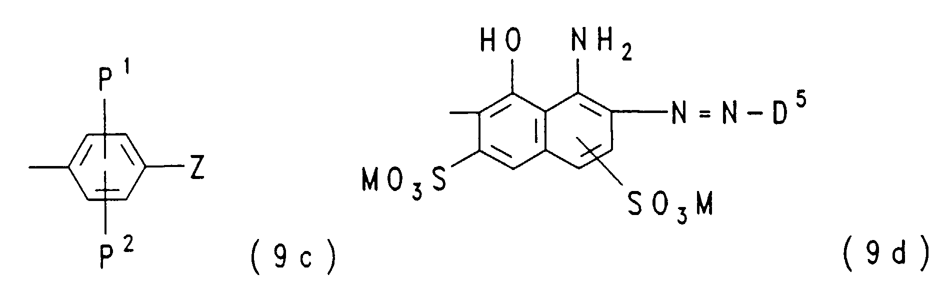 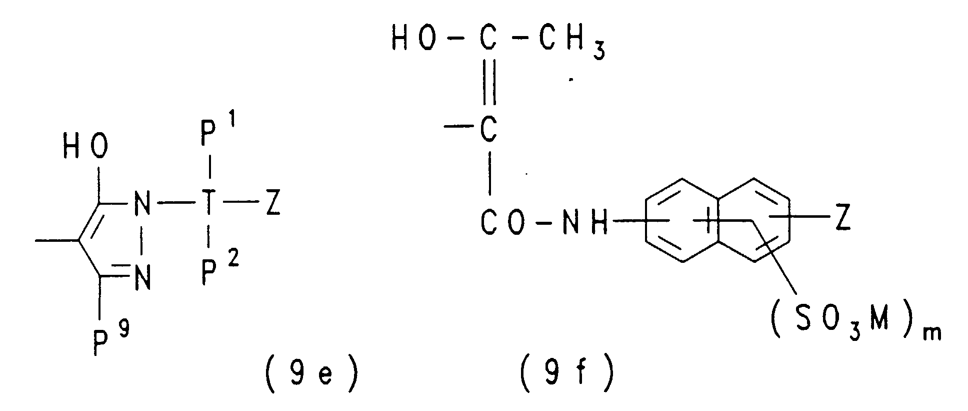 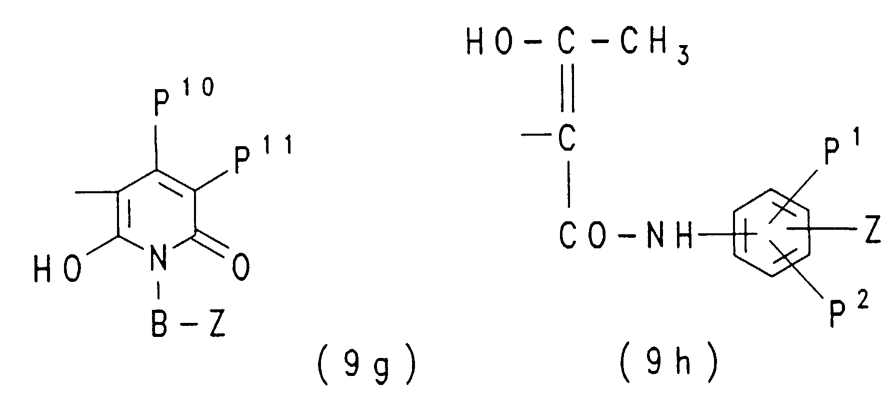 in welchen
- P¹, P², P⁹, P¹⁰, P¹¹, B, T, M, m und Z: die oben angegebenen, insbesondere bevorzugten, Bedeutungen haben und
- D⁵: als Rest einer Diazokomponente ein Rest der obengenannten und definierten allgemeinen Formel (6a) oder (6b) ist.

In den obigen Formeln (8a), (8b) und (9a) steht die freie Bindung, die zur Azogruppe führt, in ortho-Stellung zur Hydroxygruppe an den aromatischen Kern gebunden.

Reste K³ in den Formeln (3g) und (3h) mit einem metallkomplex-bindenden Sauerstoffatom sind insbesondere solche der Formeln (10a) bis (10e) 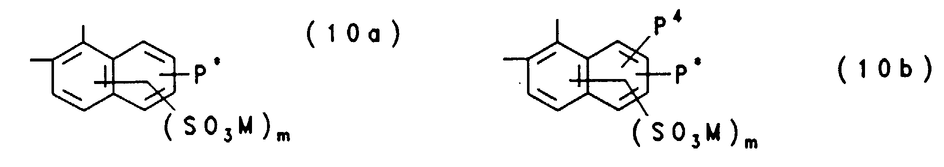 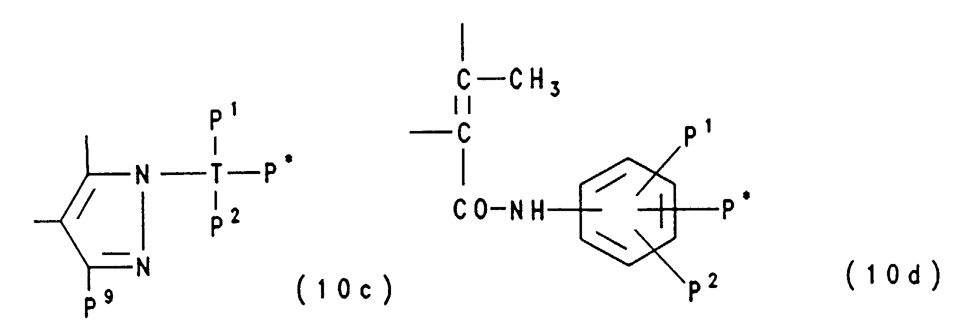 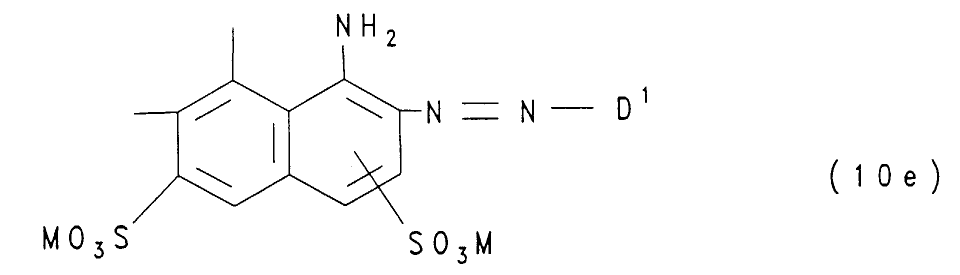 in welchen die einzelnen Formelglieder eine der obengenannten Bedeutungen haben und
- P*: Wasserstoff oder ein Rest Z oder eine Gruppierung der Formel -N=N-K²-Z oder -N=N-K-H mit K², Z und K der obengenannten Bedeutung ist.

Von den Azofarbstoffen sind weiterhin bevorzugt solche, die den allgemeinen Formeln (12A) bis (12S) 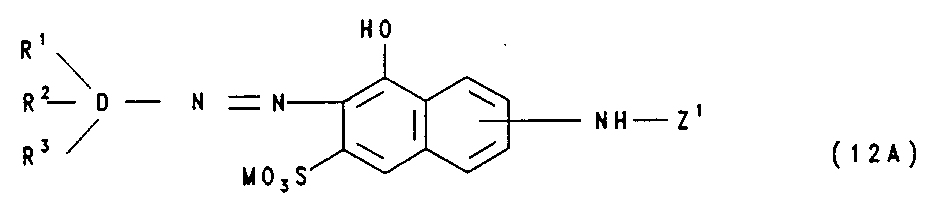 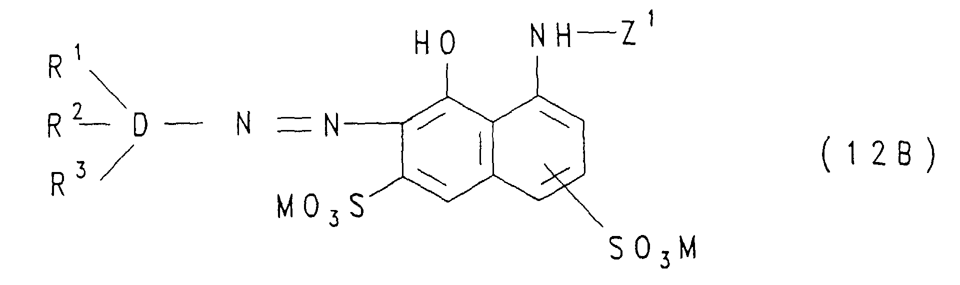 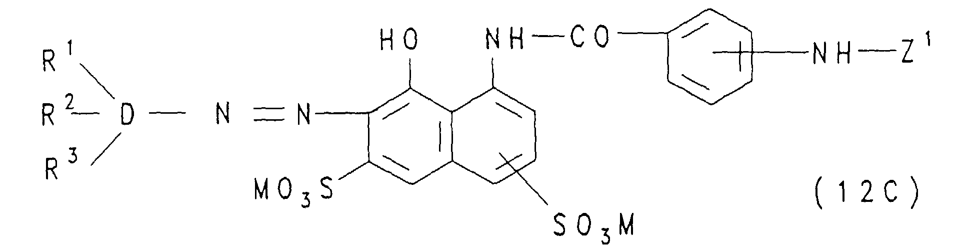 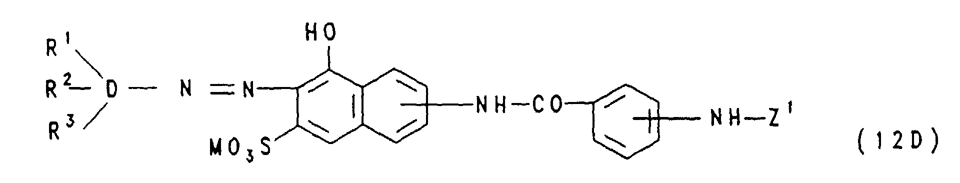 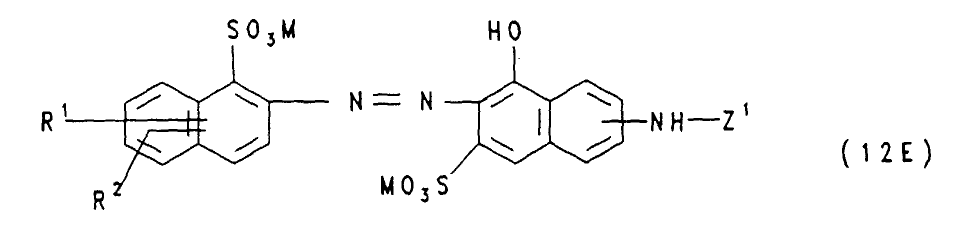 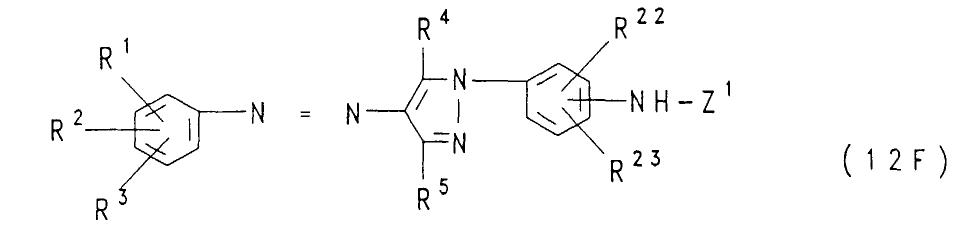 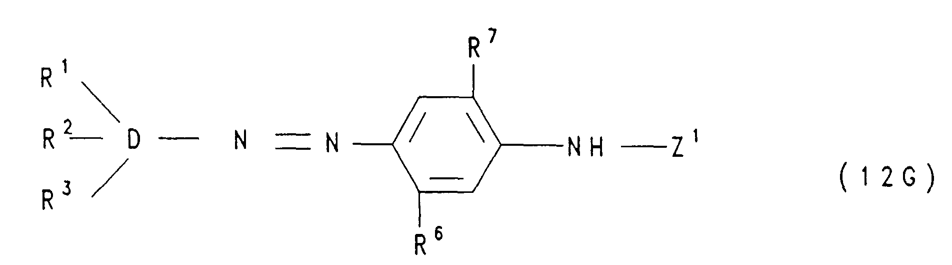 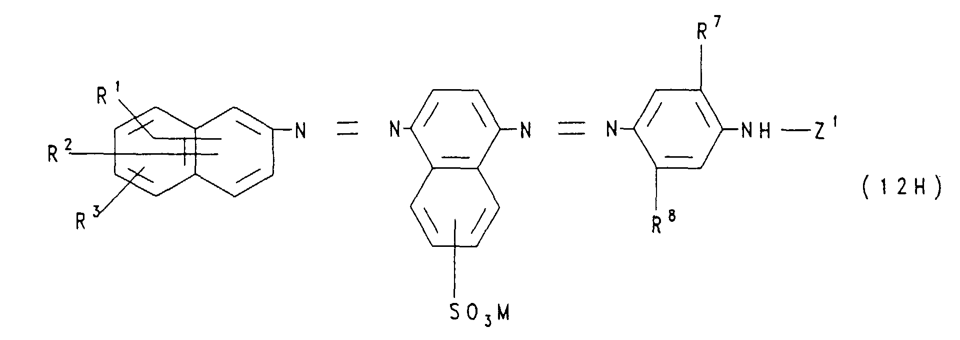 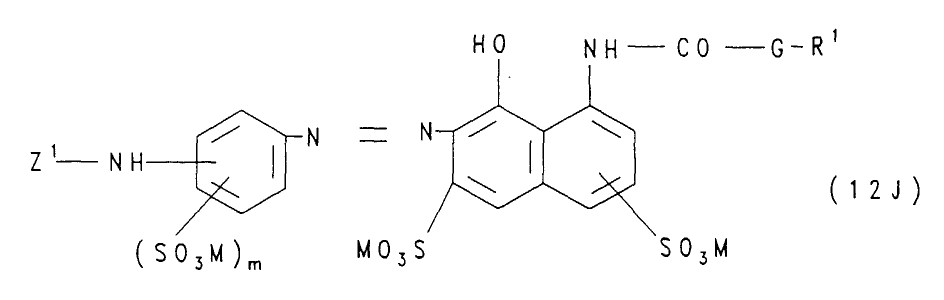 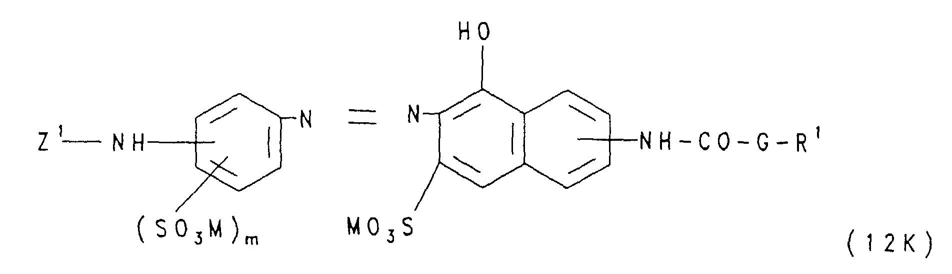 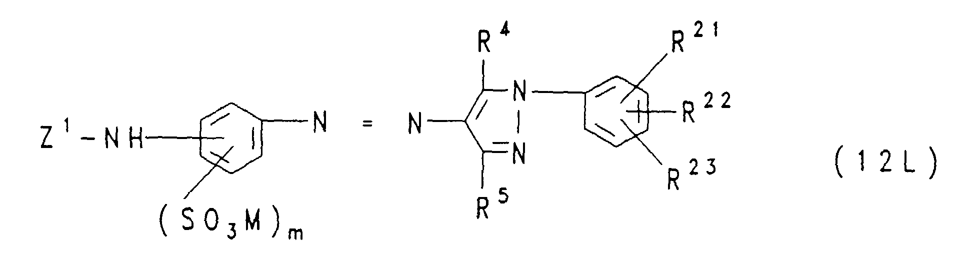 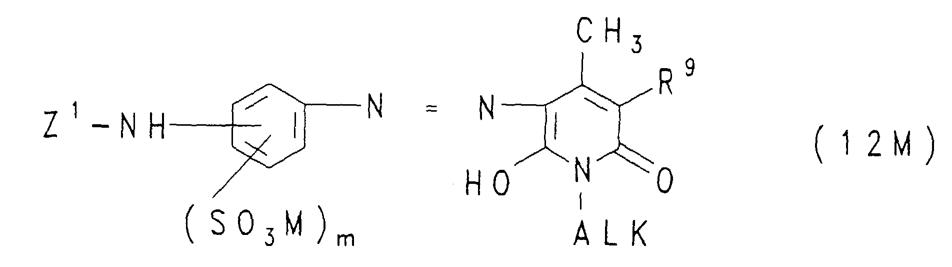 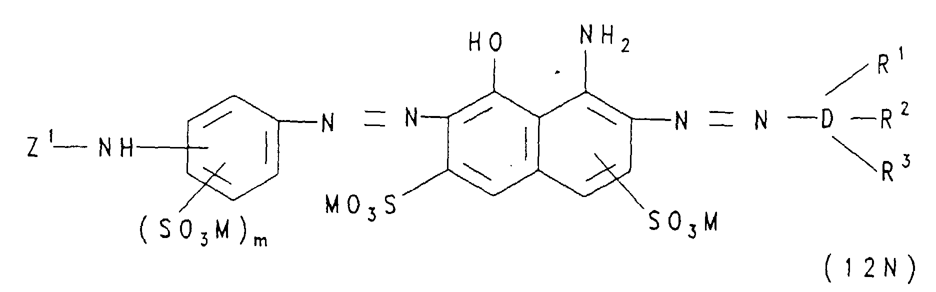 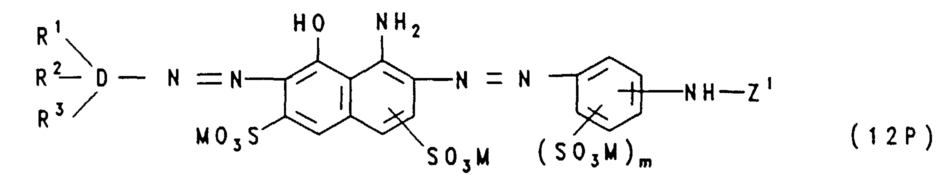 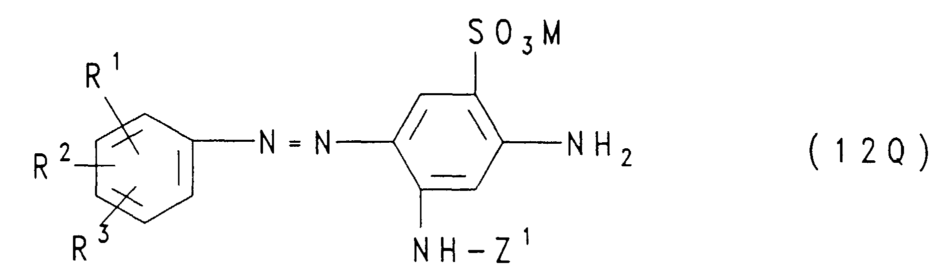 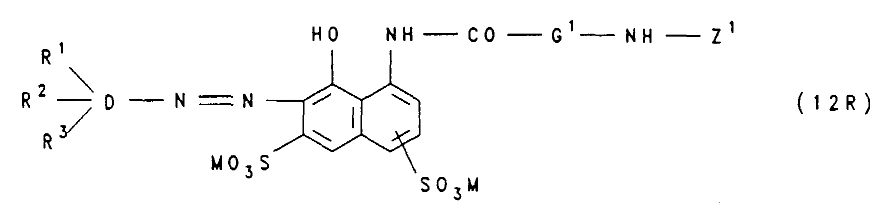 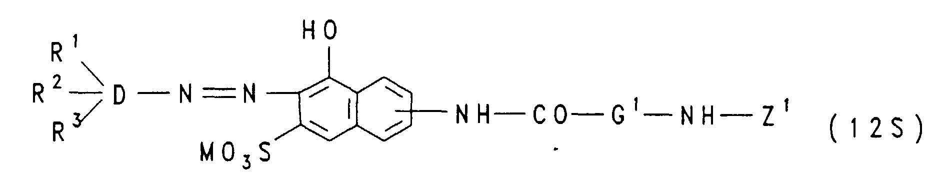 entsprechen, in welchen bedeuten:
- Z¹: ist ein Rest der allgemeinen Formel (2A) 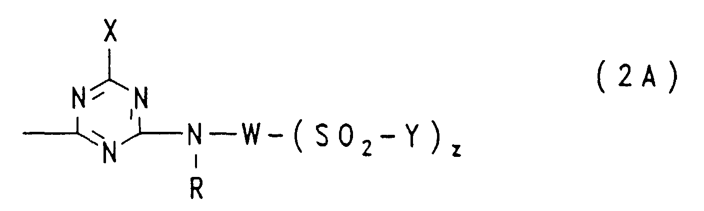 in welcher X, R, W, Y und z die obengenannten, insbesondere bevorzugten, Bedeutungen haben;
- M: hat eine der obengenannten Bedeutungen;
- D: ist ein Benzolring oder ist ein Naphthalinring, wobei die Azogruppe an den Naphthalinring bevorzugt in β-Stellung gebunden ist und wobei im Falle, daß D den Naphthalinring bedeutet, R² und R³ bevorzugt jedes, unabhängig voneinander, ein Wasserstoffatom oder eine Sulfogruppe bedeuten;
- R¹: ist Wasserstoff oder Sulfo oder eine Gruppe Y-SO₂-W^{o}- obengenannter Bedeutung;
- R²: ist Wasserstoff, Alkyl von 1 bis 4 C-Atomen, wie Ethyl und insbesondere Methyl, Alkoxy von 1 bis 4 C-Atomen, wie Ethoxy und insbesondere Methoxy, Halogen, wie Chlor und Brom, Carboxy oder Sulfo und ist bevorzugt Wasserstoff, Methyl, Methoxy, Brom, Chlor, Sulfo oder Carboxy und insbesondere bevorzugt Wasserstoff, Methoxy oder Sulfo;
- R³: ist Wasserstoff, Alkyl von 1 bis 4 C-Atomen, wie Ethyl und insbesondere Methyl, Alkoxy von 1 bis 4 C-Atomen, wie Ethoxy und insbesondere Methoxy, Halogen, wie Chlor und Brom, Nitro, Carboxy oder Sulfo, bevorzugt Wasserstoff, Methyl, Methoxy, Chlor, Carboxy, Sulfo oder Acetylamino und insbesondere bevorzugt Wasserstoff, Methoxy oder Sulfo;
- R⁴: ist Hydroxy oder Amino, bevorzugt Hydroxy;
- R⁵: ist Methyl, Carboxy, Carbomethoxy oder Carbethoxy, bevorzugt Methyl oder Carboxy;
- R⁶: ist Acetylamino, Propionylamino, Ureido oder Methyl;
- R⁷: ist Wasserstoff, Alkyl von 1 bis 4 C-Atomen, wie Methyl und Ethyl, Alkoxy von 1 bis 4 C-Atomen, wie Methoxy und Ethoxy, Halogen, wie Brom und insbesondere Chlor, vorzugsweise Wasserstoff, Methyl, Ethyl, Methoxy oder Ethoxy;
- R⁸: ist Wasserstoff oder Alkyl von 1 bis 4 C-Atomen, wie Methyl und Ethyl, Alkanoylamino von 2 bis 5 C-Atomen, wie Acetylamino und Propionylamino, oder Ureido, vorzugsweise Wasserstoff, Methyl, Ethyl, Methoxy, Ethoxy, Acetylamino oder Ureido;
- R⁹: ist Wasserstoff, Cyano, Carbamoyl, Sulfamoyl oder Sulfomethyl, bevorzugt Wasserstoff oder Carbamoyl;
- R²¹: hat eine der Bedeutungen von R¹;
- R²²: hat eine der Bedeutungen von R²;
- R²³: hat eine der Bedeutungen von R³;
- ALK: ist Alkyl von 1 bis 4 C-Atomen, wie Methyl und Ethyl, oder Alkyl von 2 bis 4 C-Atomen, wie Ethyl und Propyl, das durch Hydroxy, Carboxy, Sulfo oder Sulfato substituiert sein kann;
- G: ist Alkylen von 1 bis 4 C-Atomen, wie Ethylen und Propylen, oder ist Phenylen oder durch Sulfo, Carboxy und/oder Alkyl von 1 bis 4 C-Atomen, wie Methyl, substituiertes Phenylen;
- G¹: ist eine Gruppe der Formel -NH-alk- , -NH-phen- oder -alk- , in welchen alk für Alkylen von 2 bis 4 C-Atomen steht und phen Sulfo-phenylen oder Phenylen bedeutet;
- m: ist die Zahl Null, 1 oder 2 (wobei im Falle von m gleich Null diese Gruppe Wasserstoff ist);
in den Verbindungen der Formeln (12A), (12D), (12E), (12K) und (12S) steht die Amino- bzw. Amidogruppierung in 2- oder 3-Stellung an den 8-Naphthol-Rest gebunden und in den Verbindungen der Formeln (12B), (12C), (12J), und (12R) steht die Gruppe -SO₃M in meta- oder para-Stellung zur Amino- bzw. Acylaminogruppe gebunden.

Reste entsprechend der allgemeinen Formel (2a) 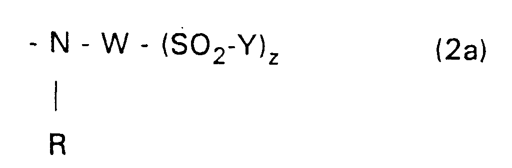 die in den Resten Z und Z¹ enthalten sind, sind beispielsweise: N-Phenyl-N-(γ-vinylsulfonyl-propyl)-amino, N-(4-Chlorphenyl)-N-(γ-vinylsulfonylpropyl)-amino, N-(2-Methylphenyl)-N-(γ-vinylsulfonyl-propyl)-amino, N-(4-Methoxyphenyl)-N-(γ-vinylsulfonyl-propyl)-amino, N-(3-Sulfophenyl)-N-(γ-vinylsulfonyl-propyl)-amino, N-(4-Sulfophenyl)-N-(γ-vinylsulfonyl-propyl)-amino, N-Phenyl-N-[γ-(β'-sulfatoethylsulfonyl)-propyl]-amino, N-(4-Chlorphenyl)-N-[γ-(β'-sulfatoethylsulfonyl)-propyl]-amino, N-(2-Methylphenyl)-N-[γ-(β'-sulfatoethylsulfonyl)-propyl]-amino, N-(4-Methoxyphenyl)-N-[γ-(β'-sulfatoethylsulfonyl)-propyl]-amino, N-(3-Sulfophenyl)-N-[γ-(β'-sulfatoethylsulfonyl)-propyl]-amino und N-(4-Sulfophenyl)-N-[γ-(β'-sulfatoethylsulfonyl)-propyl]-amino.

Bevorzugt von diesen Gruppen (2a) ist insbesondere N-Phenyl-[γ-(β'-sulfatoethylsulfonyl)-propyl]-amino zu nennen.

Schwermetallkomplex-Azofarbstoffe von besonderer Bedeutung sind beispielsweise die 1,2-Chromkomplex- und 1,2-Kobaltkomplex- und insbesondere die 1:1-Kupferkomplex-Monoazoverbindungen der Azoverbindungen der nachstehenden allgemeinen Formeln (12T) und (12U): 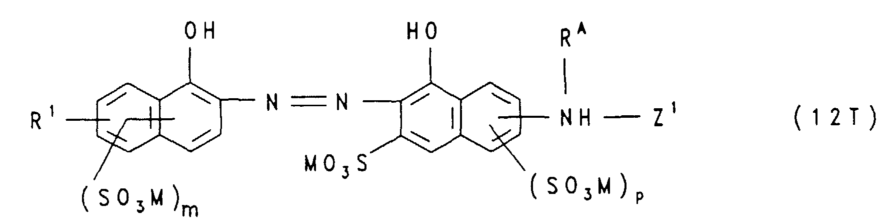 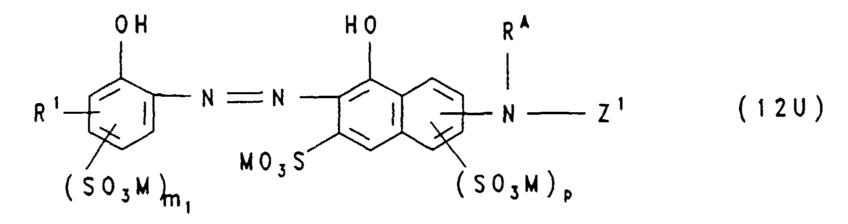 in welchen
- M, m, Z¹, R^{A} und R¹: die obengenannten, insbesondere bevorzugten, Bedeutungen haben,
- p: für die Zahl Null oder 1 steht (wobei im Falle von p gleich Null diese Gruppe Wasserstoff bedeutet) und
- m₁: die Zahl Null oder 1 ist (wobei im Falle von m gleich Null, diese Gruppe Wasserstoff bedeutet).

Von den erfindungsgemäßen Phthalocyaninfarbstoffen sind diejenigen bevorzugt, die der allgemeinen Formel (13) 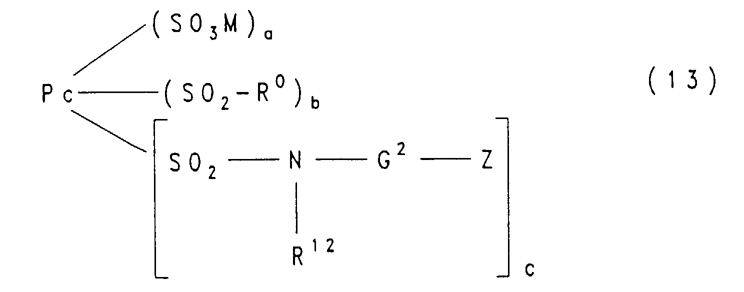 entsprechen, in welcher bedeuten:
- Pc: ist der Rest eines Nickel- oder bevorzugt Kupferphthalocyanins;
- R^{o}: ist eine Aminogruppe der Formel -NR¹³R¹⁴, in welcher R¹³ und R¹⁴ unabhängig voneinander Wasserstoff oder Alkyl von 1 bis 4 C-Atomen, das durch Hydroxy oder Sulfo substituiert sein kann, bedeuten, oder ist ein heterocyclischer, N-haltiger Rest, wie der Morpholino- oder Piperidino-Rest;
- R¹²: ist ein Wasserstoff oder Alkyl von 1 bis 4 C-Atomen, wie Methyl und Ethyl;
- G²: ist Phenylen, das durch 1 oder 2 Substituenten aus der Gruppe Alkyl von 1 bis 4 C-Atomen, wie Ethyl und Methyl, Halogen, wie Chlor und Brom, Carboxy und Sulfo substituiert sein kann, beispielsweise Sulfophenylen, oder ist Alkylen von 2 bis 6 C-Atomen, wie Ethylen;
- Z: ist die faserreaktive Gruppe der Formel (2);
- a: ist eine Zahl von Null bis 3,
- b: ist eine Zahl von Null bis 3 und
- c: ist eine Zahl von 1 bis 2,
wobei die Summe von (a + b + c) gleich einer Zahl von 2 bis 4 ist.

Von diesen sind insbesondere diejenigen zu nennen, die den allgemeinen Formeln (13a) und (13b) 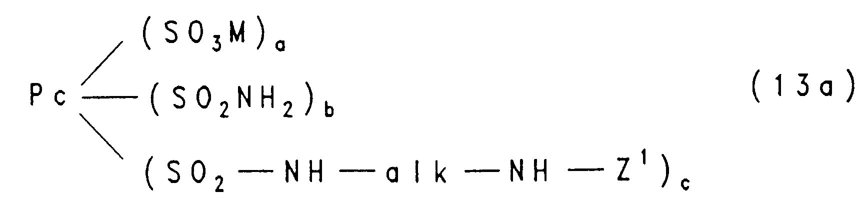 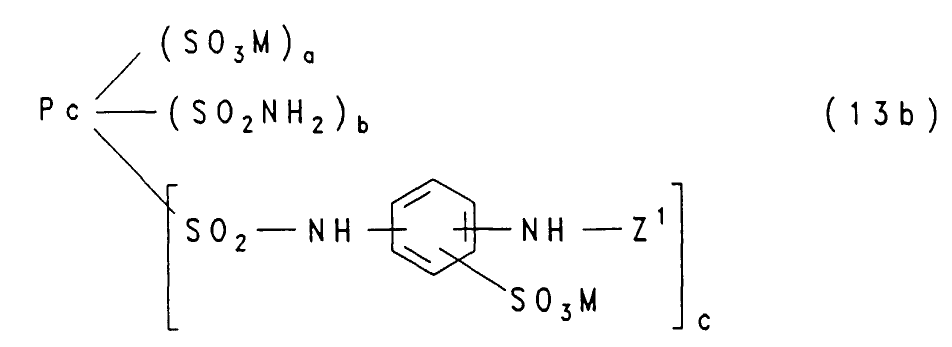 entsprechen, in welchen a für eine Zahl von 1 bis 3 steht, b eine Zahl von 0 bis 2 ist und c eine Zahl von 1 bis 2 bedeutet, wobei die Summe von (a + b + c) gleich einer Zahl von 2 bis 4 ist, Pc den Rest eines Nickel- oder bevorzugt Kupferphthalocyanins darstellt und alk Alkylen von 2 bis 4 C-Atomen, bevorzugt Ethylen, ist.

Erfindungsgemäße Kupferformazanfarbstoffe sind insbesondere diejenigen, die der allgemeinen Formel (14) 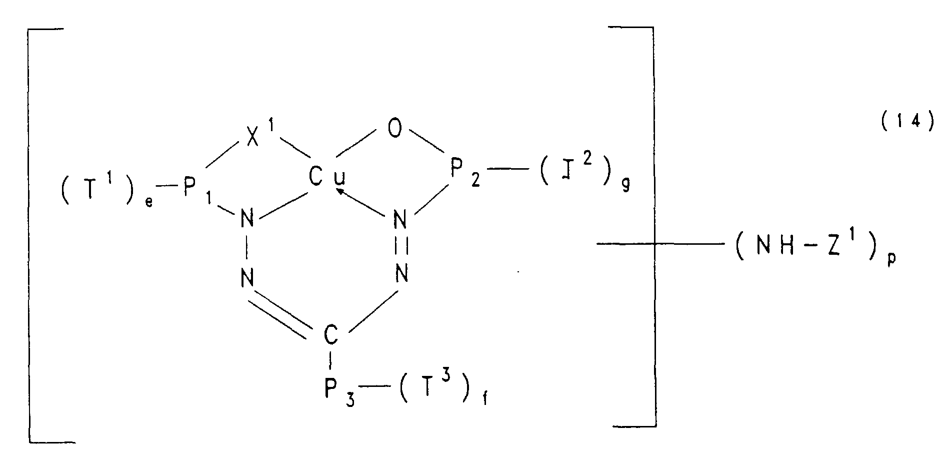 entsprechen, in welcher bedeuten:
- X¹: ist ein Sauerstoffatom oder bevorzugt die Carbonyloxygruppe der Formel -COO- ;
- P₁ und P₂: bedeuten, unabhängig voneinander, jedes einen Benzol- oder Naphthalinring, wobei an P₁ das Stickstoffatom und die Gruppe X¹ ortho-ständig zueinander und an P₂ das Sauerstoffatom und das Stickstoffatom ortho-ständig zueinander gebunden sind und die Benzolkerne bzw. Napthalinkerne noch durch einen oder zwei Substituenten aus der Gruppe Halogen, wie Chlor, Nitro, Alkyl von 1 bis 4 C-Atomen, wie Methyl und Ethyl, Alkoxy von 1 bis 4 C-Atomen, wie Methoxy und Ethoxy, Sulfamoyl, durch Alkyl von 1 bis 4 C-Atomen mono- oder disubstituiertes Sulfamoyl, Alkylsulfonyl von 1 bis 4 C-Atomen, wie Methylsulfonyl und Ethylsulfonyl, und Phenylsulfonyl substituiert sein können, wobei sowohl P₁ als auch P₂ beide bevorzugt einen Benzolring bedeuten;
- P₃: ist eine geradkettige oder verzweigte Alkylengruppe von 2 bis 6 C-Atomen, vorzugsweise von 2 bis 4 C-Atomen, die durch eine Sulfophenylgruppe substituiert sein kann, oder ist eine Phenylengruppe oder eine Naphthylengruppe, die durch 1 oder 2 Substituenten aus der Gruppe Methyl, Ethyl, Methoxy, Ethoxy und Chlor substituiert sein können, wobei P₃ bevorzugt ein Benzolring ist;
- T¹, T² und T³: bedeuten, unabhängig voneinander, jedes eine Sulfo- oder Carboxygruppe, bevorzugt Sulfogruppe;
- e, f und g: stellen, unabhängig voneinander, jedes die Zahl Null, 1 oder 2 dar, wobei die Summe von (e + f + g) eine ganze Zahl von 1 bis 4 bedeutet und vorzugsweise 2 oder 3, insbesondere 2, ist, wobei im Falle von e oder f oder g gleich Null die Gruppe T¹ bzw. T² bzw. T³ ein Wasserstoffatom bedeutet;
- p: steht für die Zahl 1 oder 2, bevorzugt 1, wobei die Gruppe -NH-Z¹ an einen aromatischen Rest von P₁, P₂ oder P₃ gebunden sein kann und bevorzugt an P₂ gebunden ist.

Bevorzugt sind von den Kupferformazanfarbstoffen der allgemeinen Formel (14) diejenigen, in welchen P₁ und P₂ beide für einen Benzolring stehen, die Gruppe -NH-Z¹ an P₂ gebunden ist und T¹ und T² jedes eine Sulfogruppe bedeutet, wobei e und g beide für die Zahl 1 stehen. Sofern die Gruppe -NH-Z¹ an P₁ gebunden ist, ist e die Zahl Null, g die Zahl 2 und T² eine Sulfogruppe. Bevorzugt ist weiterhin die Gruppierung -P₃-(T³)_{f} der Phenyl- oder ein 2- oder 4-Sulfo-phenyl-Rest.

Hiervon sind insbesondere diejenigen Kupferformazan-Farbstoffe hervorzuheben, die der allgemeinen Formel (14a) 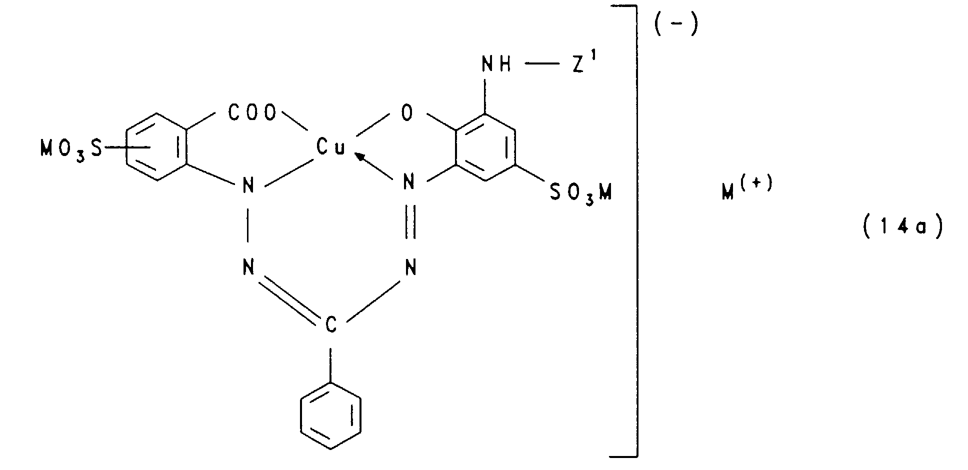 entsprechen, in welcher M und Z¹ die obengenannte, insbesondere bevorzugten Bedeutungen haben.

Von den erfindungsgemäßen Triphendioxazinfarbstoffen sind diejenigen hervorzuheben, die der allgemeinen Formel (15) 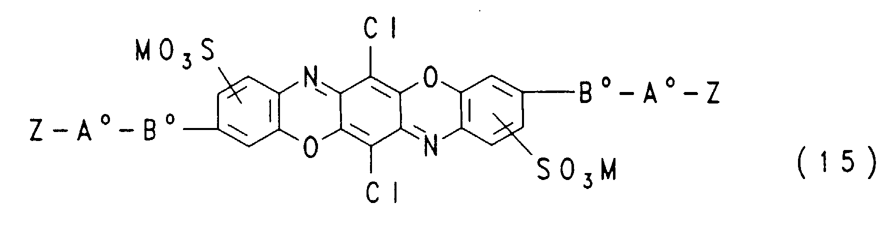 entsprechen, in welcher M und Z eine der obengenannten Bedeutungen haben, B^{o} die Oxigruppe -O- oder die Aminogruppe -NH- ist und A^{o} Alkylen von 2 bis 6 C-Atomen ist, das durch 1 oder 2 Heterogruppen, wie Gruppen der Formeln -O- , -NH- , -NH-CO- und/oder -CO-NH- , unterbrochen und/oder das substituiert sein kann, bspw. durch Hydroxy, Sulfo, Sulfato oder Carboxy, oder Cyclohexylen ist, oder in welcher die Gruppierung Z-A^{o}-B^{o}- bzw. -B^{o}-A^{o}-Z zusammen den Rest Z¹-NH- bzw. -NH-Z¹ obiger Bedeutung darstellt, und worin die beiden Sulfogruppen -SO₃M bevorzugt in ortho-Stellung zum Sauerstoffatom des heterocyclischen Ringes an den Benzolkern gebunden sind.

Von den erfindungsgemäßen Anthrachinonfarbstoffen sind insbesondere diejenigen zu erwähnen, die der allgemeinen Formel (16) 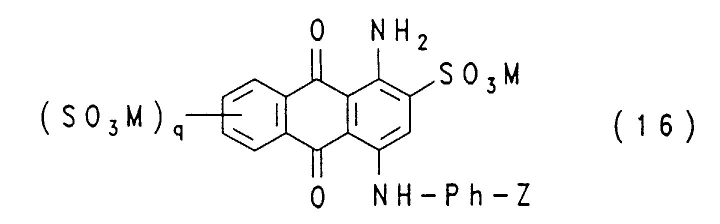 entsprechen, in welcher
- M, Z und q: eine der obengenannten Bedeutungen haben und
- Ph: Phenylen ist, das durch 1 oder 2 Substituenten aus der Gruppe Alkyl von 1 bis 4 C-Atomen, wie Methyl und Ethyl, Alkoxy von 1 bis 4 C-Atomen, wie Methoxy und Ethoxy, Halogen, wie Chlor und Brom, Carboxy und Sulfo substituiert sein kann, oder Phenylen ist, das durch 3 oder 4 Methylgruppen substituiert ist.

Die vorliegende Erfindung betrifft weiterhin Verfahren zur Herstellung der erfindungsgemäßen Farbstoffe der allgemeinen Formel (1). Sie lassen sich in an und für sich in üblicher Weise analog bekannten, für die jeweilige Farbstoffklasse spezifischen Synthesewegen herstellen, indem man für den jeweiligen Farbstoff typische Vorprodukte, von denen mindestens eines eine Gruppe der allgemeinen Formel (2) enthält, miteinander umsetzt, oder indem eine Halogen-triazin-Verbindung der allgemeinen Formel (21) 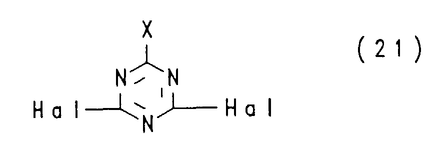 in welcher X eine der obengenannten, insbesondere bevorzugten, Bedeutungen besitzt und Hal für Halogen, wie Chlor oder Fluor, steht, wie beispielsweise 2,4,6-Trifluor-1,3,5-triazin (Cyanurfluorid) oder 2,4,6-Trichlor-1,3,5-triazin (Cyanurchlorid), in beliebiger Reihenfolge mit einer aminogruppenhaltigen Verbindung der allgemeinen Formel (20) 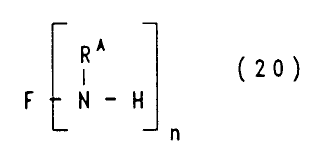 mit F, R^{A} und n der obengenannten Bedeutungen in äquimolarer Menge und einer Aminoverbindung der allgemeinen Formel (22) 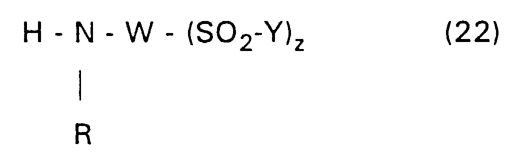 mit R, W, Y und z der obengenannten Bedeutungen in äquimolarer oder zweifach äquimolarer Menge umsetzt.

Erfindungsgemäße Verfahrensvarianten dieser erfindungsgemäßen Verfahrensweise sind beispielsweise dadurch gekennzeichnet, daß man eine Verbindung der allgemeinen Formel (23) 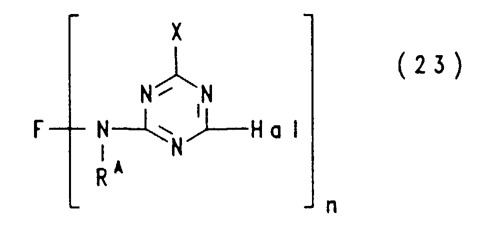 in welcher F, R^{A}, X, Hal und n die obengenannten Bedeutungen haben, mit einer Aminoverbindung der allgemeinen Formel (22) in äquimolarer oder gegebenenfalls, sofern X für ein Halogen steht, in zweifach molarer Menge umsetzt, oder daß man eine Verbindung der allgemeinen Formel (24) 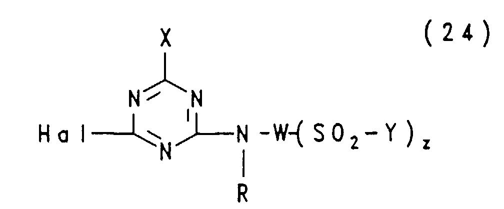 in welcher Hal, X, R, W, Y und z die obengenannten Bedeutungen haben, mit einer Verbindung der allgemeinen Formel (20) der obengenannten Bedeutung umsetzt. Hiervon ist die Verfahrensweise der Umsetzung einer Verbindung der allgemeinen Formel (23) mit einer Verbindung der allgemeinen Formel (22) bevorzugt.

Bei den Kondensationsreaktionen ist darauf zu achten, daß die faserreaktiven Gruppierungen im alkalischen Bereich nicht geschädigt werden.

Die Umsetzungen der Ausgangsverbindungen erfolgen im wäßrigen oder wäßrigorganischen Medium in Suspension oder Lösung. Führt man die Umsetzungen in einem wäßrig-organischen Medium durch, so ist das organische Medium beispielsweise Aceton, Dimethylformamid, Dimethylsulfoxid oder N-Methylpyrrolidon. Vorteilhaft wird der bei der Kondensation freiwerdende Halogenwasserstoff laufend durch Zugabe wäßriger Alkali- oder Erdalkalihydroxide, -carbonate oder -bicarbonate neutralisiert. Die erste Kondensationsreaktion des Halogen-s-triazins der allgemeinen Formel (21) erfolgt in der Regel bei einer Temperatur zwischen -5°C und +20°C, im Falle von Hal gleich Fluor bevorzugt bei einer Temperatur zwischen -5°C und +5°C, und bei einem pH-Wert zwischen 2 und 10, bevorzugt zwischen 4 und 6. Die nachfolgende Kondensationsreaktion mit der zweiten Aminoverbindung erfolgt in der Regel bei einer Temperatur zwischen 0 und 60°C, im Falle von Hal gleich Fluor bevorzugt bei einer Temperatur zwischen 0 und 20°C, und bei einem pH-Wert zwischen 3 und 10, bevorzugt zwischen 6 und 10.

Insbesondere erfolgt die Umsetzung zwischen einer Verbindung der allgemeinen Formel (23) und einer Verbindung der allgemeinen Formel (22) bei einer Temperatur zwischen 0 und 50°C, bevorzugt zwischen 10 und 30°C, und bei einem pH-Wert zwischen 3 und 10, bevorzugt zwischen 6 und 8, wobei man im Falle, daß in der Verbindung für Formel (23) Hal ein Fluoratom ist, bevorzugt die Umsetzung bei einer Temperatur zwischen 0 und 20°C durchführt.

Ebenfalls erfolgt die Umsetzung einer Verbindung der allgemeinen Formel (24) mit einer Verbindung der allgemeinen Formel (20) bei einer Temperatur zwischen 0 und 40°C und bei einem pH-Wert zwischen 3 und 10, wobei im Falle des Einsatzes einer Verbindung der allgemeinen Formel (24) mit Hal gleich Fluor bevorzugt die Umsetzung bei einer Temperatur zwischen 0 und 20°C und einem pH-Wert zwischen 7 und 10 durchgeführt wird.

Die Ausgangsverbindungen der allgemeinen Formel (23) lassen sich, ausgehend von den Verbindungen (20), in an und für sich bekannter Verfahrensweise der Umsetzung von halogensubstituierten Triazinen mit aminogruppenhaltigen Verbindungen herstellen, wie dies beispielsweise oben für die erfindungsgemäßen Verfahrensweisen beschrieben ist, so ebenfalls in wäßrigem oder wäßrig-organischem Medium in der Regel bei einer Temperatur zwischen -5°C und +40°C, bevorzugt zwischen 0 und 30°C, und bei einem pH-Wert zwischen 2 und 10, bevorzugt zwischen 5 und 7, wobei man im Falle einer Ausgangsverbindung der Formel (21) mit Hal gleich Fluor bevorzugt eine Reaktionstemperatur zwischen -5°C und +5°C wählt. In gleicher Weise und unter den gleichen Verfahrensbedingungen lassen sich die Ausgangsverbindungen der allgemeinen Formel (24) durch Umsetzung einer Verbindung der allgemeinen Formel (21) mit einer Verbindung der allgemeinen Formel (22) herstellen.

Geht man zur Herstellung der erfindungsgemäßen Farbstoffe der allgemeinen Formel (1) von Ausgangsverbindungen der allgemeinen Formel (23) aus, in welcher X ein Rest der allgemeinen Formel -N(R)-W-SO₂-Y der obigen Bedeutung ist, so erfolgt die Umsetzung mit der Aminoverbindung der allgemeinen Formel (22) bei einer Temperatur zwischen 60 und 95°C, vorzugsweise zwischen 80 und 90°C, und bei einem pH-Wert zwischen 6 und 10, bevorzugt zwischen 8 und 9,5, sofern Hal Chlor bedeutet und Y sowohl im Aminorest X als auch in der Ausgangsverbindung der Formel (22) Hydroxy bedeutet; die so erhaltene Farbstoffverbindung mit β-Hydroxyethylsulfonyl-Gruppen kann anschließend durch Veresterung in einen erfindungsgemäßen Farbstoff übergeführt werden, beispielsweise in die β-Sulfatoethylsulfonyl-Farbstoffe mittels Schwefelsäure oder Schwefeltrioxid enthaltende Schwefelsäure, vorzugsweise bei einer Temperatur zwischen 20 und 40°C. Ist in den genannten Ausgangsverbindungen der Formel (23) mit X gleich einem Rest der Formel -N(R)-W-SO₂-CH₂-CH₂-OH mit R und W der obengenannten Bedeutung Hal gleich Fluor, so erfolgt die Umsetzung mit der Aminoverbindung der allgemeinen Formel (22) bei einer Temperatur zwischen 25 und 70°C, vorzugsweise zwischen 40 und 60°C, und bei einem pH-Wert zwischen 3 und 8, bevorzugt zwischen 4 und 7.

Die Triphendioxazinverbindungen der allgemeinen Formel (15) werden, sofern X gleich Chlor ist, bevorzugt in der Weise hergestellt, daß man eine Verbindung der allgemeinen Formel (23A) 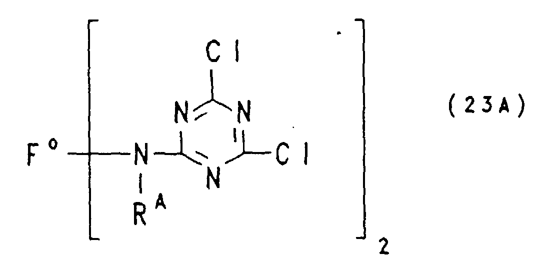 in welcher R^{A} die obengenannte Bedeutung hat und F^{o} ein Rest der allgemeinen Formel (15a) 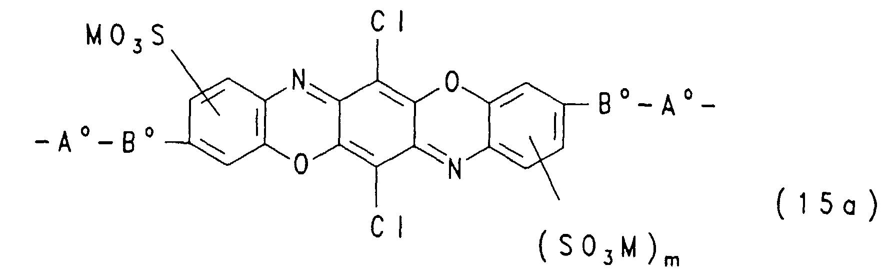 mit M, A^{o} und B^{o} der obengenannten Bedeutung ist, mit einer Aminoverbindung der allgemeinen Formel (22) obengenannter Definition in zweifach molarer Menge bei einer Temperatur zwischen 40 und 60°C und einem pH-Wert zwischen 3 und 10, bevorzugt bei einem pH-Wert zwischen 4 und 5, umsetzt. Geht man von Triphendioxazinverbindungen entsprechend der allgemeinen Formel (23) aus, in welcher F ein Rest der Formel (15a), n gleich 2 und Hal gleich Fluor ist und R^{A} die obengenannte Bedeutung besitzt, erfolgt die Umsetzung mit dem Amin (22) bevorzugt bei einer Temperatur zwischen 0 und 20°C und einem pH-Wert zwischen 6 und 10.

Die Ausgangsverbindungen der allgemeinen Formel (21) und der allgemeinen Formel (20) und deren Vorprodukte sind allgemein bekannt und in der Literatur zahlreich beschrieben. Verbindungen der allgemeinen Formel (21) sind beispielsweise Cyanurchlorid und Cyanurfluorid.

Aromatische Amine, die als Diazokomponenten zur Synthese der erfindungsgemäßen Azofarbstoffe der allgemeinen Formel (1) dienen und den allgemeinen Formeln D¹-NH₂ entsprechen, sind beispielsweise:
1-Amino-2-, -3- oder -4-methoxybenzol, 1-Amino-2-, -3- oder -4-chlorbenzol, 1-Amino-2,5-dichlorbenzol, 1-Amino-2,5-dimethylbenzol, 1-Amino-3-methyl-6-methoxybenzol, 1-Amino-2-methoxy-4-nitrobenzol, 4-Aminodiphenyl, 1-Aminobenzol-2-, -3- oder -4-carbonsäure, 2-Aminodiphenylether, 1-Aminobenzol-2-, -3- oder -4-sulfonsäureamid, -N-methylamid, -N-ethylamid, -N,N-dimethylamid oder -N,N-diethylamid, Dehydrothio-p-toluidin-sulfonsäure, 1-Amino-3-trifluormethyl-6-sulfonsäure, 1-Amino-3- oder -4-nitrobenzol, 1-Amino-3- oder -4-acetylaminobenzol, 1-Aminobenzol-2-, -3- oder -4-sulfonsäure, 1-Aminobenzol-2,4- und -2,5-disulfonsäure, 1-Amino-4-methylbenzol-2-sulfonsäure, 1-Amino-3-methylbenzol-6-sulfonsäure, 1-Amino-6-methylbenzol-3- oder -4-sulfonsäure, 1-Amino-2-carboxybenzol-4-sulfonsäure, 1-Amino-4-carboxybenzol-2-sulfonsäure, 1-Amino-4- oder -5-chlorbenzol-2-sulfonsäure, 1-Amino-6-chlorbenzol-3- oder -4-sulfonsäure, 1-Amino-3,4-dichlorbenzol-6-sulfonsäure, 1-Amino-2,5-dichlorbenzol-6-sulfonsäure, 1-Amino-2,5-dichlorbenzol-4-sulfonsäure, 1-Amino-4-methyl-5-chlorbenzol-2-sulfonsäure, 1-Amino-5-methyl-4-chlorbenzol-2-sulfonsäure, 1-Amino-4-- oder -5-methoxybenzol-2-sulfonsäure, 1-Amino-6-methoxybenzol-3- oder -4-sulfonsäure, 1-Amino-6-ethoxybenzol-3- oder -4-sulfonsäure, 1-Amino-2,4-dimethoxybenzol-6-sulfonsäure, 1-Amino-2,5-dimethoxybenzol-4-sulfonsäure, 1-Amino-3-acetylaminobenzol-6-sulfonsäure, 1-Amino-4-acetylaminobenzol-2-sulfonsäure, 1-Amino-3-acetylamino-4-methylbenzol-6-sulfonsäure, 2-Amino-1-methylbenzol-3,5-disulfonsäure, 1-Amino-4-methoxybenzol-2,5-disulfonsäure, 1,4-Diaminobenzol-2,5-disulfonsäure, 1-Amino-3- oder -4-nitrobenzol-6-sulfonsäure, 1-Aminonaphthalin, 2-Aminonaphthalin, 1-Aminonaphthalin-2-, -4-, -5-, -6-, -7- oder -8-sulfonsäure, 1-Aminonaphthalin-3,6- oder -5,7-disulfonsäure, 2-Amino-naphthalin-1-sulfonsäure, 2-Aminonaphthalin-1,5-, -1,7-, -3,6-, -5,7-, -4,8- oder -6,8-disulfonsäure, 1-Aminonaphthalin-2,5,7-trisulfonsäure, 2-Aminonaphthalin-1,5,7-, 3,6,8- oder -4,6,8-trisulfonsäure, 1-Hydroxy-2-aminobenzol-4-sulfonsäure, 1-Hydroxy-2-aminobenzol-5-sulfonsäure, 1-Hydroxy-2-aminobenzol-4,6-disulfonsäure, 1-Hydroxy-2-amino-4-acetylaminobenzol-6-sulfonsäure, 1-Hydroxy-2-amino-6-acetylaminobenzol-4-sulfonsäure, 1-Hydroxy-2-amino-4-chlorbenzol-5-sulfonsäure, 1-Hydroxy-2-amino-4-methylsulfonylbenzol, 1-Amino-2-hydroxy-6-nitronaphthalin-4-sulfonsäure, 2-Amino-1-hydroxynaphthalin-4,8-disulfonsäure, 4-Aminoazobenzol-3,4'-disulfonsäure, 3-Methoxy-4-amino-6-methylazobenzol-2',4'-disulfonsäure, 3-Methoxy-4-amino-6-methylazobenzol-2',5'-disulfonsäure, 2-(β-Sulfatoethylsulfonyl)-anilin, 3-(β-Sulfatoethylsulfonyl)-anilin, 4-(β-Sulfatoethylsulfonyl)-anilin, 2-Carboxy-5-(β-sulfatoethylsulfonyl)-anilin, 2-Chlor-3-(sulfatoethylsulfonyl)-anilin, 2-Chlor-4-(β-sulfatoethylsulfonyl)-anilin, 2-Brom-4-(β-sulfatoethylsulfonyl)-anilin, 4-Methoxy-3-(β-sulfatoethylsulfonyl)-anilin, 4-Chlor-3-(β-sulfatoethylsulfonyl)-anilin, 2-Ethoxy-4- oder -5-(β-sulfatoethylsulfonyl)-anilin, 2-Methyl-4-(β-sulfatoethylsulfonyl)-anilin, 2-Methoxy-5- oder -4-(β-sulfatoethylsulfonyl)-anilin, 2,4-Diethoxy-5-(β-sulfatoethylsulfonyl)-anilin, 2,4-Dimethoxy-5-(β-sulfatoethylsulfonyl)-anilin, 2,5-Dimethoxy-4-(β-sulfatoethylsulfonyl)-anilin, 2-Methoxy-5-methyl-4-(β-sulfatoethylsulfonyl)-anilin, 2- oder 3- oder 4-(β-Thiosulfatoethylsulfonyl)-anilin, 2-Methoxy-5-(β-thiosulfatoethylsulfonyl)-anilin, 2-Sulfo-4-(β-phosphatoethylsulfonyl)-anilin, 2-Sulfo-4-vinylsulfonyl-anilin, 2-Hydroxy-4- oder -5-(β-sulfatoethylsulfonyl)-anilin, 2-Chlor-4- oder -5-(β-chlorethylsulfonyl)-anilin, 2-Hydroxy-5-(β-sulfatoethylsulfonyl)-anilin, 2-Hydroxy-3-sulfo-5-(β-sulfatoethylsulfonyl)-anilin, 3- oder 4-(β-Acetoxyethylsulfonyl)-anilin, 6-Carboxy-1-sulfo-2-aminonaphthalin, 5-(β-Sulfatoethylsulfonyl)-2-aminonaphthalin, 6- oder 7- oder 8-(β-Sulfatoethylsulfonyl)-2-aminonaphthalin, 6-(β-Sulfatoethylsulfonyl)-1-sulfo-2-aminonaphthalin, 5-(β-Sulfatoethylsulfonyl)-1-sulfo-2-aminonaphthalin, 8-(β-Sulfatoethylsulfonyl)-6-sulfo-2-aminonaphthalin, 4-[N-Methyl-N-(β-sulfatoethylsulfonyl)]-amino-anilin, 3-[N-Methyl-N-(β-sulfatoethylsulfonyl)]-amino-anilin, 4-[β-(β'-Sulfatoethylsulfonyl)-ethyl]-anilin, 3- oder 4-[β-(β'-Chlorethylsulfonyl)-ethylamino]-anilin, 3- oder 4-[β-(β'-Sulfatoethylsulfonyl)-ethylamino]-anilin, 3- oder 4-[γ-(β'-Chlorethylsulfonyl)-propylamino]-anilin, 3- oder 4-[γ-(β'-Sulfatoethylsulfonyl)-propylamino]-anilin und 3- oder 4-[γ-(Vinylsulfonyl)-propylamino]-anilin.

Ausgangsverbindungen entsprechend der allgemeinen Formel H₂N-D²-NH₂ sind beispielsweise 1,4-Phenylen-diamin, 1,4-Phenylendiamin-2-sulfonsäure, 1,4-Phenylendiamin-2-carbonsäure, 1,4-Diamino-naphthalin-2-sulfonsäure, 2,6-Diamino-naphthalin-8-sulfonsäure, 2,6-Diamino-naphthalin-4,8-disulfonsäure, 1,3-Phenylen-diamin, 1,3-Phenylendiamin-4-sulfonsäure, 1,3-Phenylen-diamin-4,6-disulfonsäure, 1,4-Phenylendiamin-2,6-disulfonsäure, 1,4-Phenylendiamin-2,5-disulfonsäure, 1,4-Diamino-naphthalin-6-sulfonsäure, 4,4'-Diamino-diphenyl-3-sulfonsäure und 4,4'-Diamino-stilben-2,2'-disulfonsäure.

Ausgangsverbindungen, die zur Herstellung von erfindungsgemäßen Disazofarbstoffen der allgemeinen Formel (1) zunächst als Kupplungskomponente und sodann, in Form der gebildeten Amino-Azoverbindung, als Diazokomponente dienen und der allgemeinen Formel H-E-NH₂ entsprechen, sind beispielsweise Anilin, 3-Methyl-anilin, 2-Methoxy-5-methyl-anilin, 2,5-Dimethyl-anilin, 3-Ureido-anilin, 3-Acetylamino-anilin, 3-Propionylamino-anilin, 3-Butyrylamino-anilin, 3-Methoxy-anilin, 2-Methyl-5-acetylamino-anilin, 2-Methoxy-5-acetylamino-anilin, 2-Methoxy-5-methyl-anilin, 3-(Hydroxyacetylamino)-anilin, 1,3-Diaminobenzol-4-sulfonsäure, 1-Amino-naphthalin-6-, -7- oder -8-sulfonsäure, 1-Amino-2-methoxy-naphthalin-6-sulfonsäure, 2-Amino-5-hydroxy-naphthalin-7-sulfonsäure, 2-Amino-5-hydroxy-naphthalin-1,7-disulfonsäure, 2-Amino-8-hydroxy-naphthalin-6-sulfonsäure, 2-(4'-Amino-benzoylamino)-5-naphthol-7-sulfonsäure, 1-(4'-Amino-2'-sulfo-phenyl)-3-methyl-5-pyrazolon, 1-(4'-Amino-2'-sulfo-phenyl)-3-carboxy-5-pyrazolon und N-(Acetoacetyl)-3-sulfo-4-amino-anilid.

Ausgangsverbindungen zur Herstellung der erfindungsgemäßen Azoverbindungen der allgemeinen Formel (1), die als Kupplungskomponenten dienen können und den allgemeinen Formeln H-K¹ oder H-K²-N(R^{A})H oder H-K-H entsprechen, sind beispielsweise:
Phenol, 1-Hydroxy-3- oder -4-methylbenzol, 1-Hydroxybenzol-4-sulfonsäure, 1-Hydroxynaphthalin, 2-Hydroxynaphthalin, 2-Hydroxynaphthalin-6- oder -7-sulfonsäure, 2-Hydroxynaphthalin-3,6- oder -6,8-disulfonsäure, 1-Hydroxynaphthalin-4-sulfonsäure, 1-Hydroxynaphthalin-4,6- oder -4,7-disulfonsäure, 1-Amino-3-methylbenzol, 1-Amino-2-methoxy-5-methylbenzol, 1-Amino-2,5-dimethylbenzol, 3-Aminophenylharnstoff, 1-Amino-3-acetylaminobenzol, 1-Amino-3-hydroxyacetylaminobenzol, 1,3-Diaminobenzol-4-sulfonsäure, 1-Aminonaphthalin-6- oder -8-sulfonsäure, 1-Amino-2-methoxynaphthalin-6-sulfonsäure, 2-Aminonaphthalin-5,7-disulfonsäure, 1-Amino-8-hydroxynaphthalin-6-sulfonsäure, 1-Amino-8-hydroxynaphthalin-2,4-disulfonsäure, 2-Hydroxy-3-aminonaphthalin-5,7-disulfonsäure, 1-Amino-8-hydroxynaphthalin-2,4,6-trisulfonsäure, 1-Hydroxy-8-acetylaminonaphthalin-3-sulfonsäure, 1-Benzoylamino-8-hydroxynaphthalin-3,6- oder -4,6-disulfonsäure, 2-Benzoylamino-5-hydroxynaphthalin-7-sulfonsäure, 2-Amino-5-hydroxynaphthalin-7-sulfonsäure, 2-Methyl- und 2-Ethylamino-5-hydroxynaphthalin-7-sulfonsäure, 2-(N-Acetyl-N-methylamino)-5-hydroxynaphthalin-7-sulfonsäure, 2-Acetylamino-5-hydroxynaphthalin-7-sulfonsäure, 2-Amino-5-hydroxynaphthalin-1,7-disulfonsäure, 2-Amino-8-hydroxynaphthalin-6-sulfonsäure, 2-Methyl- bzw. Ethylamino-8-hydroxynaphthalin-6-sulfonsäure, 2-(N-Acetyl-N-methylamino)-8-hydroxynaphthalin-6-sulfonsäure, 2-Acetylamino-8-hydroxynaphthalin-6-sulfonsäure, 2-Amino-8-hydroxynaphthalin-3,6-disulfonsäure, 2-Acetylamino-8-hydroxynaphthalin-3,6-disulfonsäure, 1-Amino-5-hydroxynaphthalin-7-sulfonsäure, 1-Amino-8-hydroxynaphthalin-3,6- bzw. -4,6-disulfonsäure, 1-Acetylamino-8-hydroxynaphthalin-3,6- und -4,6-disulfonsäure (1-(4'-Aminobenzoylamino)-8-hydroxynaphthalin-3,6- bzw. -4,6-disulfonsäure, 1-(4'-Nitrobenzoylamino)-8-hydroxynaphthalin-3,6- bzw. -4,6-disulfonsäure, 1-(3'-Aminobenzoylamino)-8-hydroxynaphthalin-3,6- bzw. -4,6-disulfonsäure, 1-(3'-Nitrobenzoylamino)-8-hydroxynaphthalin-3,6- bzw. -4,6-disulfonsäure, 2-(4'-Amino-3'-sulfophenylamino)-5-hydroxynaphthalin-7-sulfonsäure, 3-Methylpyrazolon-(5), 1-Phenyl-3-methyl-5-pyrazolon, 1-(4'-Sulfophenyl)-3-methyl-5-pyrazolon, 1-(4'-Sulfophenyl)-pyrazolon-(5)-3-carbonsäure, 1-(3'-Aminophenyl)-3-methyl-5-pyrazolon, 1-(2',5'-Disulfophenyl)-3-methyl-5-pyrazolon, 1-(2'-Methyl-4'-sulfophenyl)-5-pyrazolon-3-carbonsäure, 1-(4',8'-Disulfonaphthyl-[2']-3-methyl-5-pyrazolon, 1-(5',7'-Disulfonaphthyl-[2']-3-methyl-5-pyrazolon, 1-(2',5'-Dichlor-4'-sulfophenyl)-3-methyl-5-pyrazolon, 3-Aminocarbonyl-4-methyl-6-hydroxypyridon-2, 1-Ethyl-3-cyan- oder -3-chlor-4-methyl-6-hydroxypyridon-2, 1-Ethyl-3-sulfomethyl-4-methyl-6-hydroxypyridon-2, 2,4,6-Triamino-3-cyanpyridin, 2-(3'-Sulfophenylamino)-4,6-diamino-3-cyanpyridin, 2-(2'-Hydroxyethylamino-3-cyan-4-methyl-6-aminopyridin, 2,6-Bis-(2'-hydroxyethylamino)-3-cyan-4-methylpyridin, 1-Ethyl-3-carbamoyl-4-methyl-6-hydroxypyridon-(2), 1-Ethyl-3-sulfomethyl-4-methyl-5-carbamoyl-6-hydroxypyridon-(2), N-Acetoacetylaminobenzol, 1-(N-Acetoacetylamino)-2-methoxybenzol-5-sulfonsäure, 4-Hydroxychinolon-(2), 1-Amino-8-hydroxy-7-(phenylazo)-naphthalin-3,6-disulfonsäure, 1-Amino-8-hydroxy-7-(4'-sulfophenylazo)-naphthalin-3,6-disulfonsäure, 1-Amino-8-hydroxy-7-(2',5'-disulfophenylazo)naphthalin-3,6-disulfonsäure, 1-[4'-(β-Sulfatoethyl-sulfonyl)-phenyl]-3-methyl-5-pyrazolon, 1-[4'-(Vinyl-sulfonyl)-phenyl]-3-methyl-5-pyrazolon, 1-[4'-(β-Sulfatoethylsulfonyl)-phenyl]-3-carboxy-5-pyrazolon, 1-[3'-[β-Chlorethylsulfonyl)-benzoylamino]-3,6-disulfo-8-naphthol, 1-[3'-(Vinylsulfonyl)-benzoylamino]-3,6-disulfo-8-naphthol, 1-[3'-(Vinylsulfonyl)-benzoylamino]-4,6-disulfo-8-naphthol, 1-[3'-(β-Sulfatoethylsulfonyl)-benzoylamino]-4,6-disulfo-8-naphthol, 2-[3'-(β-Chlorethylsulfonyl)-benzoylamino]-6-sulfo-8-naphthol, 2-[3'-(Vinylsulfonyl)-benzoylamino]-6-sulfo-8-naphthol, 3-[3'-(β-Chlorethylsulfonyl)-benzoylamino]-6-sulfo-8-naphthol, 3-[3'-(Vinylsulfonyl)-benzoyl-amino]-6-sulfo-8-naphthol, 6-Sulfo-1-[3'-(β-chlorethylsulfonyl)-benzoylamino]-naphthol, 7-Sulfo-[3'-(vinylsulfonyl)-benzoylamino]-naphthol, 2-[N-Methyl-N-(β-sulfatoethylsulfonyl)-amino]-6-sulfo-8-naphthol, 3-[N-Methyl-N-(β-sulfatoethylsulfonyl)-amino]-6-sulfo-8-naphthol, 2-[N-Ethyl-N-(β-sulfatoethylsulfonyl)-amino]-6-sulfo-8-naphthol, 1-[N'-(3'-β-Chlorethylsulfonyl-phenyl)-ureido]-3,6-disulfo-8-naphthol, 1-[N'-(3'-Vinylsulfonyl-phenyl)-ureido]-3,6-disulfo-8-naphthol, 1-[N'-(3'-Vinylsulfonyl-propyl)-ureido]-3,6-disulfo-8-naphthol, 1-[N'-(3'-β-Chlor-ethylsulfonyl-phenyl)-ureido]-4,6-disulfo-8-naphthol, 1-[N'-(3'-Vinylsulfonyl-phenyl)-ureido]-4,6-disulfo-8-naphthol, 1-[N'-(3'-β-Chlorethylsulfonyl-propyl)-ureido]-4,6-disulfo-8-naphthol, 2-[N'-(3'-β-Sulfatoethylsulfonyl-phenyl)-ureido]-6-sulfo-8-naphthol, 2-[N'-(3'-Chlorethylsulfonyl-propyl)-ureido]-6-sulfo-8-naphthol, 3-[N'-(3'-β-Chlorethylsulfonyl-phenyl)-ureido]-6-sulfo-8-naphthol, 3-[N'-(3'-Vinylsulfonyl-propyl)-ureido]-6-sulfo-8-naphthol, 2-Sulfo-5-[N'-(3''-β-chlorethylsulfonyl)-phenyl]-ureido-anilin, 3-[N'-(3''-β-Sulfatoethylsulfonyl)-phenyl]-ureido-anilin und 6-Sulfo-1-[N'-(3''-β-Sulfatoethylsulfonyl)-phenyl]-ureido-8-naphthol.

Kupplungskomponenten entsprechend der allgemeinen Formel H-K-N(R^{A})H , die zum Aufbau der erfindungsgemäßen Azofarbstoffe dienen können, in welchen der faserreaktive Rest Z in der Kupplungskomponente enthalten ist, wobei bei dem zunächst erhaltenen aminogruppenhaltigen Azofarbstoff entsprechend der allgemeinen Formel (20) in dessen Aminogruppe -N(R^{A})H der faserreaktive Rest Z¹ anschließend eingeführt werden kann bzw. wird, sind beispielsweise Anilin, 3-Methyl-anilin, 2,5-Dimethyl-anilin, 2,5-Dimethoxy-anilin, 3-Methoxy-anilin, 3-Acetylamino-anilin, 3-Propionylamino-anilin, 3-Butyrylamino-anilin, 3-Benzoylamino-anilin, 3-(Hydroxyacetylamino)-anilin, 3-Ureido-anilin, 2-Methyl-5-acetylamino-anilin, 2-Methoxy-5-acetylamino-anilin, 2-Methoxy-5-methyl-anilin, 1-Amino-naphthalin-6-sulfonsäure, 1-Amino-naphthalin-7-sulfonsäure, 4-Sulfo-1,3-diamino-benzol, 6-Sulfo-2-methoxy-1-amino-naphthalin, 5,7-Disulfo-2-aminonaphthalin, 1-Amino-8-hydroxy-naphthalin-4-sulfonsäure, 1-Amino-8-hydroxynaphthalin-6-sulfonsäure, 1-Amino-8-hydroxynaphthalin-2,4-disulfonsäure, 1-Amino-8-hydroxy-naphthalin-2,4,6-trisulfonsäure, 2-(Methylamino)- und 2-(Ethylamino)-5-hydroxynaphthalin-7-sulfonsäure, 2-Amino-5-hydroxynaphthalin-1,7-disulfonsäure, 2-(Methylamino)- und 2-(Ethylamino)-8-hydroxynaphthalin-6-sulfonsäure, 2-Amino-8-hydroxynaphthalin-3,6-disulfonsäure, 2-(4'-Amino-3'-sulfophenylamino)-5-hydroxynaphthalin-7-sulfonsäure, 1-Amino-8-hydroxy-2-(phenylazo)-naphthalin-3,6-disulfonsäure, 1-Amino-8-hydroxy-2-(4'-sulfophenylazo)-naphthalin-3,6-disulfonsäure, 1-Amino-8-hydroxy-2-(2',5'-disulfophenylazo)-naphthalin-3,6-disulfonsäure, 1-(β-Aminoethyl)-3-cyano-4-methyl-6-hydroxy-pyrid-2-on, 1-(γ-Aminopropyl)-3-sulfomethyl-4-methyl-6-hydroxy-pyrid-2-on, 1,3-Diaminobenzol, 3-[N,N-Di-(β-hydroxyethyl)]-amino-anilin, 3-[N,N-Di-(β-sulfatoethyl)]-amino-4-methoxy-anilin, 3-(Sulfo-benzylamino)-anilin, 3-(Sulfobenzoylamino)-4-chlor-anilin und 3-[N,N-Di-(sulfobenzyl)]-amino-anilin, 2-Sulfo-5-acetylamino-anilin, 2-Amino-5-naphthol-7-sulfonsäure, 2-Amino-8-naphthol-6-sulfonsäure, 1-(4'-Aminobenzoyl)-amino-8-hydroxynaphthalin-3,6-disulfonsäure, 1-(4'-Aminobenzoyl)-amino-8-hydroxynaphthalin-4,6-disulfonsäure, 1-(3'-Aminobenzyl)-amino-8-hydroxynaphthalin-3,6-disulfonsäure, 1-(3'-Aminobenzoyl)-amino-8-hydroxynaphthalin-4,6-disulfonsäure, 1-(2'-Aminobenzoyl)-amino-8-hydroxy-naphthalin-3,6-disulfonsäure, 1-(2'-Aminobenzoyl)-amino-8-hydroxy-naphthalin-4,6-disulfonsäure, 2-(3'-Aminobenzoyl)-amino-5-hydroxy-naphthalin-7-sulfonsäure, 2-(2'-Aminobenzoyl)-amino-5-hydroxy-naphthalin-7-sulfonsäure, 2-(4'-Aminobenzoyl)-amino-8-hydroxynaphthalin-6-sulfonsäure, 2-(3'-Aminobenzoyl)-amino-8-hydroxynaphthalin-6-sulfonsäure, 2-(2'-Aminobenzoyl)-amino-8-hydroxynaphthalin-6-sulfonsäure, 2-(4'-Aminobenzoyl)-amino-5-naphthol-7-sulfonsäure, 1-(4'-Amino- oder 1-(4'-Acetylamino-2-sulfophenyl)-3-methyl- oder 3-carboxy-5-pyrazolon, 3-Sulfo-4-amino-acetoacetylanilid, 1-Amino-8-naphthol-3,6- oder -4,6-disulfonsäure, 1-(3'-Aminobenzoylamino)- oder 1-(4'-Aminobenzoylamino)-8-naphthol-3,6- oder -4,6-disulfonsäure, 1-Acetylamino-8-naphthol-3,6- oder -4,6-disulfonsäure, 2-Acetylamino-5-naphthol-7-sulfonsäure, 2-Acetylamino-8-naphthol-6-sulfonsäure, 3-Acetylamino-8-naphthol-6-sulfonsäure, 3-(N-Methylamino)-8-naphthol-6-sulfonsäure, 1-(3'-Amino- oder 1-(3'-Acetylamino-6'-sulfophenyl)-3-methyl- oder -3-carboxy-5-pyrazolon, 2-(N-Methyl-N-acetylamino)- oder 2-Methylamino-5-naphthol-7-sulfonsäure, N-Methyl-anilin und N-Propyl-m-toluidin.

Geht man von Diazokomponenten der Formel H₂N-D²-NH₂ aus, so können diese auch in der Form der Monoacylamino-amino-Verbindungen eingesetzt werden, wobei der Acylrest insbesondere der Acetylrest ist. Diese Monoacylamino-amino-Verbindungen werden zunächst diazotiert und mit einer kuppelfähigen Verbindung gekuppelt; anschließend wird der Acylrest hydrolytisch abgespalten, und die so nunmehr wieder frei gewordene Aminogruppe kann mit dem faserreaktiven Rest Z¹ verbunden werden. Solche monoacylierten Diamine sind beispielsweise 2-Sulfo-5-acetylamino-anilin und 2-Sulfo-4-acetylamino-anilin. In gleicher Weise können aminogruppenhaltige Kupplungskomponenten in Form des Acylamino-Derivates in die Kupplungsreaktion eingesetzt werden, wobei anschließend auch hier der Acylrest hydrolytisch abgespalten werden kann, um die freiwerdende Aminogruppe mit der faserreaktiven Gruppe Z¹ zu verbinden.

Bivalente Kupplungskomponenten, die zum Aufbau erfindungsgemäßer Disazofarbstoffe dienen können, in welchen der bivalente Kupplungsrest mit zwei Diazokomponenten verbunden ist, von denen die eine oder beide einen faserreaktiven Rest Z enthalten, beispielsweise von Farbstoffen der allgemeinen Formel (3d), sind beispielsweise Resorcin, 1,3-Diaminobenzol, 5,5'-Dihydroxy-7,7'-disulfo-2,2'-dinaphthyl-harnstoff, 1,8-Dihydroxy-3,6-disulfo-naphthalin und insbesondere 1-Amino-8-naphthol-3,6-disulfonsäure und 1-Amino-8-naphthol-4,6-disulfonsäure.

Geht man bei der erfindungsgemäßen Synthese der Azofarbstoffe von Diazo- oder Kupplungskomponenten aus, die bereits die Gruppe der allgemeinen Formel (2) enthalten, erfolgen die Umsetzungen in der üblichen Verfahrensweise der Diazotierungs- und Kupplungsreaktionen, so die Diazotierung in der Regel bei einer Temperatur zwischen -5°C und +15°C und einem pH-Wert unterhalb von 2 mittels einer starken Säure und Alkalinitrit in bevorzugt wäßrigem Medium und die Kupplungsreaktion in der Regel bei einem pH-Wert zwischen 1,5 und 4,5 im Falle einer aminogruppenhaltigen Kupplungskomponente und bei einem pH-Wert zwischen 3 und 7,5 im Falle einer hydroxygruppenhaltigen Kupplungskomponente und bei einer Temperatur zwischen 0 und 25°C, ebenso bevorzugt in wäßrigem Medium.

Bei der erfindungsgemäßen Synthese von Schwermetallkomplex-Azofarbstoffen, beispielsweise solchen entsprechend der allgemeinen Formel (3g) und (3h), geht man in der Regel von solchen schwermetallfreien Azoverbindungen aus, die in der Kupplungskomponente eine phenolische oder naphtholische Hydroxygruppe in ortho-Stellung bzw. vicinaler Stellung zur Azogruppe gebunden enthalten und deren Diazokomponentenrest in ortho-Stellung zur Azogruppe ein Wasserstoffatom oder eine Hydroxygruppe oder eine niedere Alkoxygruppe, wie Methoxygruppe, gebunden enthält, wobei die schwermetallfreien Ausgangs-Azoverbindungen zudem einen Acylaminorest gebunden enthalten können, wie einen Acetylaminorest. Beispielsweise kann man bei der Synthese der Kupferkomplex-Azofarbstoffe der Formel (3h) von einer Ausgangsverbindung entsprechend der allgemeinen Formel (25) 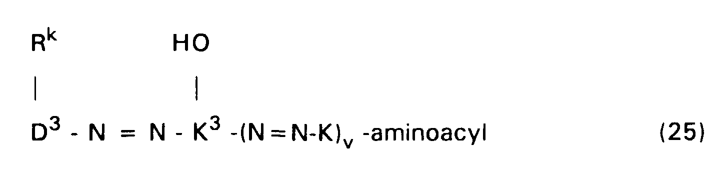 ausgehen, in welcher D³, K, K³ und v eine der obengenannten Bedeutungen haben und R^{k} ein Wasserstoffatom oder eine in ortho-Stellung zur Azogruppe an D³ gebundene Hydroxy- oder Methoxygruppe ist, und diese acylaminogruppenhaltige Ausgangs-Azoverbindung analog bekannten und üblichen Verfahrensweisen mit einem kupferabgebenden Mittel, wie einem Kupfersalz, umsetzen. Ist R^{k} ein Wasserstoffatom oder eine Methoxygruppe, so kann man die Verbindung (25) einer auf üblichen Wege durchzuführenden oxidativen oder entalkylierenden Kupferungsreaktion unterwerfen. Die nun erhaltene Kupferkomplex-Azoverbindung mit der Acylaminogruppe kann sodann analog bekannten Verfahrensweisen nach oder unter Hydrolyse der Acylaminogruppe zur Aminogruppe mit einer Verbindung der Formel (21) oder (24) zu dem erfindungsgemäßen Farbstoff der allgemeinen Formel (1) umgesetzt werden.

Die Ausgangsverbindungen der allgemeinen Formel (22) sind teilweise bekannt, so bspw. aus einigen der anfangs genannten Schriften des Standes der Technik und der Britischen Patentschrift Nr. 1 576 237.

Ausgangsverbindungen der allgemeinen Formel (22) sind beispielsweise N-Phenyl-N-(γ-vinylsulfonyl-propyl)-amin, N-(4-Chlorphenyl)-N-(γ-vinylsulfonylpropyl)-amin, N-(2-Methylphenyl)-N-(γ-vinylsulfonyl-propyl)-amin, N-(4-Methoxyphenyl)-N-(γ-vinylsulfonyl-propyl)-amin, N-(3-Sulfophenyl)-N-(γ-vinylsulfonyl-propyl)-amin, N-(4-Sulfophenyl)-N-(γ-vinylsulfonyl-propyl)-amin, N-Phenyl-N-[γ-(β'-sulfatoethylsulfonyl)-propyl]-amin, N-(4-Chlorphenyl)-N-[γ-(β'-sulfatoethylsulfonyl)-propyl]-amin, N-(2-Methylphenyl)-N-[γ-(β'-sulfatoethylsulfonyl)-propyl]-amin, N-(4-Methoxyphenyl)-N-[γ-(β'-sulfatoethylsulfonyl)-propyl]-amin, N-(3-Sulfophenyl)-N-[γ-(β'-sulfatoethylsulfonyl)-propyl]-amin, N-(4-Sulfophenyl)-N-[γ-(β'-sulfatoethylsulfonyl)-propyl]-amin, bevorzugt hiervon insbesondere N-Phenyl-[γ-(β'-sulfatoethylsulfonyl)-propyl]-amin.

Die Verbindungen der allgemeinen Formel (22), in welcher W die n-Propylen-Gruppe ist und z für die Zahl 1 steht, sind bisher noch nicht beschrieben und somit neu. Die vorliegende Erfindung betrifft deshalb auch Verbindungen entsprechend der allgemeinen Formel (22A) 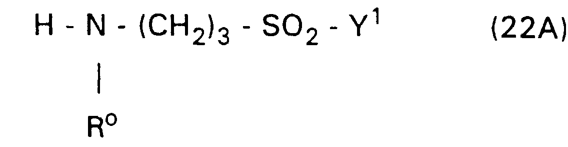 in welcher Y¹ eine der für Y angegebenen, insbesondere bevorzugten, Bedeutungen besitzt oder die β-Hydroxyethyl-Gruppe ist und R^{o} für Phenyl steht, das durch 1, 2 oder 3, bevorzugt 1 oder 2, Substituenten aus der Gruppe Halogen, wie Chlor und Brom, Alkyl von 1 bis 4 C-Atomen, wie Methyl, Alkoxy von 1 bis 4 C-Atomen, wie Methoxy und Ethoxy, Sulfo und Carboxy, bevorzugt Methyl, Methoxy und Sulfo, substituiert sein kann. Die Erfindung betrifft auch die Salze dieser Aminoverbindungen der allgemeinen Formel (22), insbesondere hiervon die Salze von starken anorganischen Säuren, wie der Salzsäure und der Schwefelsäure.

Weiterhin betrifft die Erfindung die Verwendung der Verbindungen der allgemeinen Formel (22A) und deren Salze als Ausgangsverbindungen zur Synthese von faserreaktiven Verbindungen, insbesondere Farbstoffen, wie beispielsweise von Farbstoffen der allgemeinen Formel (1).

Die Aminoverbindungen der allgemeinen Formel (22A) lassen sich beispielsweise in der Weise herstellen, daß man N-Allyl-N-acetyl-anilin (s. J. Org. Chem. 14, 1099 (1949)) analog der in der deutschen Offenlegungsschrift Nr. 41 06 106 beschriebenen Verfahrensweise mit 2-Mercapto-ethanol in Gegenwart eines Radikalinitiators umsetzt, die erhaltene N-[γ-(β'-Hydroxyethylthio)-propyl]-N-acetyl-anilin-Verbindung zur Sulfonylverbindung oxidiert, beispielsweise mittels Wasserstoffperoxid in Gegenwart einer katalytischen Menge einer Übergangsmetallverbindung, wie beispielsweise Wolframoxid, und aus der so erhaltenen Sulfonylverbindung die Acetylgruppe im alkalischen oder sauren Bereich, vorzugsweise in salzsaurer wäßriger Lösung, wie beispielsweise in 5 bis 30 %iger wäßriger Salzsäure, bei einer Temperatur zwischen 80 und 100°C hydrolytisch abspaltet.

Das so erhaltene N-Phenyl-N-[γ-(β'-hydroxyethylsulfonyl)-propyl]-amin kann aus der neutral gestellten wäßrigen Syntheselösung von der wäßrigen Phase abgetrennt werden. Dessen β-Hydroxyethylsulfonyl-Gruppe läßt sich nach üblichen Methoden verestern, so beispielsweise mittels konzentrierter Schwefelsäure bei einer Temperatur zwischen 10 und 30°C in die β-Sulfatoethylsulfonyl-Verbindung überführen.

Erfindungsgemäße Verbindungen der allgemeinen Formel (22A) sind beispielsweise N-Phenyl-N-(γ-vinylsulfonyl-propyl)-amin, N-(4-Chlorphenyl)-N-(γ-vinylsulfonyl-propyl)-amin, N-(2-Methylphenyl)-N-(γ-vinylsulfonyl-propyl)-amin, N-(4-Methoxyphenyl)-N-(γ-vinylsulfonyl-propyl)-amin, N-(3-Sulfophenyl)-N-(γ-vinylsulfonyl-propyl)-amin, N-(4-Sulfophenyl)-N-(γ-vinylsulfonyl-propyl)-amin, N-Phenyl-N-[γ-(β'-sulfatoethylsulfonyl)-propyl]-amin, N-(4-Chlorphenyl)-N-[γ-(β'-sulfatoethylsulfonyl)-propyl]-amin, N-(2-Methylphenyl)-N-[γ-(β'-sulfatoethylsulfonyl)-propyl]-amin, N-(4-Methoxyphenyl)-N-[γ-(β'-sulfatoethylsulfonyl)-propyl]-amin, N-(3-Sulfophenyl)-N-[γ-(β'-sulfatoethylsulfonyl)-propyl]-amin und N-(4-Sulfophenyl)-N-[γ-(β'-sulfatoethylsulfonyl)-propyl]-amin sowie deren β-Hydroxyethylsulfonyl-Derivate.

Die Abscheidung der erfindungsgemäß hergestellten Farbstoffe der allgemeinen Formel (1) aus den Syntheseansätzen erfolgt nach allgemein bekannten Methoden entweder durch Ausfällen aus dem Reaktionsmedium mittels Elektrolyten, wie beispielsweise Natriumchlorid oder Kaliumchlorid, oder durch Eindampfen der Reaktionslösung, beispielsweise durch Sprühtrocknung, wobei dieser Reaktionslösung eine Puffersubstanz zugeführt werden kann.

Die Farbstoffe der allgemeinen Formel (1) - im nachfolgenden Farbstoffe (1) genannt - eignen sich zum Färben und Bedrucken der verschiedensten Materialien, wie Seide, Leder, Wolle, Polyamidfasern und Polyurethanen, und insbesondere cellulosehaltiger Fasermaterialien aller Art. Solche Fasermaterialien sind beispielsweise die natürlichen Cellulosefasern, wie Baumwolle, Leinen und Hanf, sowie Zellstoff und regenerierte Cellulose. Die Farbstoffe (1) sind auch zum Färben oder Bedrucken von hydroxygruppenhaltigen Fasern geeignet, die in Mischgeweben enthalten sind, z.B. von Gemischen aus Baumwolle mit Polyesterfasern oder Polyamidfasern.

Die Farbstoffe (1) lassen sich auf verschiedene Weise auf das Fasermaterial applizieren und auf der Faser fixieren, insbesondere in Form von wäßrigen Farbstofflösungen und -druckpasten. Sie eignen sich sowohl für das Ausziehverfahren als auch zum Färben nach dem Foulard-Färbeverfahren, wonach die Ware mit wäßrigen, gegebenenfalls salzhaltigen Farbstofflösungen imprägniert und der Farbstoff nach einer Alkalibehandlung oder in Gegenwart von Alkali, gegebenenfalls unter Wärmeeinwirkung, fixiert wird. Besonders geeignet sind die erfindungsgemäßen Farbstoffe für das sogenannte Kaltverweilverfahren, wonach der Farbstoff zusammen mit dem Alkali auf dem Foulard aufgebracht und danach durch mehrstündiges Lagern bei Raumtemperatur fixiert wird. Nach dem Fixieren werden die Färbungen oder Drucke mit kaltem und heißem Wasser, gegebenenfalls unter Zusatz von eines dispergierend wirkenden und die Diffusion der nicht fixierten Anteile fördernden Mittels, gründlich gespült. Diese Färbe- und Druckverfahren sind zahlreich in der allgemeinen Fachliteratur wie auch in der Patentliteratur, wie beispielsweise in den anfangs genannten Druckschriften, beschrieben.

Gegenstand der vorliegenden Erfindung ist deshalb auch die Verwendung der Farbstoffe (1) zum Färben (einschließlich des Bedrucken) dieser Materialien bzw. Verfahren zum Färben (und Bedrucken) solcher Materialien in an und für sich üblicher Verfahrensweise, bei welchen ein Farbstoff (1) als Farbmittel eingesetzt wird, indem man den Farbstoff (1) im wäßrigen Medium auf das Material appliziert und ihn mittels Wärme oder mittels einer alkalisch wirkenden Verbindung oder mittels beidem auf dem Material fixiert.

Die Farbstoffe (1) zeichnen sich durch hohe Reaktivität, gutes Fixiervermögen und ein sehr gutes Aufbauvermögen aus. Sie können daher nach dem Ausziehfärbeverfahren bei niedrigen Färbetemperaturen eingesetzt werden und erfordern bei Pad-Steam-Verfahren nur kurze Dämpfzeiten. Die Fixiergrade sind hoch, und die nicht fixierten Anteile können leicht ausgewaschen werden, wobei die Differenz zwischen Ausziehgrad und Fixiergrad bemerkenswert klein, d.h. der Seifverlust sehr gering ist. Die Farbstoffe (1) eignen sich auch besonders zum Druck, vor allem auf Baumwolle, ebenso aber auch zum Bedrucken von stickstoffhaltigen Fasern, z.B. von Wolle oder Seide oder von Mischgeweben, die Wolle oder Seide enthalten.

Des weiteren zeichnen sich die erfindungsgemäßen Farbstoffe (1) darin aus, daß nach dem Färbeprozeß auf dem Fasermaterial nicht fixierte Farbstoffanteile sich sehr leicht auswaschen lassen, ohne daß Weißwäsche, die sich mit in dem Waschprozeß befindet, durch den sich ablösenden Farbstoff angeschmutzt wird. Hieraus ergeben sich Vorteile für den Färbeprozeß; Waschzyklen und damit Kosten werden eingespart. Dieser anwendungstechnische Vorteil besteht insbesondere für die erfindungsgemäßen Farbstoffe, die den allgemeinen Formeln (12A) und (12B) entsprechen.

Die mit den Farbstoffen (1) hergestellten Färbungen und Drucke besitzen, insbesondere auf Cellulosefasermaterialien, eine hohe Farbstärke und eine hohe Faser-Farbstoff-Bindungsstabilität sowohl in saurem als auch in alkalischem Bereich, weiterhin eine gute Lichtechtheit und sehr gute Naßechtheitseigenschaften, wie Wasch-, Wasser-, Seewasser-, Ueberfärbe- und Schweißechtheiten, sowie eine gute Plissierechtheit, Bügelechtheit und Reibechtheit.

Die nachfolgenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in den Beispielen formelmäßig beschriebenen Verbindungen sind in Form der freien Säure angegeben; im allgemeinen werden sie in Form ihrer Alkalimetallsalze, wie Lithium-, Natrium oder Kaliumsalze, hergestellt und isoliert und in Form ihrer Salze zum Färben verwendet. Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, in die Synthese eingesetzt werden.

Die für die erfindungsgemäßen Farbstoffe angegebenen Absorptionsmaxima (λₘₐₓ) im sichtbaren Bereich wurden anhand ihrer Alkalimetallsalze in wäßriger Lösung ermittelt. In den Tabellenbeispielen sind die λₘₐₓ-Werte bei der Farbtonangabe in Klammern gesetzt; die Wellenlängenangabe bezieht sich auf nm.

### Beispiel A

Man versetzt unter Rühren bei etwa 20°C eine Lösung von 350 Teilen N-Allyl-N-acetyl-anilin in 1500 Vol.-Teilen Aceton mit 145 Teilen 2-Mercapto-ethanol, erhitzt den Ansatz unter Rückfluß auf Siedetemperatur und gibt portionsweise, über mehrere Stunden verteilt, 25 Teile Azoisobutyronitril hinzu. Man rührt den Ansatz bei Siedetemperatur noch einige Stunden weiter, destilliert sodann das Aceton ab und versetzt den Rückstand mit 2 Teilen Natriumwolframat. Unter Rühren gibt man bei etwa 70°C langsam 340 Vol.-Teile einer 35 %igen wäßrigen Wasserstoffperoxid-Lösung hinzu und hält die Reaktionstemperatur bei 70 bis 80°C. Nach Beendigung der Oxidationsreaktion gibt man 200 Vol.-Teile einer 31 %igen wäßrigen Salzsäure hinzu und spaltet die Acetylgruppe hydrolytisch bei 100°C ab. Danach kühlt man den Ansatz auf Raumtemperatur und neutralisiert ihn mit festem Natriumhydroxid, worauf sich das N-Phenyl-N-[γ-(β'-hydroxyethylsulfonyl)-propyl]-amin als braunes Öl abscheidet. Es wird von der wäßrigen Phase abgetrennt und unter reduziertem Druck getrocknet.

Die erfindungsgemäße Verbindung N-Phenyl-N-[γ-(β'-hydroxyethylsulfonyl)-propyl]-amin zeigt im 300 MHz-Feld folgende ¹H-NMR-Daten in Deutorochloroform mit Tetramethylsilan als innerem Standard:
2,02-2,21 ppm (m,2H), 3,16-3,23 ppm (m,2H), 3,30 ppm (t,2H), 3,81 ppm (t,2H), 4,04-410 ppm (m,2H),
[6,59-6,74 + 7,14-7,20 + 7,35-7,48] ppm (m,5H).

### Beispiel B

312 Teile der Verbindung N-Phenyl-N-[γ-(β'-hydroxyethylsulfonyl)-propyl]-amin werden als viskoses Öl bei einer Temperatur von 10 bis 15°C innerhalb einer Stunde langsam in 700 Teile Schwefelsäure-Monohydrat eingerührt. Den Ansatz rührt man noch etwa eine Stunde bei 15°C nach und gießt ihn sodann langsam auf 3000 Teile eines Gemisches aus Wasser und Eis und rührt noch einige Zeit nach.
Die erhaltene Lösung wird filtriert und mit 1200 Teilen Calciumcarbonat und mit Natriumhydrogencarbonat versetzt, bis sie einen pH-Wert von 5 aufweist. Das ausgefallene Calciumsulfat wird abfiltriert, mit 1500 Teilen Wasser gewaschen und das vereinigte Filtrat unter reduziertem Druck bei 60°C eingedampft. Der erhaltene Rückstand wird in 2000 Vol.-Teilen Aceton angerührt, die Lösung abgesaugt und das Filtrat eingedampft.

Man erhält etwa 250 Teile N-Phenyl-N-[γ-(β'-sulfatoethylsulfonyl)-propyl]-amin-Natriumsalz als feste Verbindung.
1H-NMR-Analyse (100 MHz) mit Si(CH₃)₄ in d₆-Dimethylsulfoxid:

| | |
|---|---|
| 1,71-2,03 ppm (m,2H), | 2,94-3,24 ppm (dt,2H), |
| 3,25-3,50 ppm (t,2H;t,2H), | 4,07 ppm (t,2H) |
| 5,61 ppm (breit, 1H), | 6,40-6,63 ppm (m,3H), |
| 6,87-7,14 ppm (m,2H). | |

### Beispiel 1

a) Eine Lösung mit einem pH-Wert von 7 von 16,8 Teilen N-Phenyl-N-[γ-(β'-sulfatoethylsulfonyl)-propyl]-amin in 300 Teilen Wasser wird bei einer Temperatur von 0 bis 3°C unter intensivem Rühren schnell mit 7,2 Teilen 2,4,6-Trifluor-1,3,5-triazin (Cyanurfluorid) unter Einhaltung eines pH-Wertes zwischen 3 und 4 durch Zugabe einer wäßrigen Natriumhydrogencarbonatlösung versetzt. Zu dem Ansatz wird sodann eine etwa 5°C kalte Lösung von 16 Teilen 1-Amino-8-hydroxy-naphthalin-3,6-disulfonsäure in 150 Teilen Wasser unter Rühren gegeben. Es wird ein pH-Wert zwischen 9,5 und 10 eingestellt und der Ansatz etwa 10 Minuten nachgerührt. Danach wird ein pH-Wert von 7 eingestellt und die Kondensationsreaktion unter weiterem Rühren bei etwa 20°C zu Ende geführt.
b) Zu der so hergestellten, als Kupplungskomponente dienenden Verbindung 1-{2'-Fluor-4'-[N-phenyl-N-γ-(β'-sulfatoethylsulfonyl)-propyl]-amino-1',3',5'-triazin-6'-yl}-amino-8-hydroxy-naphthalin-3,6-disulfonsäure gibt man eine auf üblichem Wege aus 8,7 Teilen 2-Sulfo-anilin in 100 Teilen Wasser unter Verwendung von 10 Vol.-Teilen konzentrierter wäßriger Salzsäure und 25 Vol.-Teilen einer wäßrigen 2N-Natriumnitritlösung hergestellte Diazoniumsalzlösung hinzu und führt die Kupplungsreaktion bei einem pH-Wert von 6 und einer langsam von 5°C auf 20°C steigenden Temperatur durch. Man rührt noch einige Zeit nach und isoliert den erfindungsgemäßen Farbstoff, der, in Form der freien Säure geschrieben, die Formel 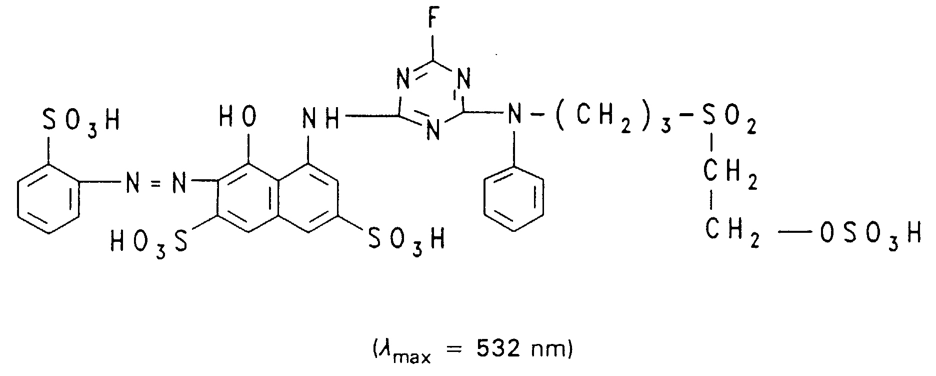 besitzt, in üblicher Weise, beispielsweise durch Aussalzen mit Natriumchlorid.

Der erfindungsgemäße Farbstoff zeigt sehr gute anwendungstechnische und faserreaktive Eigenschaften und färbt die in der Beschreibung genannten Materialien, wie insbesondere Cellulosefasermaterialien, nach den in den Technik für faserreaktive Farbstoffe üblichen Färbe- und Druckverfahren in farbstarken roten Tönen mit guten Echtheitseigenschaften, von denen insbesondere die guten Chlorbadewasserechtheiten hervorgehoben werden können.

### Beispiel 2

Die gemäß den Angaben des Beispieles 1a) hergestellte Lösung der Kupplungskomponente wird mit einer wäßrigen Natriumcarbonatlösung bei 20 bis 25°C auf einen pH-Wert von 11 bis 12 gestellt; dieser pH-Bereich wird etwa 20 Minuten unter weiterem Rühren gehalten. Man isoliert die so hergestellte Vinylsulfonylverbindung 1-[2'-Fluor-4'-(N-phenyl-N-γ-vinylsulfonyl-propyl)-amino-1',3',5'-triazin-6'-yl]-amino-8-hydroxy-naphthalin-3,6-disulfonsäure durch Aussalzen mit Natriumchlorid und Filtration.
64 Teile dieser Verbindung werden in 500 Teilen Wasser bei einem pH-Wert von 7 und einer Temperatur von 15 bis 20°C gelöst. Man gibt eine auf üblichem Wege mittels 30 Teilen einer 31%igen wäßrigen Salzsäure und 20 Teilen einer wäßrigen 5N-Natriumnitritlösung hergestellten Diazoniumsalzsuspension aus 41 Teilen 1-Sulfo-6-(β-sulfatoethylsulfonyl)-2-amino-naphthalin in 1000 Teilen Wasser von 0°C hinzu, stellt den pH-Wert auf 7 und rührt noch einige Zeit unter Einhaltung des pH-Wertes bei etwa 15 bis 20°C nach. Man versetzt die so hergestellte Farbstofflösung mit 12 Teilen Dinatriumhydrogenphosphat und stellt mit Phosphorsäure einen pH-Wert von 7 ein.

Man isoliert den erhaltenen erfindungsgemäßen Azofarbstoff der Formel (in Form der freien Säure geschrieben) 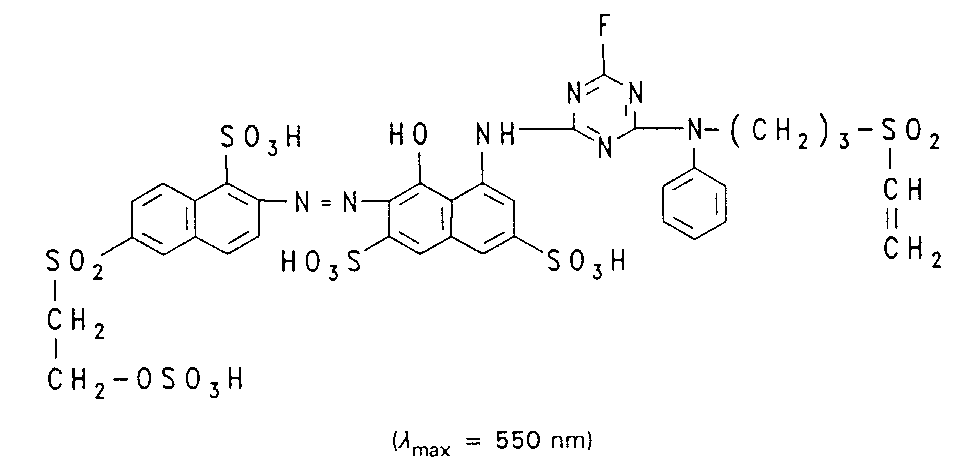 auf üblichem Wege, beispielsweise durch Eindampfen unter reduziertem Druck bei 50°C. Der Farbstoff zeigt sehr gute anwendungstechnische Eigenschaften und färbt beispielsweise Cellulosefasermaterialien bei einem hohen Fixiergrad nach den für faserreaktive Farbstoffe üblichen Anwendungsmethoden in brillanten blaustichig roten Tönen mit guten Echtheiten, von denen insbesondere die Chlorbadewasserechtheit hervorgehoben werden kann.

### Beispiel 3

Eine Lösung von 36 Teilen N-Phenyl-N-[γ-(β'-sulfatoethylsulfonyl)-propyl]-amin und 17,9 Teilen Dinatriumhydrogenphosphat in 250 Teilen Wasser versetzt man unter kräftigem Rühren bei einer Temperatur von 0°C und einem pH-Wert von 6,5 mit 14 Teilen Trifluortriazin, rührt noch 10 Minuten weiter und gibt sodann eine Lösung des Lithiumsalzes von 24 Teilen 3-Amino-6-sulfo-8-naphthol in 250 Teilen Wasser hinzu und rührt den Ansatz noch etwa fünf Stunden bei einer Temperatur zwischen 0 und 5°C unter Einhaltung eines pH-Wertes von 6 bis 6,5 mittels einer wäßrigen 1N-Lithiumhydroxydlösung. Nach Beendigung dieser Kondensationsreaktion gibt man eine auf üblichem Wege hergestellte salzsaure wäßrige Diazoniumsalzlösung aus 21 Teilen 2-Sulfo-4-methoxy-anilin hinzu und führt die Kupplungsreaktion bei einer Temperatur zwischen 15 und 20°C und einem pH-Wert zwischen 6 und 7 zu Ende.
Der erfindungsgemäße Azofarbstoff, der, in Form der freien Säure geschrieben, die Formel 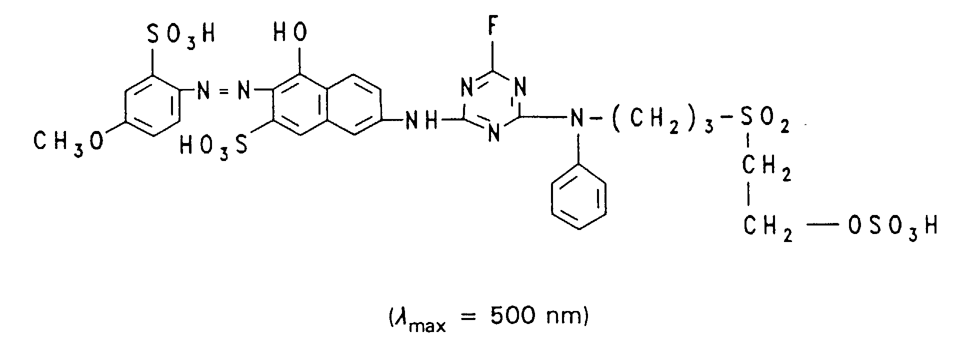 besitzt, wird in üblicher Weise, beispielsweise durch Aussalzen mit Natriumchlorid, als Alkalimetallsalz isoliert. Er zeigt sehr gute anwendungstechnische Eigenschaften und färbt die in der Beschreibung genannten Fasermaterialien, wie Wolle und insbesondere Cellulosefasermaterialien, wie Baumwolle, in farbstarken, brillanten, scharlachroten Tönen mit guten Echtheitseigenschaften.

### Beispiel 4

Unter Einhaltung eines pH-Wertes zwischen 7,5 und 8 und einer Temperatur von etwa 0°C gibt man langsam 14,2 Teile Cyanurfluorid zu einer neutralen Lösung von 54,5 Teilen der Azoverbindung 4-(3',6',8'-Trisulfo-naphth-2'-yl)-azo-3-ureido-anilin in 800 Teilen Wasser. Man rührt den Ansatz noch einige Zeit unter Einhaltung dieser Reaktionsbedingungen bis zur Beendigung der Umsetzung weiter und gibt sodann bei etwa 5°C und unter Einhaltung des pH-Wertes zwischen 6 und 7 33,8 Teile N-Phenyl-N-[γ-(β'-sulfatoethylsulfonyl)-propyl]-amin hinzu. Man rührt den Ansatz noch einige Zeit weiter und läßt die Reaktionstemperatur auf 20°C ansteigen.

Nach Beendigung der Umsetzung isoliert man den erfindungsgemäßen Farbstoff der Formel (in Form der freien Säure geschrieben) 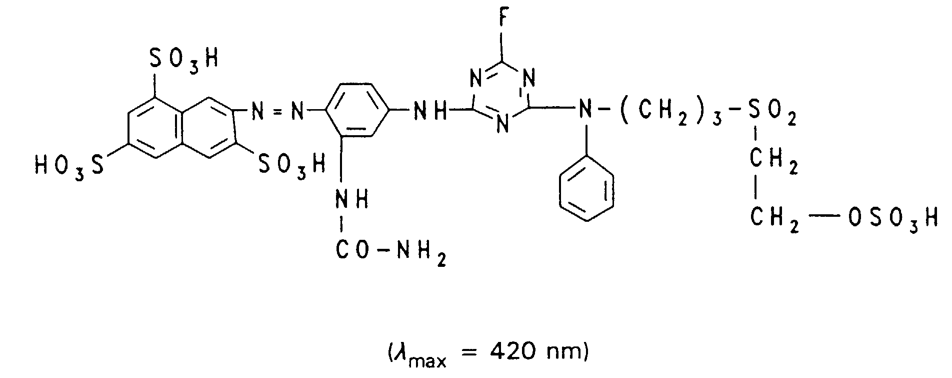 in üblicher Weise als Alkalimetallsalz (Natriumsalz). Er zeigt sehr gute faserreaktive Farbstoffeigenschaften und färbt die in der Beschreibung genannten Materialien, wie insbesondere Cellulosefasermaterialien, nach den in der Technik für faserreaktive Farbstoffe üblichen Applikations- und Fixierverfahren in farbstarken rotstichig gelben Tönen mit guten Echtheitseigenschaften.

### Beispiel 5

Eine Lösung von 211 Teilen 4-(4',8'-Disulfo-naphth-2'-yl)-azo-3-acetylamino-anilin in 1000 Teilen Wasser mit einer Temperatur von 0 bis 2°C und einem pH-Wert von 8,5 wird unter gutem Rühren und unter Einhaltung eines pH-Wertes zwischen 6,5 und 7 mittels einer wäßrigen Natriumcarbonatlösung mit 7,2 Teilen Cyanurfluorid versetzt. Man rührt den Ansatz noch etwa 10 Minuten nach und gibt sodann bei etwa 5°C 19,5 Teile N-Phenyl-N-[γ-(β'-sulfatoethylsulfonyl)-propyl]-amin in 200 Teilen Wasser hinzu und führt die Umsetzung bei einem pH-Wert von 6,5 und einer Temperatur von etwa 20°C zu Ende.

Man isoliert den erfindungsgemäßen Azofarbstoff der Formel (in Form der freien Säure geschrieben) 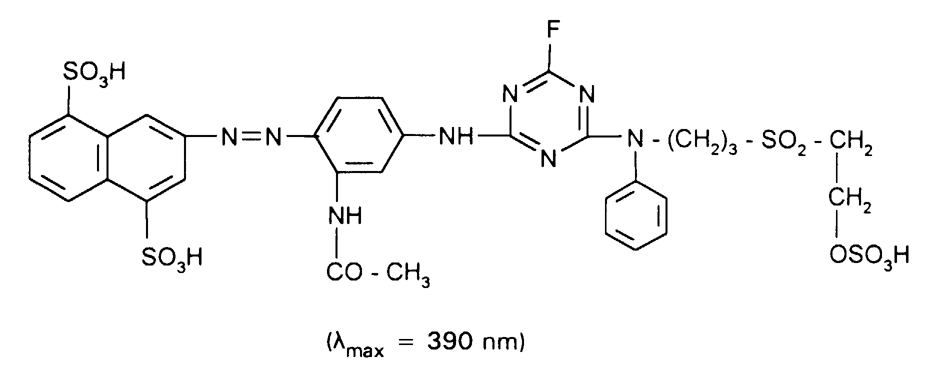 in üblicher Weise, beispielsweise durch Aussalzen mit Natriumchlorid. Er besitzt sehr gute faserreaktive, anwendungstechnische Eigenschaften und färbt die in der Beschreibung genannten Materialien, wie beispielsweise Baumwolle, in grünstichig gelben Tönen mit guten Echtheitseigenschaften.

### Beispiel 6

Eine Lösung von 211 Teilen 4-(4',8'-Disulfo-naphth-2'-yl)-azo-3-acetylaminoanilin in einer Mischung aus 500 Teilen Wasser und 200 Teilen Eis wird unter Einhaltung eines pH-Wertes von 7,5 innerhalb von 30 Minuten unter gutem Rühren mit einer Lösung von 10 Teilen 2,4,6-Trichlor-1,3,5-triazin (Cyanurchlorid) in 100 Teilen Aceton versetzt. Man rührt bei pH 7,5 noch etwa 10 Minuten nach und gibt sodann eine Lösung von 19,5 Teilen N-Phenyl-N-[γ-(β'-sulfatoethylsulfonyl)-propyl]-amin in 200 Teilen Wasser hinzu. Man führt die Umsetzung während etwa 3 Stunden unter Einhaltung eines pH-Wertes von 6 und einer Temperatur von 35 bis 40°C zu Ende.

Man isoliert den erfindungsgemäßen Farbstoff der Formel (in Form der freien Säure geschrieben) 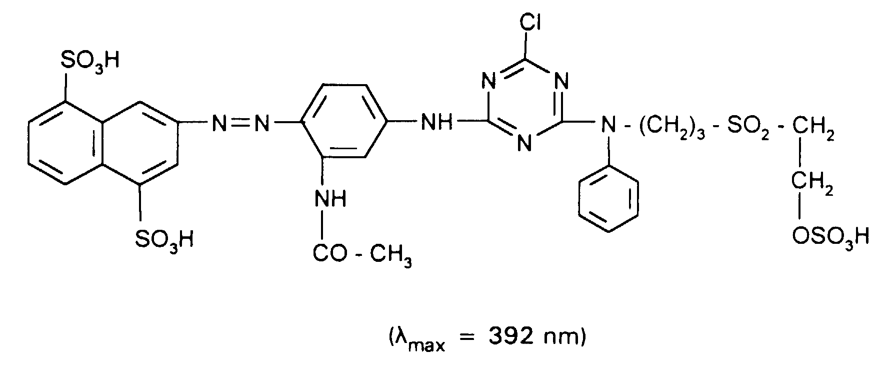 in üblicher Weise, beispielsweise durch Eindampfen der wäßrigen Syntheselösung unter reduziertem Druck bei 60°C, als Alkalimetallsalz. Der Farbstoff zeigt sehr gute anwendungstechnische Eigenschaften und färbt beispielsweise Baumwolle nach den für faserreaktive Farbstoffe üblichen Färbe- und Druckverfahren in grünstichig gelben Tönen mit guten Echtheitseigenschaften.

### Beispiel 7

Eine neutrale Lösung von 31,9 Teilen 1-Amino-3,6-disulfo-8-naphthol in 800 Teilen Wasser wird bei 0°C und einem pH-Wert von 4,5 langsam innerhalb von 5 Minuten mit 14,2 Teilen Cyanurfluorid versetzt. Anschließend gibt man 33,1 Teile N-Phenyl-N-[γ-(β'-sulfatoethylsulfonyl)-propyl]-amin unter Einhaltung eines pH-Wertes von 7 und einer Temperatur von 5 bis 15°C hinzu und rührt noch einige Zeit nach. Sodann gibt man bei 50 bis 55°C und einem pH-Wert von 6,5 nochmals 29,9 Teile dieses Propylamins hinzu und rührt bei dieser Temperatur und dem pH-Wert von 6,5 noch weitere 4 Stunden nach. Anschließend versetzt man den Ansatz bei 15 bis 30°C mit der wäßrigen salzsauren Lösung des Diazoniumsalzes von 51,1 Teilen 1-Sulfo-2-amino-6-(β-sulfatoethylsulfonyl)-naphthalin und führt die Kupplungsreaktion bei einem pH-Wert von 6 durch.

Der gebildete erfindungsgemäße Monoazofarbstoff wird durch Sprühtrocknen bei einem pH-Wert von 7 aus der Syntheselösung isoliert. Er zeigt sehr gute Farbstoffeigenschaften und liefert auf den in der Beschreibung genannten Fasermaterialien, wie insbesondere Baumwolle, nach den üblichen Applikations- und Fixierverfahren für faserreaktive Farbstoffe farbstarke rote Färbungen. Der Farbstoff besitzt, in Form der freien Säure geschrieben, die Formel

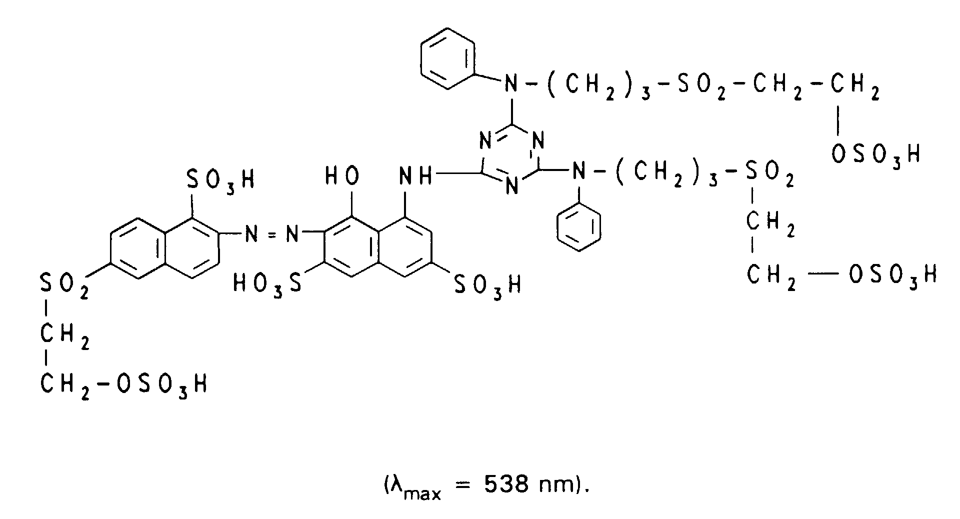

### Beispiel 8

Zur Herstellung des im Beispiel 7 beschriebenen erfindungsgemäßen Azofarbstoffes kann man auch erfindungsgemäß wie folgt verfahren: Man gibt unter Rühren und unter Einhaltung eines pH-Wertes von 1,7 bis 2,2 155,2 Teile Cyanurchlorid in eine Suspension von 225,2 Teilen 1-Amino-3,6-disulfo-8-naphthol in 1440 Teilen Wasser und 720 Teilen Eis. Man rührt den Ansatz noch etwa vier Stunden bei 0 bis 3°C und dem angegebenen pH-Bereich und fügt sodann 280 Teile N-Phenyl-N-[γ-(β'-hydroxyethylsulfonyl)-propyl]-amin-Hydrochlorid hinzu und führt die zweite Kondensationsreaktion bei einem pH-Wert von 7 und einer Temperatur von 60°C durch. Die dritte Kondensationsreaktion wird nach Zugabe von weiteren 280 Teilen dieses Propylaminhydrochlorids bei einem pH-Wert von 9 und einer Temperatur von 80 bis 90°C ausgeführt. Der Ansatz wird danach bei 15 bis 30°C mit der wäßrigen, salzsauren Suspension des Diazoniumsalzes von 411 Teilen 1-Sulfo-2-amino-6-(β-sulfatoethylsulfonyl)-naphthalin versetzt; die Kupplungsreaktion erfolgt innerhalb dieses Temperaturbereiches und bei einem pH-Wert von 7. Die Syntheselösung wird danach geklärt und sprühgetrocknet. Das elektrolythaltige Produkt wird innerhalb einer Stunde bei maximal 10°C in ein Gemisch aus 800 Volumenteilen Schwefelsäure-Monohydrat und 424 Volumenteilen 20%igem Oleum eingetragen. Nach etwa zwei Stunden ist bei 10°C die Veresterung beendet. Der Ansatz wird auf Eis gegeben und überschüssige Schwefelsäure mittels Calciumcarbonat neutralisiert. Das Calciumsulfat wird abgesaugt und mit Wasser gewaschen. Der erfindungsgemäße Farbstoff wird aus den vereinigten Filtraten durch Eindampfen unter reduziertem Druck bei 60°C oder durch Sprühtrocknung als Alkalimetallsalz isoliert.

Der auf diese Weise hergestellte erfindungsgemäße Azofarbstoff, der die chemische Konstitution des im Beispiel 7 beschriebenen Azofarbstoffs besitzt, zeigt die gleichen guten Eigenschaften und liefert dementsprechend die gleichen guten Färbungen und Drucke in guten Gebrauchsechtheiten.

### Beispiel 9

Man versetzt unter Einhaltung eines pH-Wertes von 5 bis 6 und einer Temperatur von 0°C eine neutrale Lösung von 18,1 Teilen der Azoverbindung 7-(2'-Sulfo-4'-methoxy-phenyl)-azo-6-sulfo-8-hydroxy-3-amino-naphthalin in 500 Teilen Wasser mit 9,5 Teilen Cyanurchlorid, rührt noch einige Zeit nach, erwärmt auf 25°C, gibt unter Einhaltung des angegebenen pH-Bereiches 16,8 Teile N-Phenyl-N-[γ-(β'-sulfatoethylsulfonyl)-propyl]-amin hinzu und erhöht die Reaktionstemperatur langsam auf 40°C. Nach Beendigung der Umsetzung wird der erhaltene erfindungsgemäße Farbstoff, der, in Form der freien Säure geschrieben, die Formel 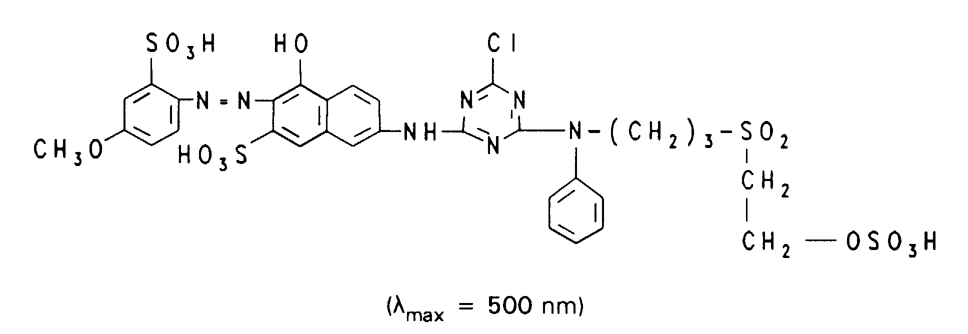 besitzt, in üblicher Weise, beispielsweise durch Aussalzen mit Natriumchlorid, als Alkalimetallsalz isoliert.

Der erfindungsgemäße Farbstoff zeigt sehr gute anwendungstechnische Eigenschaften und färbt die in der Beschreibung genannten Fasermaterialien, wie Wolle und insbesondere Cellulosefasermaterialien, wie Baumwolle, in farbstarken gelbstichig roten Tönen mit guten Echtheitseigenschaften.

### Beispiel 10

Eine neutrale Lösung von 40,9 Teilen der Disazoverbindung 4-[4'-(4",6",8"-Trisulfo-naphth-2"-yl)-azo-6'-sulfo-naphth-1'-yl]-3-methyl-anilin (auf üblichem Wege herstellbar durch Kupplung von 6-Sulfo-1-amino-naphthalin mit dem Diazoniumsalz der 2-Aminonaphthalin-4,6,8-trisulfonsäure und Diazotierung der erhaltenen Monoazoverbindung und Kupplung mit 3-Methyl-anilin) in 300 Teilen Wasser wird bei einer Temperatur zwischen -3°C und +5°C unter Einhaltung eines pH-Wertes zwischen 6 und 7 mit 9,2 Teilen Cyanurfluorid versetzt. Man rührt noch einige Zeit unter den angegebenen Reaktionsbedingungen nach und gibt sodann 21,5 Teile N-Phenyl-N-[γ-(β'-sulfatoethylsulfonyl)-propyl]-amin hinzu und führt diese zweite Kondensationsreaktion bei einem pH-Wert zwischen 6 und 7 und einer Temperatur von zunächst etwa 5°C, die man im Verlaufe der Reaktion auf 20°C ansteigen läßt, durch. Man rührt noch einige Zeit bei 20°C nach und gibt sodann zu der erhaltenen wäßrigen Syntheselösung des erfindungsgemäßen Disazofarbstoffes 8,9 Teile Dinatriumhydrogenphosphat.

Der Disazofarbstoff wird in üblicher Weise isoliert, beispielsweise durch Eindampfen der Syntheselösung. Er besitzt, in Form der freien Säure geschrieben, die Formel

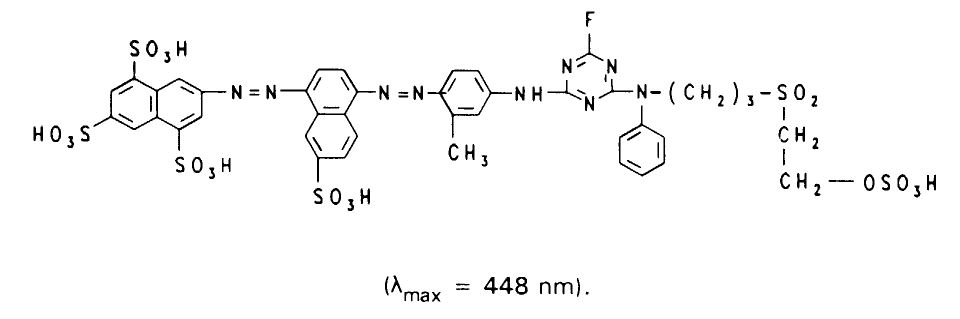

Nach den in der Technik für faserreaktive Farbstoffe üblichen Färbeverfahren erhält man mit dem erfindungsgemäßen Disazofarbstoff farbstarke, echte Färbungen in rotstichig braunen Tönen.

### Beispiel 11

45,3 Teile 7-(2'-Sulfo-4'-methoxy-phenyl)-azo-8-hydroxy-3-amino-naphthalin-6-sulfonsäure werden in wäßriger Lösung bei einer Temperatur von 0 bis 3°C und einem pH-Wert von 3,5 bis 4 mit 19 Teilen Cyanurchlorid umgesetzt. Nach beendeter Kondensationsreaktion werden 28,0 Teile N-Phenyl-N-[γ-(β'-hydroxyethylsulfonyl)-propyl]-amin-Hydrochlorid hinzugegeben, und die zweite Kondensationsreaktion wird bei 60°C und einem pH-Wert von 7 während zwei Stunden zu Ende geführt (wie in den vorherigen Beispielen, kann die Umsetzung dünnschichtchromatographisch verfolgt werden). Danach gibt man 4,4 Teile Cyanamid hinzu und führt die dritte Kondensationsreaktion bei einem pH-Wert von 10 und einer Temperatur von 85°C durch. Die gebildete Azoverbindung mit der β-Hydroxyethylsulfonyl-Gruppe wird nach Einstellen der Syntheselösung auf einen pH-Wert von 7 durch Zugabe von Kaliumchlorid ausgesalzen, isoliert und getrocknet. 10 Teile dieses Produktes, das ca. 70%ig an der Azoverbindung ist, werden bei 5 bis 10°C in 15 Teile 100%ige Schwefelsäure unter Rühren eingetragen. Nachdem die Azoverbindung gelöst ist, gibt man den Ansatz auf 80 Teile Eis, neutralisiert das Gemisch mittels Calciumcarbonat, saugt vom ausgefallenen Calciumsulfat ab, wäscht den Rückstand mit Wasser und dampft das Filtrat unter reduziertem Druck ein.

Der als Alkalimetallsalz (Natriumsalz) erhaltene erfindungsgemäße Azofarbstoff besitzt, in Form der freien Säure geschrieben, die Formel

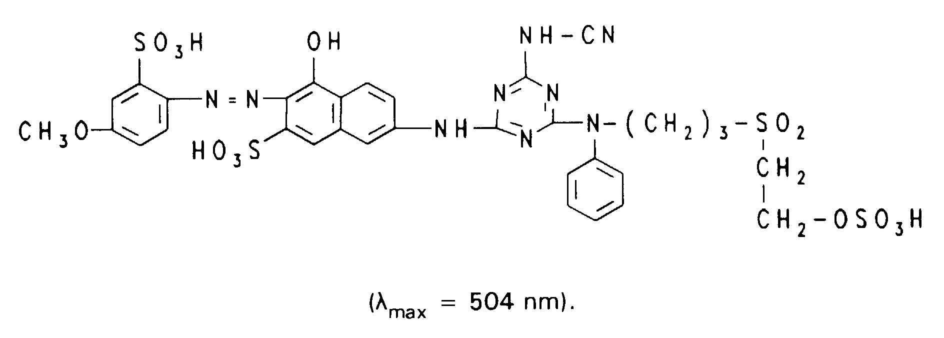

Er zeigt sehr gute faserreaktive Farbstoffeigenschaften und färbt die in der Beschreibung genannten Materialien, wie insbesondere Cellulosefasermaterialien, wie Baumwolle, in farbstarken, leuchtend scharlachroten Tönen mit hohem Fixiergrad.

### Beispiele 12 bis 133

In den nachfolgenden Tabellenbeispielen werden weitere erfindungsgemäße Farbstoffe entsprechend der allgemeinen Formel (A) 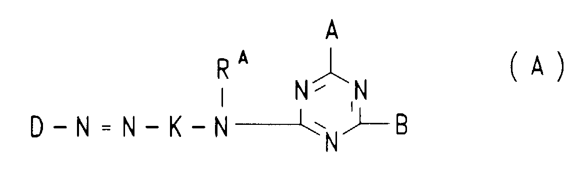 anhand ihrer Komponenten (dem Rest D der Diazokomponente D-NH₂ , dem Rest -K-N(R^{A})- der aminogruppenhaltigen Kupplungskomponente der Formel H-K-N(R^{A})H und den Substituenten A und B) beschrieben. Sie lassen sich in einer der erfindungsgemäßen Verfahrensweisen, beispielsweise analog einem der obigen Ausführungsbeispiele, herstellen. Sie besitzen sehr gute faserreaktive Farbstoffeigenschaften und färben beispielsweise Baumwolle in dem in dem jeweiligen Tabellenbeispiel angegebenen Farbton in hoher Farbstärke und guten Echtheiten.

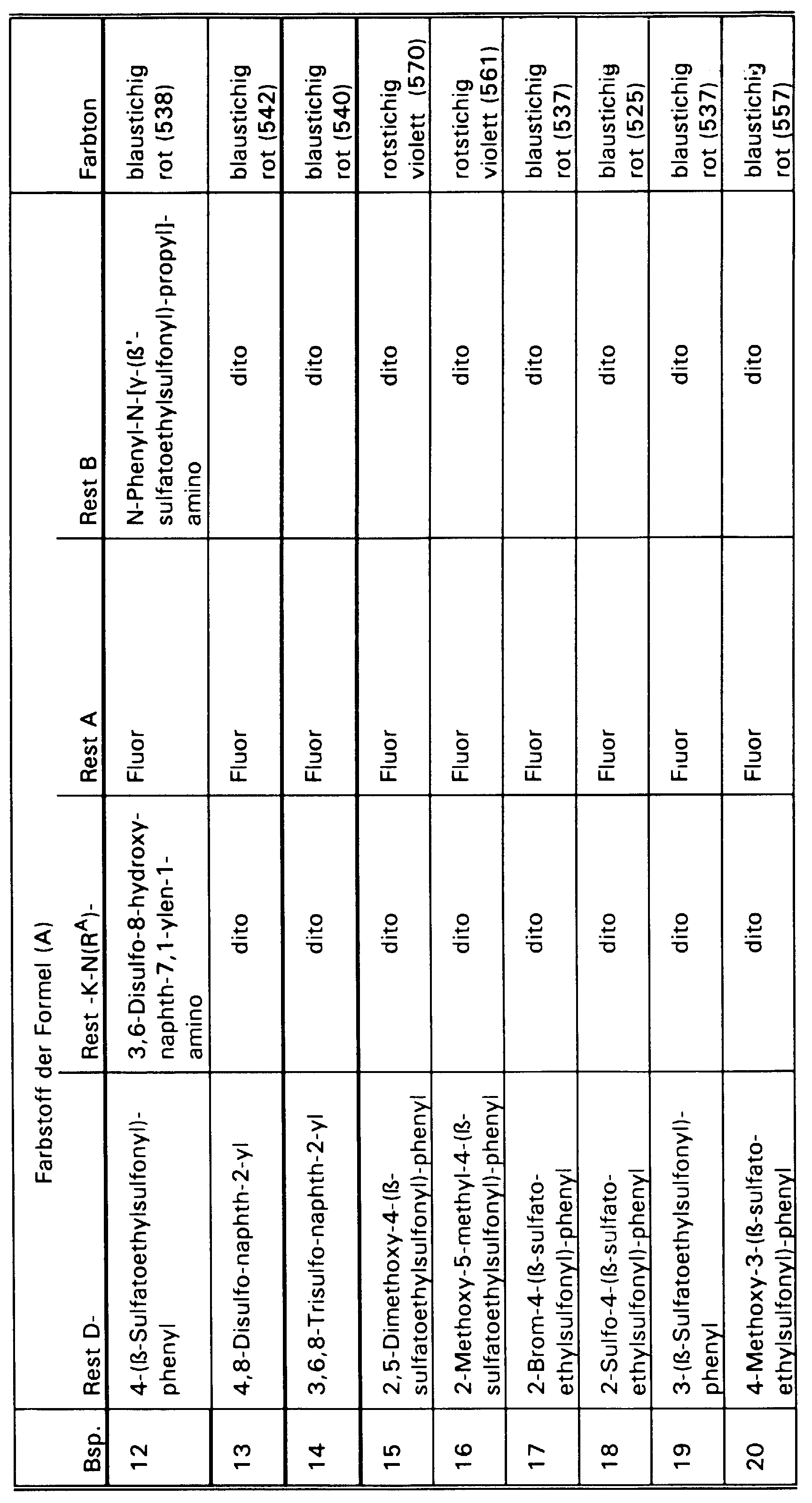

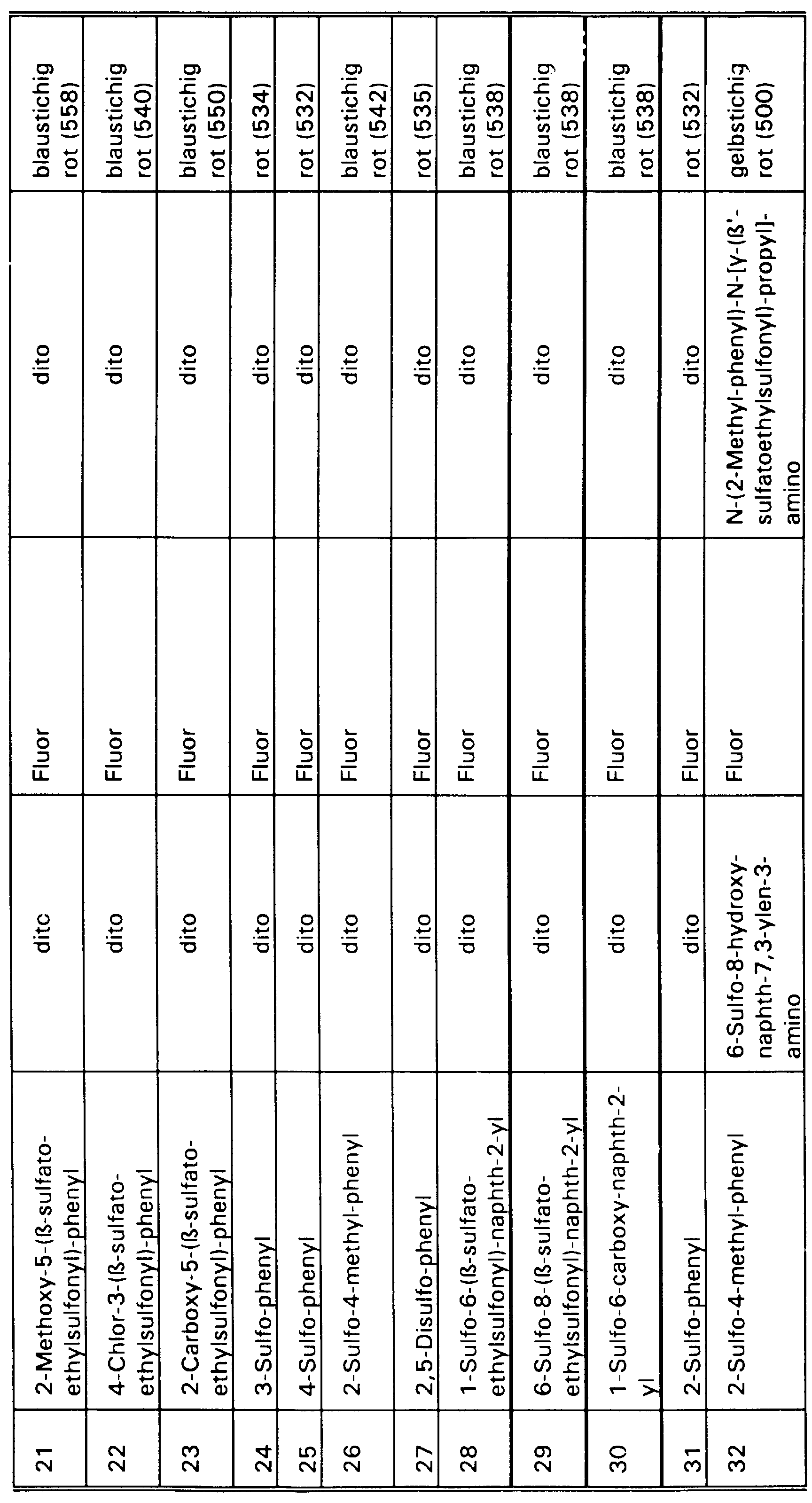

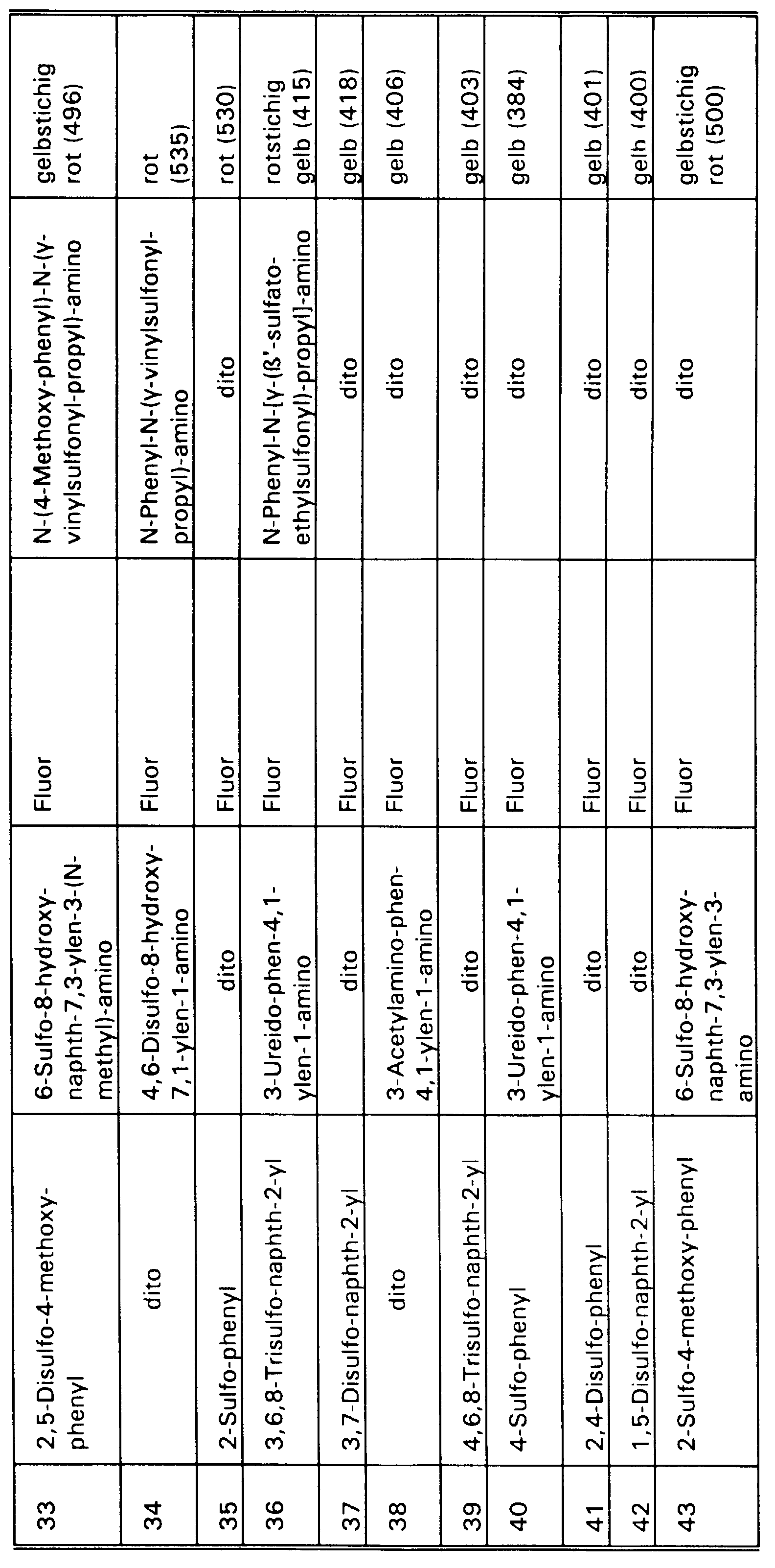

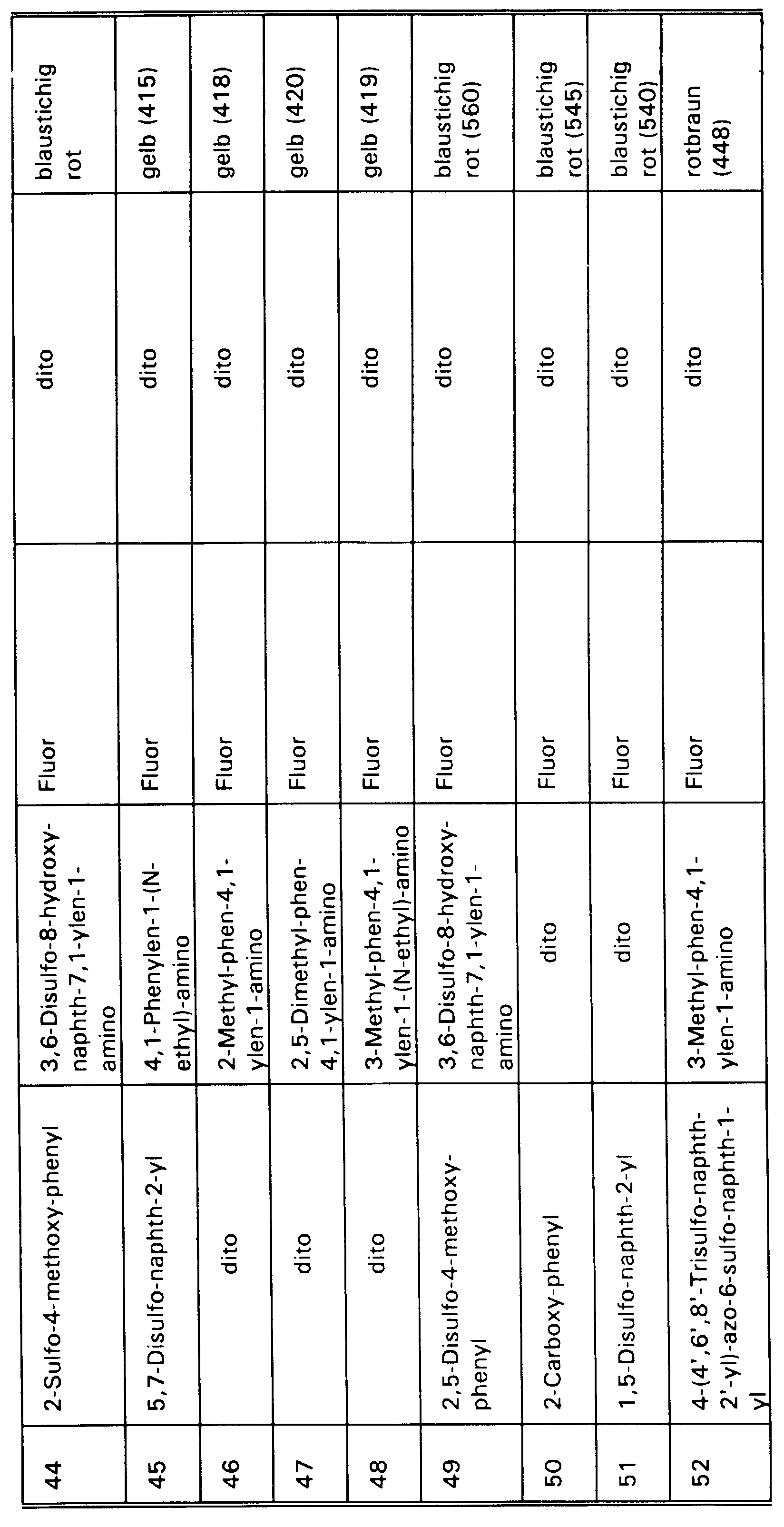

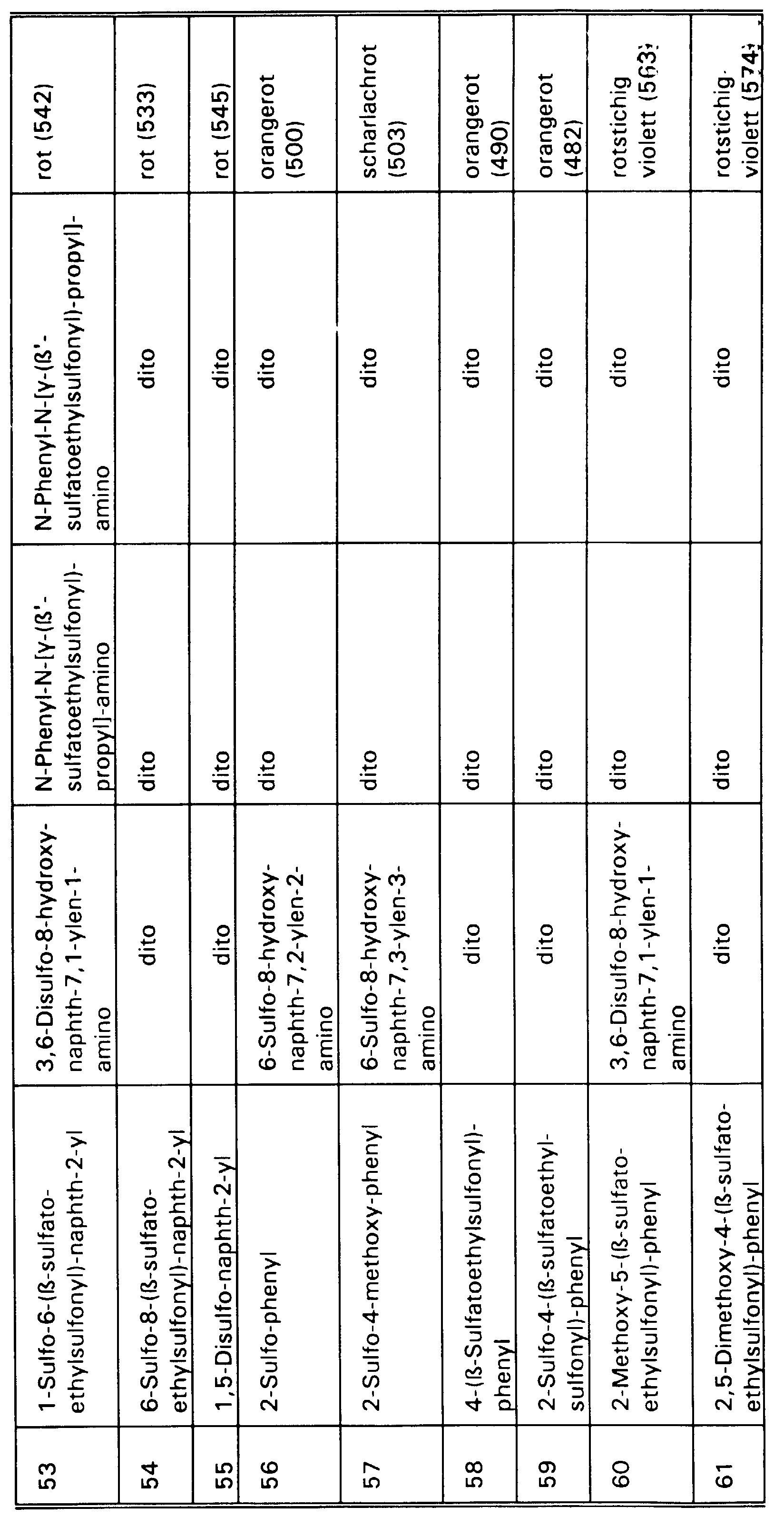

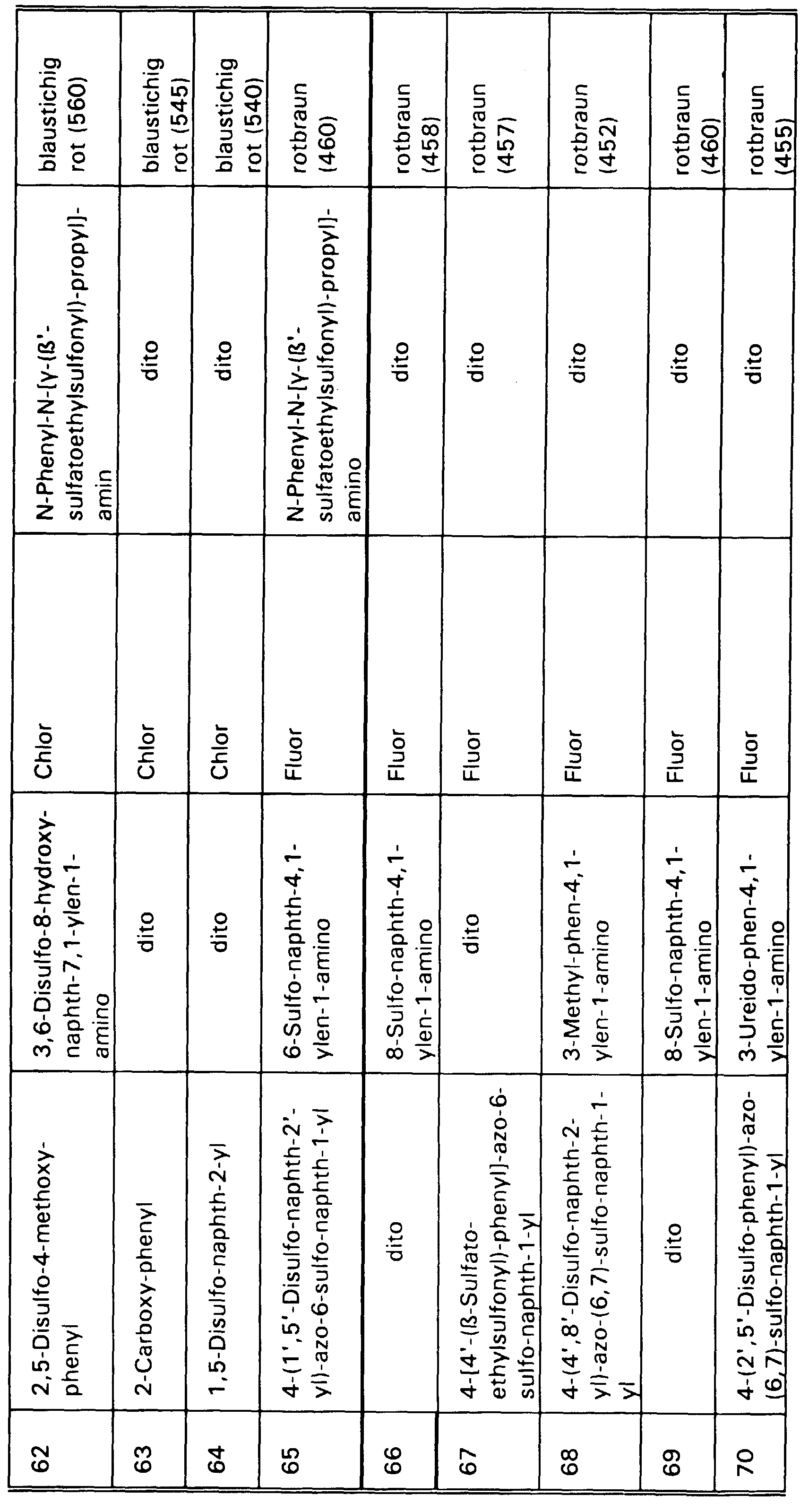

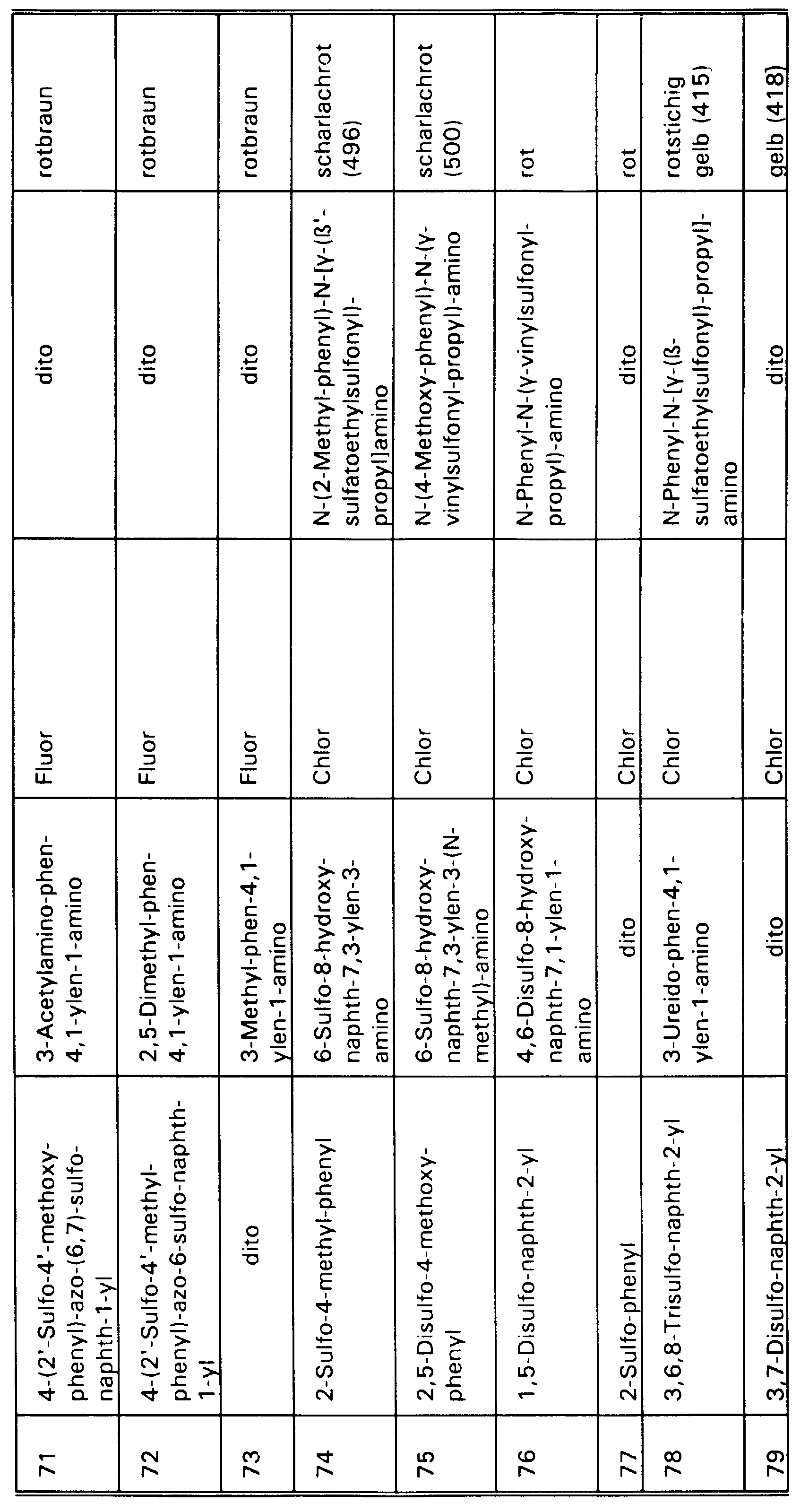

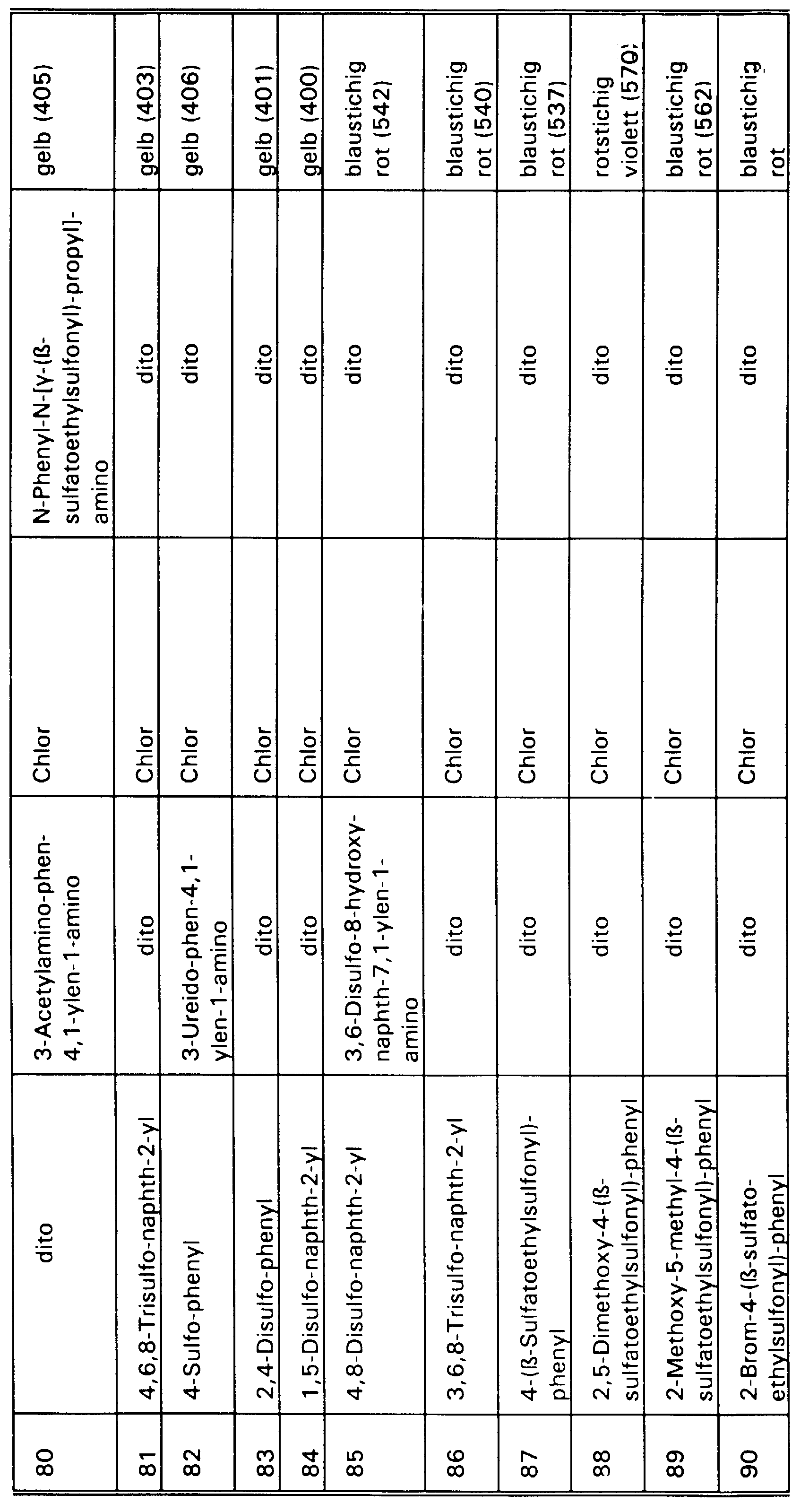

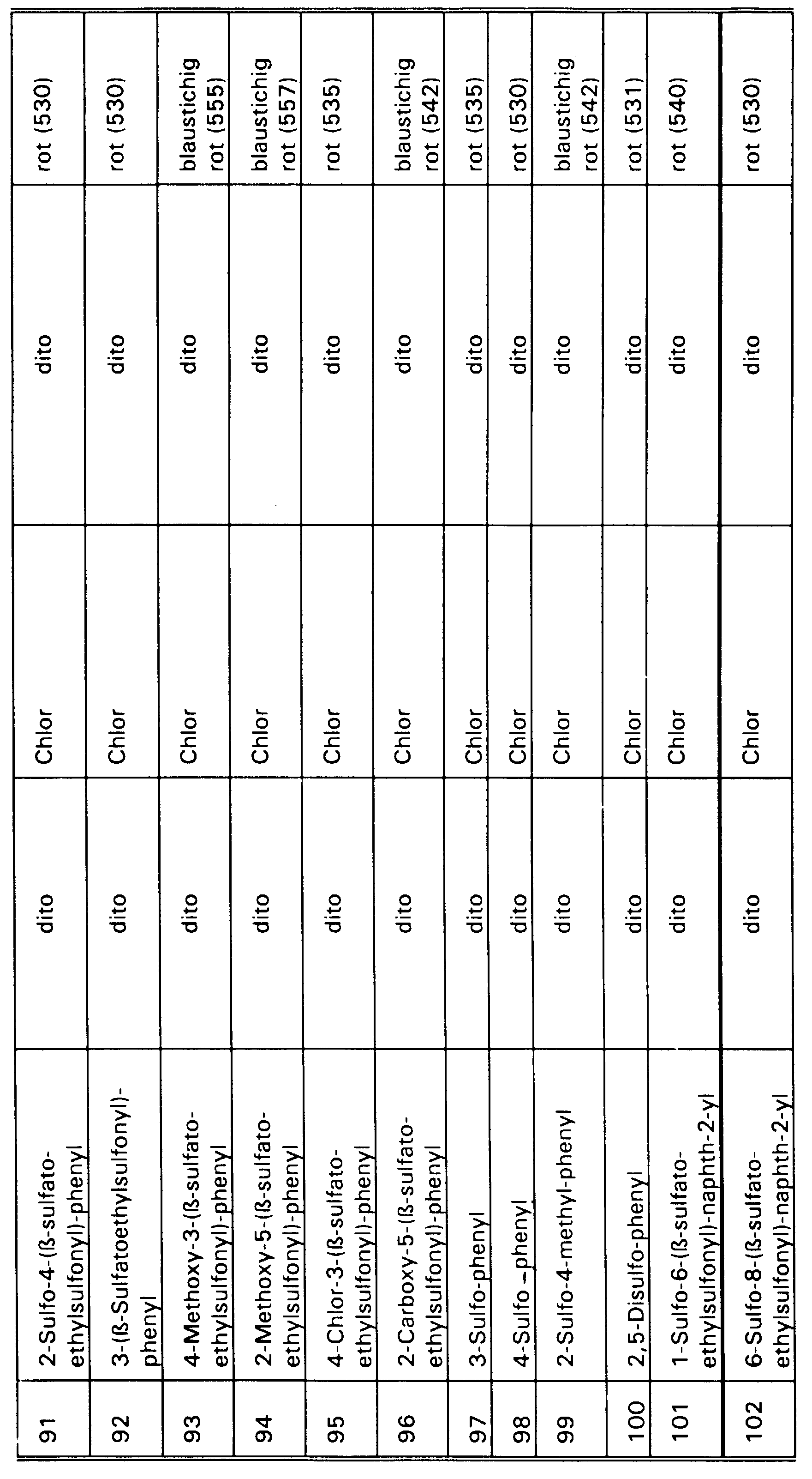

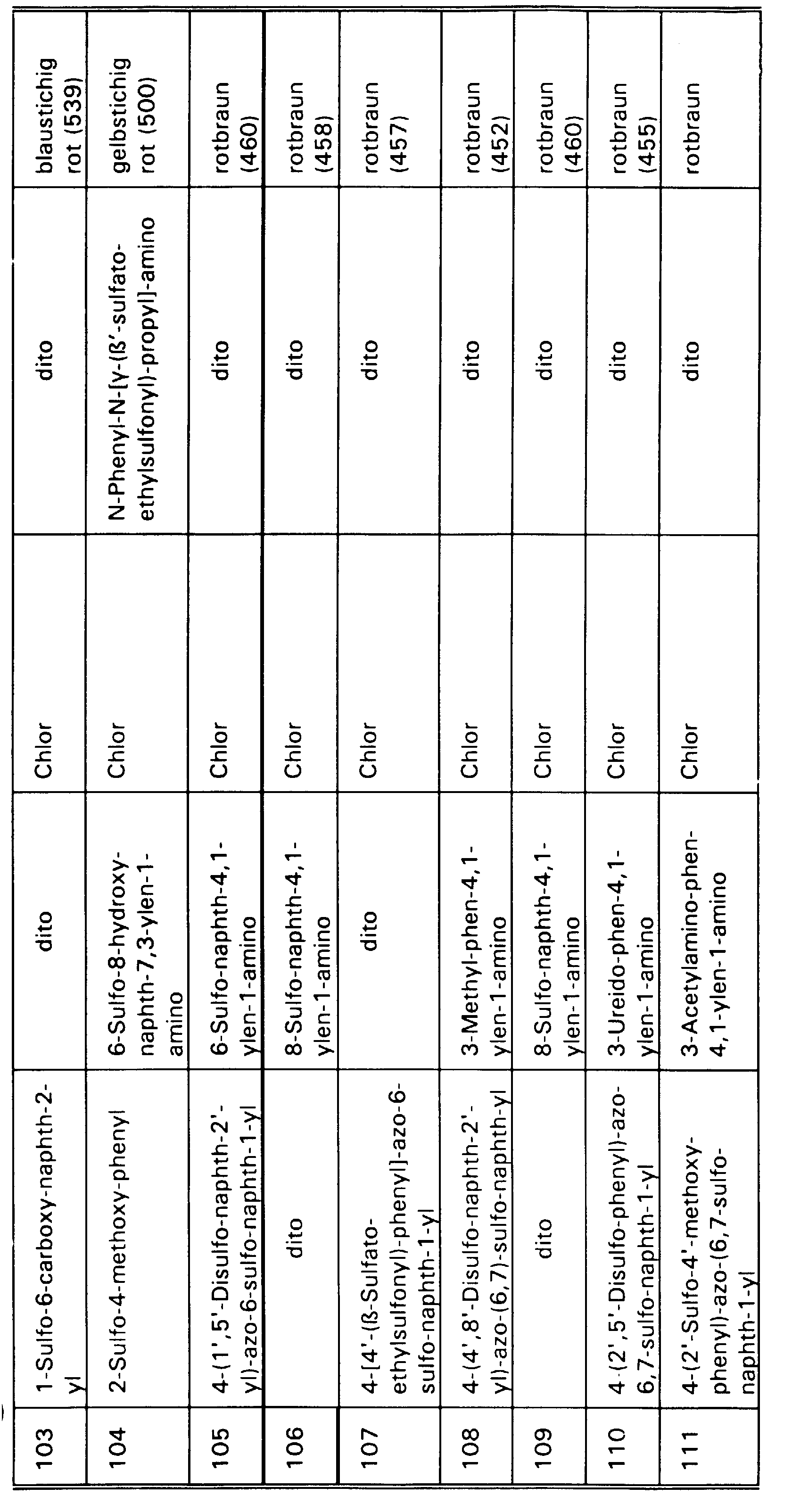

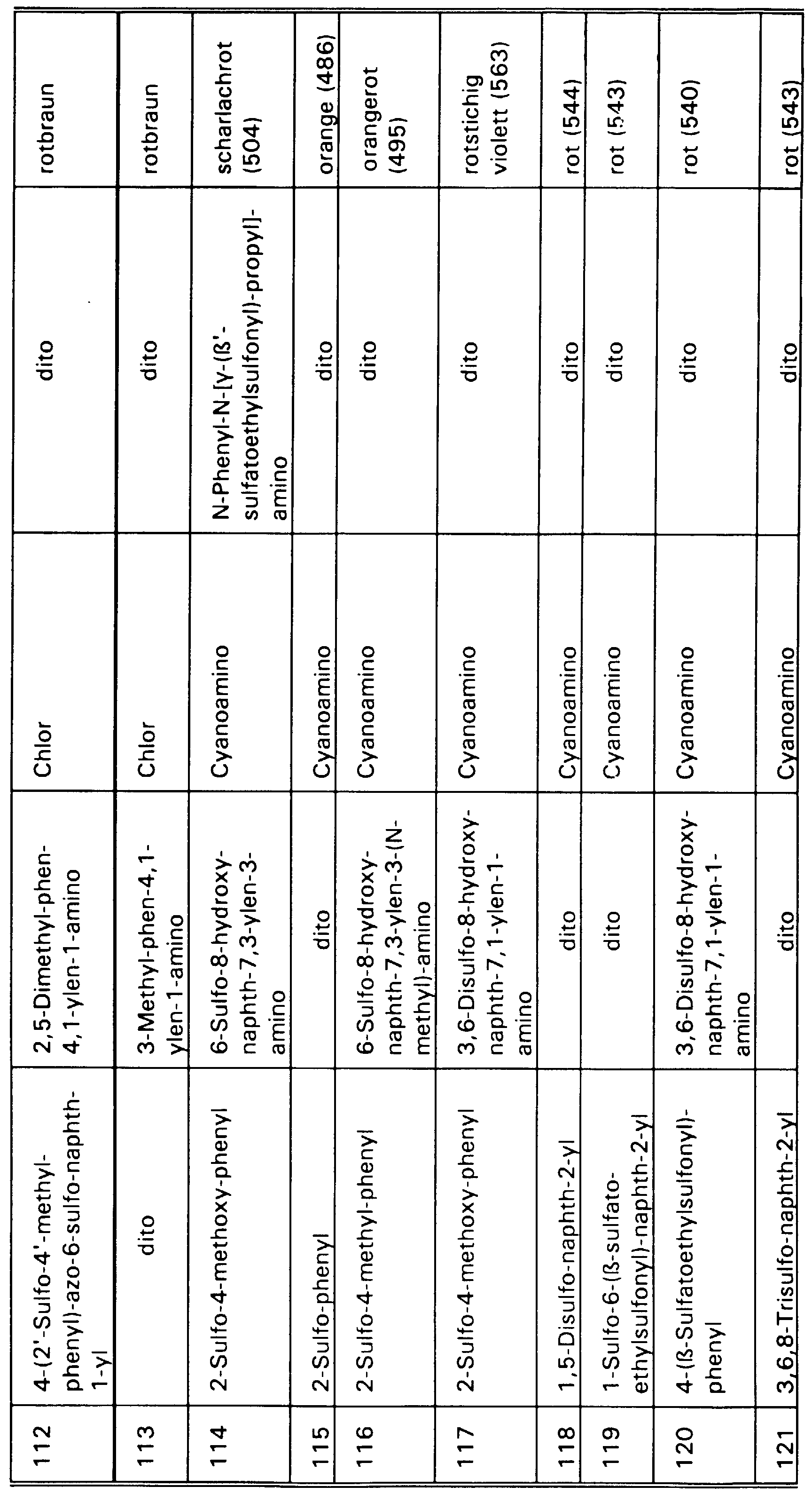

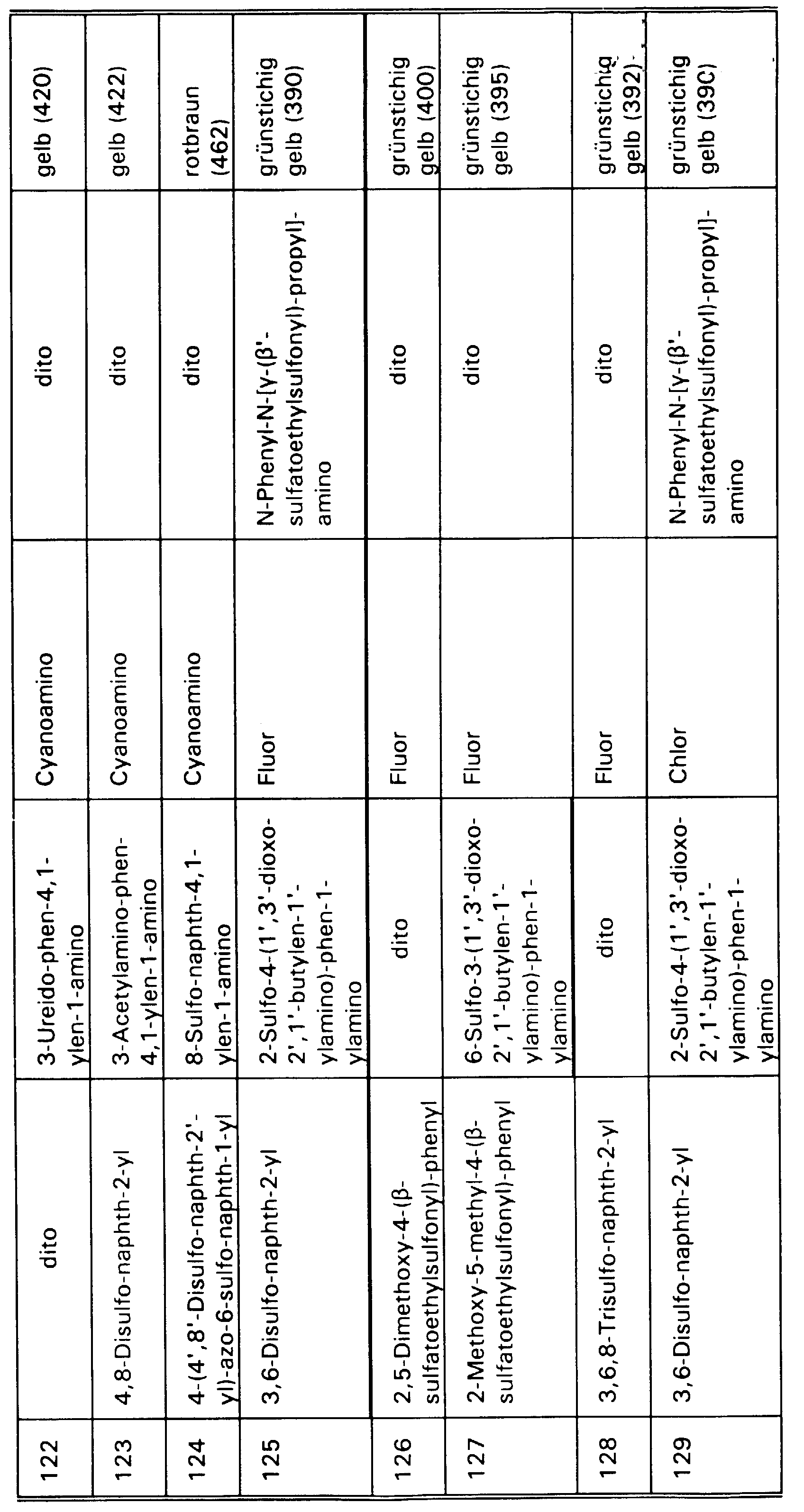

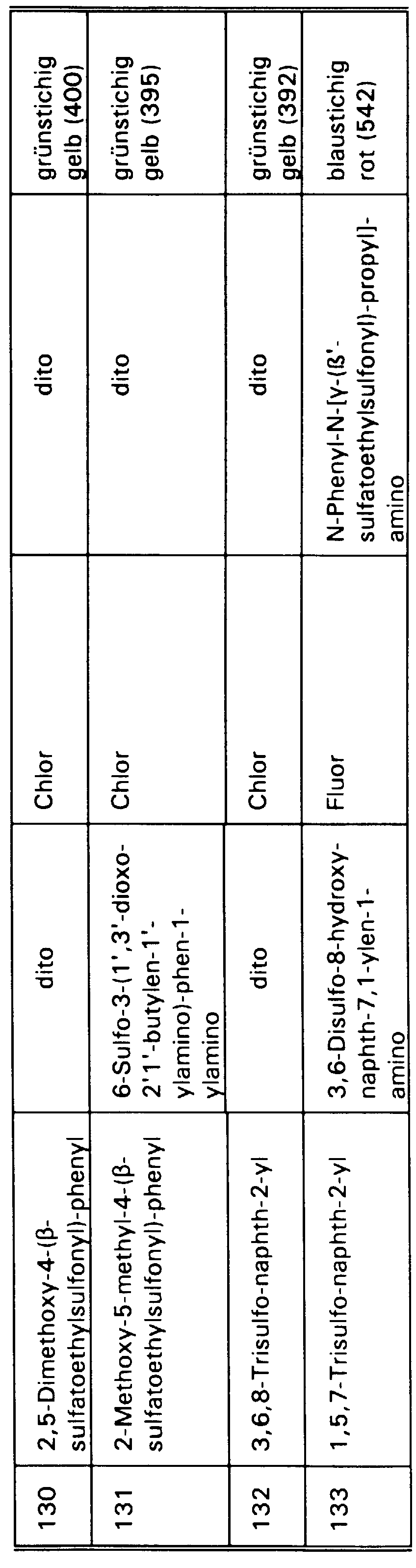

### Beispiel 134

680 Teile einer neutralen wäßrigen Lösung von 0°C von 81 Teilen der Disazoverbindung 2-[4'-(β'-Sulfatoethylsulfonyl)-phenyl]-azo-7-(2"-sulfo-5"-amino-phenyl)-azo-3,6-disulfo-1-amino-8-hydroxy-naphthalin (in üblicher Weise herstellbar durch Kupplungsreaktion von 3,6-Disulfo-1-amino-8-naphthol mit dem Diazoniumsalz von 4-(β-Sulfatoethylsulfonyl)-anilin im stark sauren Bereich und anschließende Kupplungsreaktion der erhaltenen Monoazoverbindung mit dem Monodiazoniumsalz von 1,3-Diaminobenzol-6-sulfonsäure im schwach sauren bis neutralen Bereich) in 600 Teilen Wasser werden unter Einhaltung eines pH-Wertes zwischen 5 und 5,5 und einer Temperatur von 0 bis 5°C langsam mit 15 Teilen Cyanurfluorid versetzt. Anschließend führt man die zweite Kondensationsreaktion nach Zugabe von 38,7 Teilen N-Phenyl-N-[γ-(β'-sulfatoethylsulfonyl)-propyl]amin bei einer Temperatur von 0 bis 5°C und einem pH-Wert von 7 bis 7,5 durch.

Man isoliert den erfindungsgemäßen Disazofarbstoff nach Zugabe von 1 Teil Dinatriumhydrogenphosphat und 3 Teilen Natriumdihydrogenphosphat durch Eindampfen der Syntheselösung, wie beispielsweise durch Sprühtrocknung. Man erhält das Alkalimetallsalz (Natriumsalz) der Verbindung der Formel

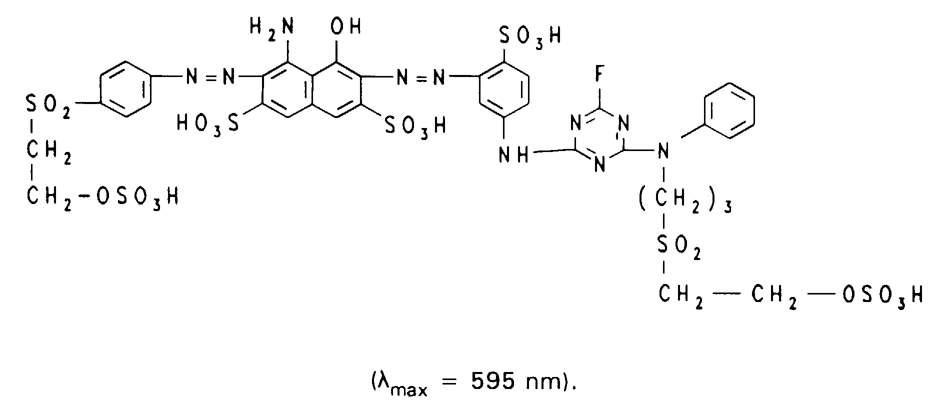

Der erfindungsgemäße Disazofarbstoff besitzt sehr gute faserreaktive Farbstoffeigenschaften und färbt nach den in der Technik für faserreaktive Farbstoffe üblichen Färbe- und Druckverfahren beispielsweise Cellulosefasermaterialien, wie Baumwolle, je nach Einsatz der Farbstoffmenge in hellblauen bis marineblauen Tönen mit guten Echtheitseigenschaften, von denen insbesondere die guten Chlorbadewasserechtheiten hervorgehoben werden können.

### Beispiel 135

Zur Herstellung des erfindungsgemäßen Azofarbstoffes der Formel (in Form der freien Säure geschrieben) 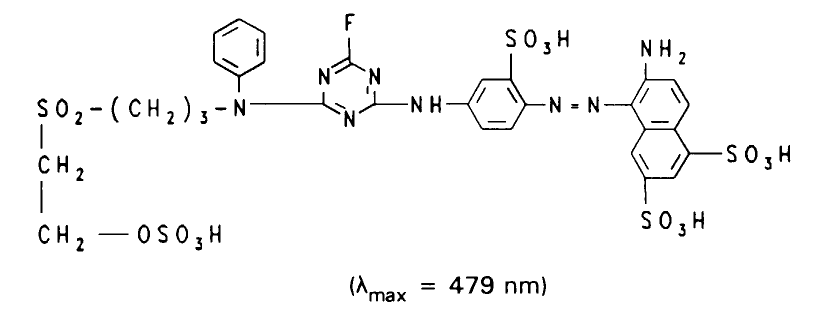 diazotiert man 23,0 Teile 2-Sulfo-4-acetylamino-anilin in 100 Teilen Eiswasser, das 6,9 Teile Natriumnitrit gelöst enthält, durch Zugabe von 5 Teilen konz. wäßriger Salzsäure; die erhaltene Diazoniumsalzsuspension wird sodann bei 0 bis 5°C in eine Lösung von 30,3 Teilen 2-Aminonaphthalin-5,7-disulfonsäure in 570 Teilen Wasser eingerührt. Die Kupplungsreaktion erfolgt bei einer Temperatur von 20°C und einem pH-Wert von 4.

Nach beendeter Kupplungsreaktion werden 150 Teile einer 33%igen wäßrigen Natriumhydroxidlösung hinzugegeben und die Acetylaminogruppe bei 60°C während etwa vier Stunden alkalisch hydrolysiert. Zu der so erhaltenen Lösung der aminogruppenhaltigen Azoverbindung gibt man bei einer Temperatur von 0 bis 2°C und unter Einhaltung eines pH-Wertes von 4 bis 5 15 Teile Cyanurfluorid. Nach Beendigung dieser Kondensationsreaktion versetzt man den Ansatz mit 35,4 Teilen N-Phenyl-N-[γ-(β'-sulfatoethylsulfonyl)-propyl]-amin und führt die letzte Kondensationsreaktion bei einem pH-Wert von 6,5 bis 7,5 und einer Temperatur von 5 bis 10°C durch.

Man isoliert den erhaltenen erfindungsgemäßen Farbstoff in üblicher Weise, beispielsweise durch Aussalzen mit Natriumchlorid. Er zeigt sehr gute faserreaktive Farbstoffeigenschaften und liefert beispielsweise auf Cellulosefasermaterialien farbstarke orange Färbungen mit guten Echtheiten.

### Beispiel 136

Eine unter Zusatz eines Dispergiermittels hergestellte Suspension von 18 Teilen Cyanurchlorid in 500 Teilen Wasser von 0°C wird bei einem pH-Wert zwischen 2 und 2,5 und einer Temperatur zwischen 0 bis 5°C innerhalb von 20 Minuten mit 17,5 Teilen 1,3-Diaminobenzol-5-sulfonsäure versetzt. Man rührt noch eine Stunde nach und gibt sodann eine neutrale Lösung von 36 Teilen N-Phenyl-N-[γ-(β'-sulfatoethylsulfonyl)-propyl]-amin in 200 Teilen Wasser hinzu, erwärmt den Ansatz auf 30°C und führt die Umsetzung bei einem pH-Wert von 5 während zwei Stunden zu Ende. Sodann kühlt man den Ansatz auf 0°C ab, gibt 20 Teile 31 %ige wäßrige Salzsäure hinzu, diazotiert mittels 6,9 Teilen Natriumnitrit, rührt noch zwei Stunden bei 0°C nach, gibt die erhaltene Suspension zu einer Lösung von 19,6 Teilen N-Ethyl-4-methyl-3-aminocarbonyl-2-hydroxy-pyrid-6-on in 300 Teilen Wasser und führt die Kupplungsreaktion bei 20°C und einem pH-Wert von 5 während drei Stunden zu Ende.

Der erfindungsgemäße Azofarbstoff, der, in Form der freien Säure geschrieben, die Formel 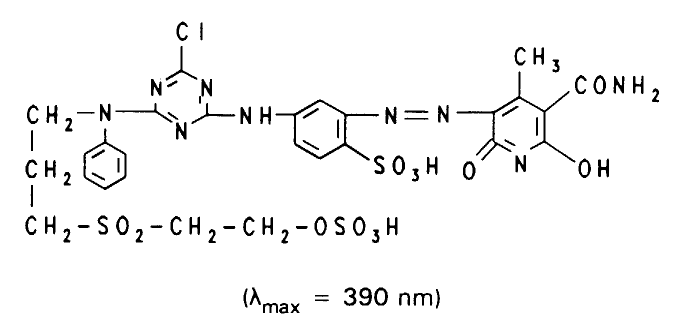 besitzt, wird durch Eindampfen aus der wäßrigen Syntheselösung isoliert. Er färbt beispielsweise Baumwolle in brillanten gelben, echten Farbtönen bei hohem Fixiergrad.

### Beispiele 137 bis 177

In den nachfolgenden Tabellenbeispielen sind weitere erfindungsgemäße Azofarbstoffe entsprechend der allgemeinen Formel (B) 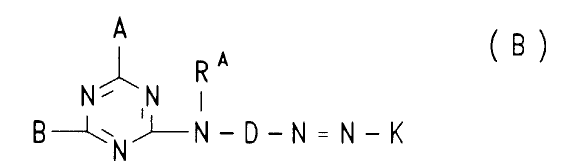 mit Hilfe der daraus ersichtlichen Komponenten beschrieben. Sie lassen sich in einer der erfindungsgemäßen Verfahrensweisen, beispielsweise analog den Angaben der Beispiele 134 bis 136, durch Umsetzung der aus den Formelresten ersichtlichen Ausgangsverbindungen herstellen. Sie besitzen sehr gute faserreaktive Farbstoffeigenschaften und liefern auf den in der Beschreibung genannten Fasermaterialien, wie insbesondere Baumwolle, nach den für faserreaktiven Farbstoffe üblichen Applikations- und Fixierverfahren farbstarke Färbungen und Drucke mit dem in dem jeweiligen Tabellenbeispiel für Baumwolle angegebenen Farbton.

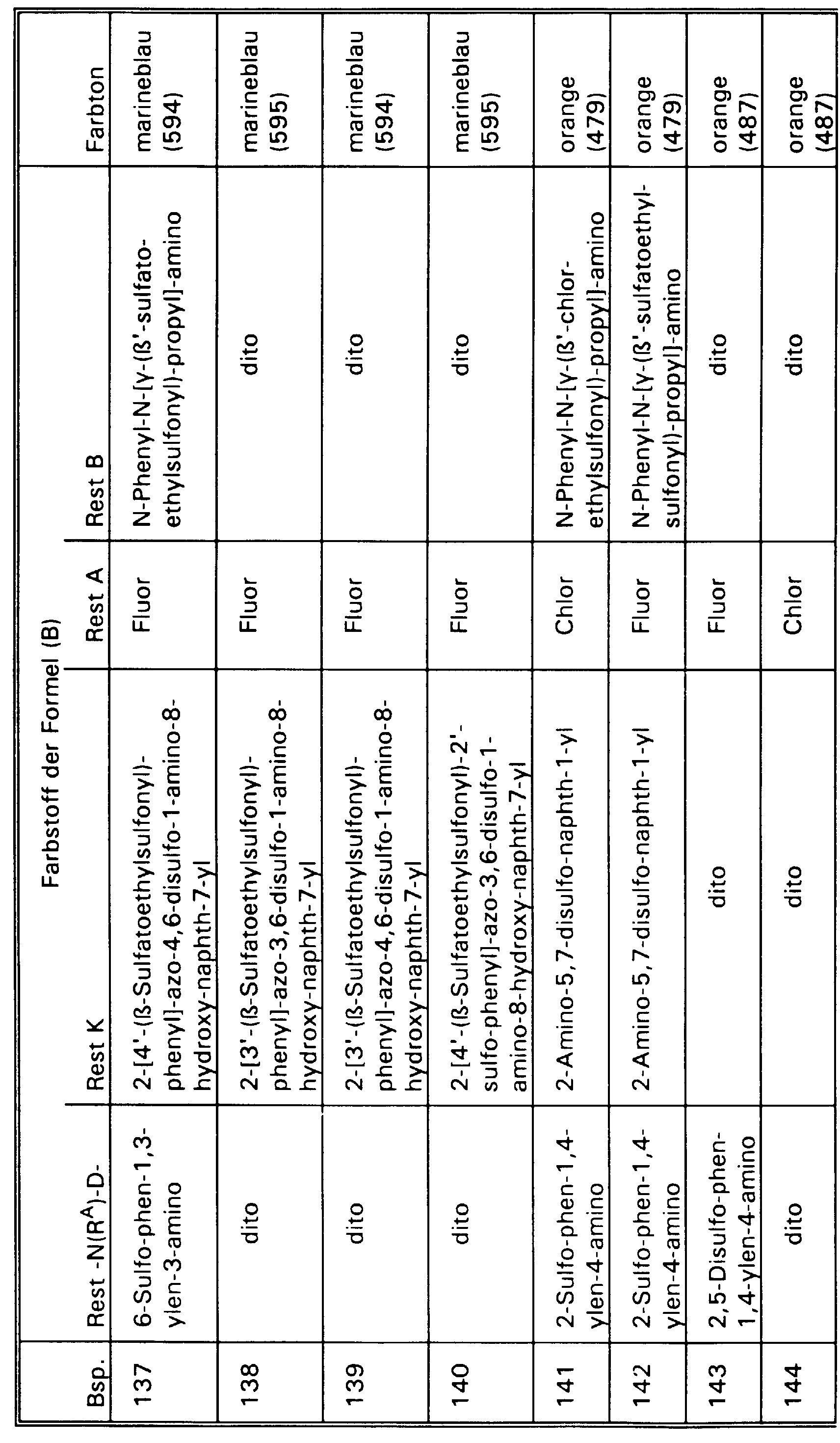

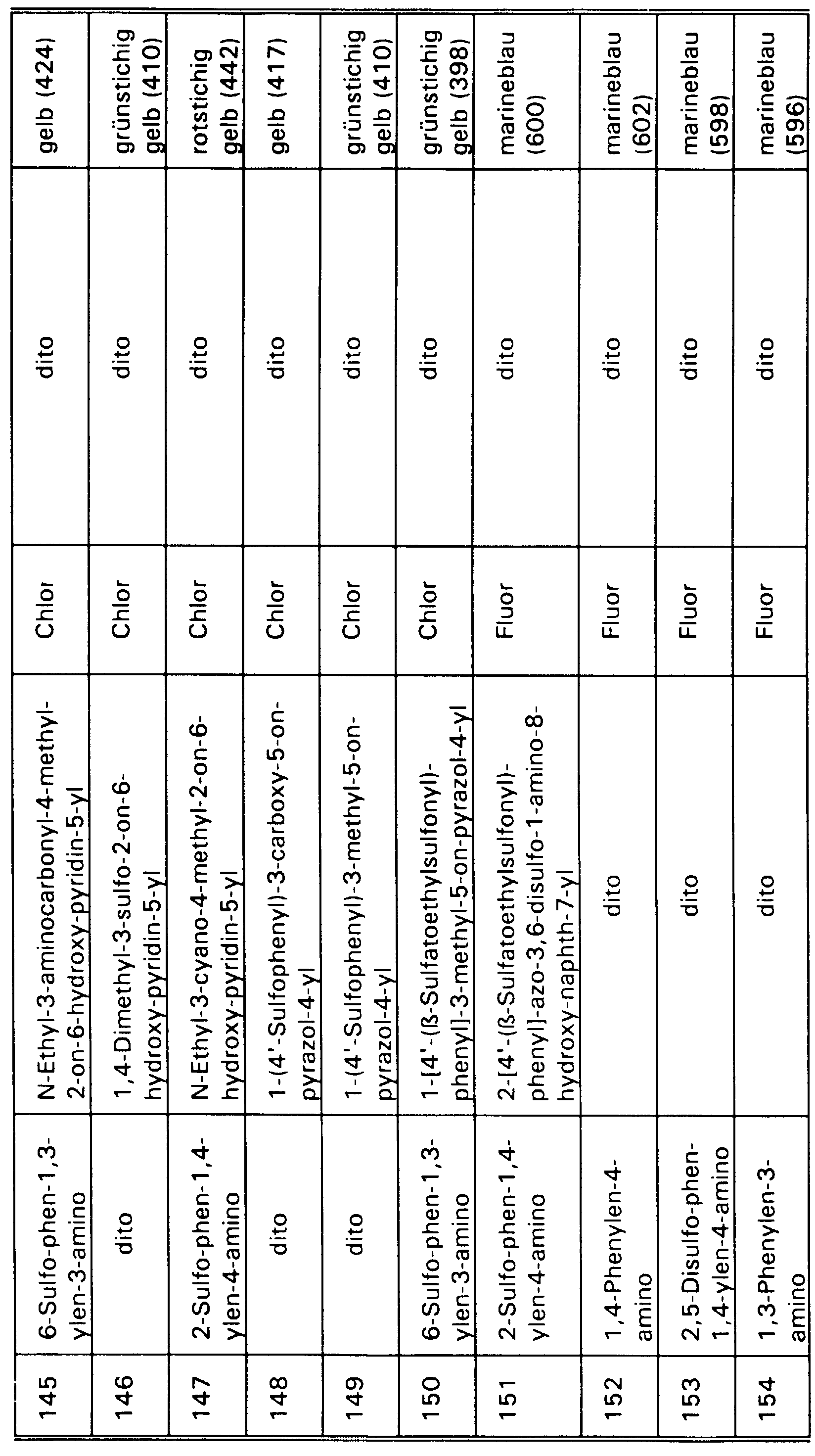

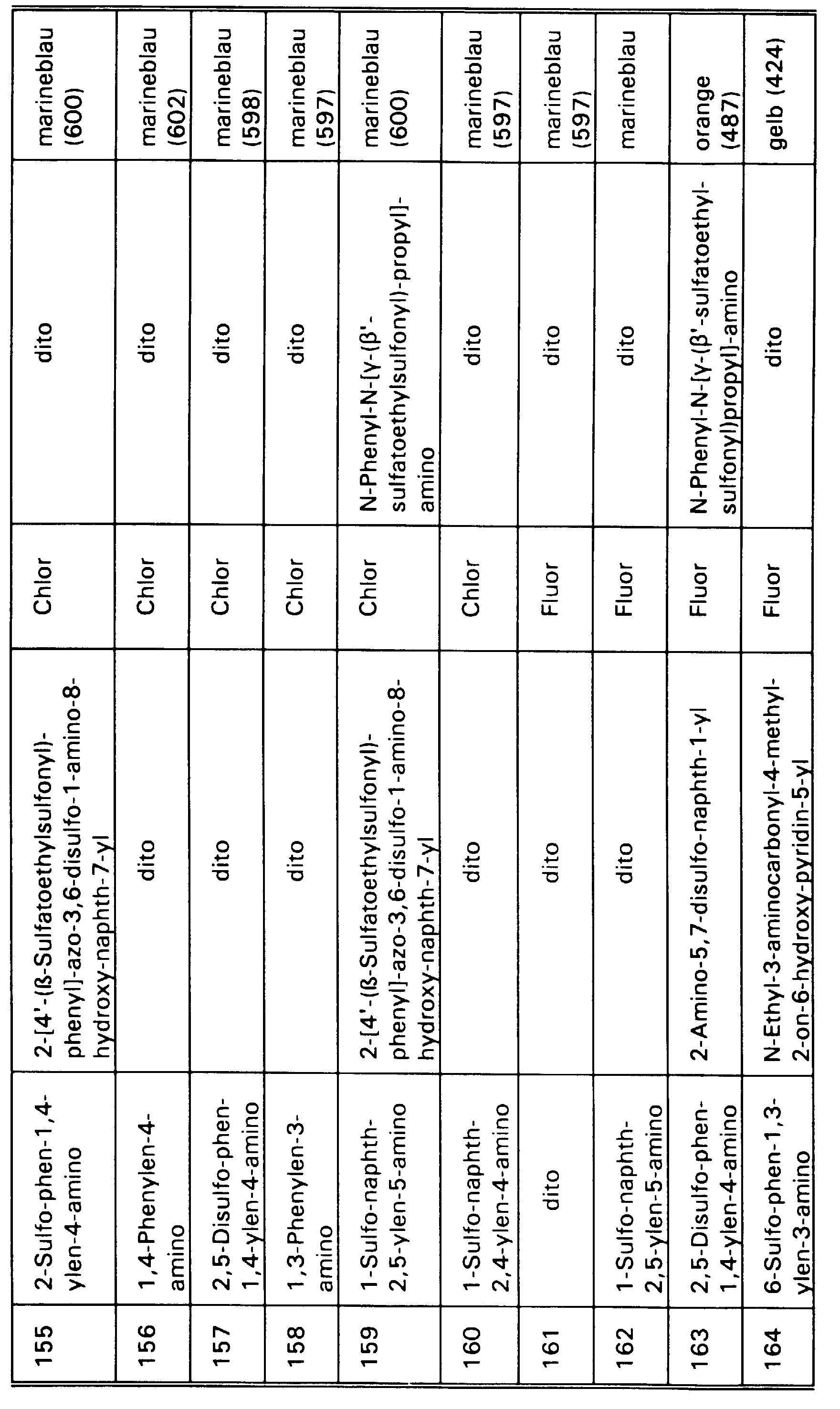

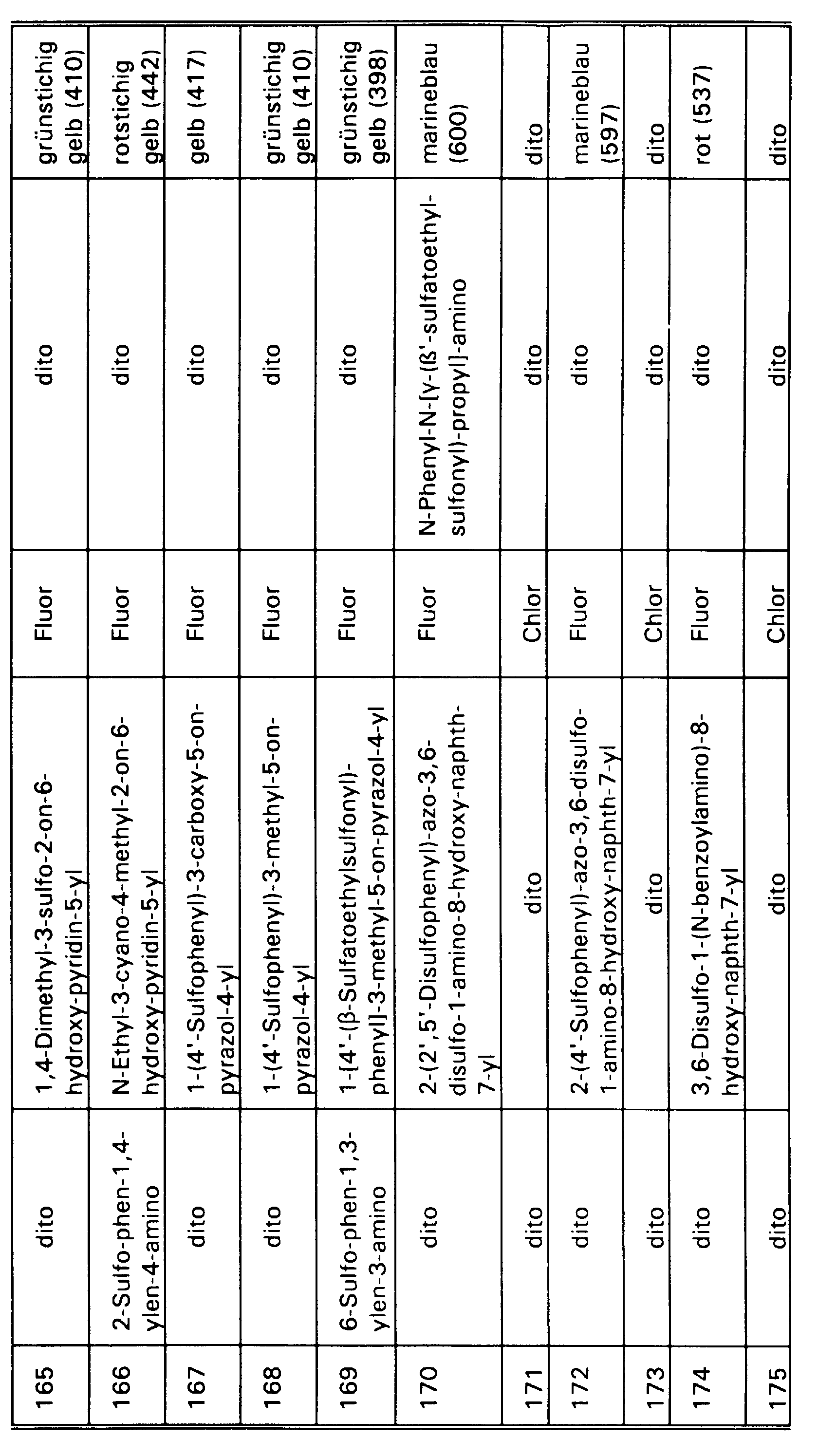

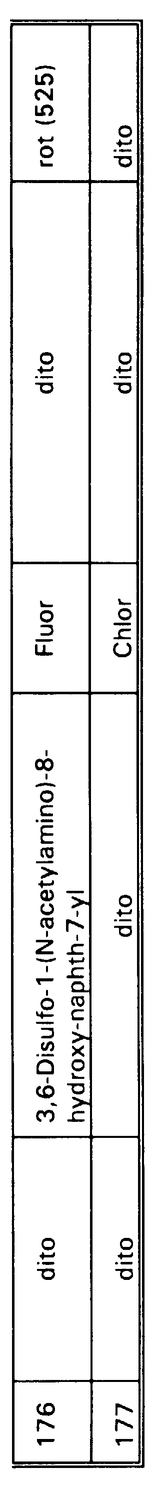

### Beispiel 178

Eine Lösung von 29,8 Teilen der Ausgangsverbindung N-(2-Carboxy-5-sulfophenyl)-N'-(2"-hydroxy-3'-amino-5'-sulfo-phenyl)-ms-phenyl-Kupferformazan wird in einer Mischung aus 300 Teilen Wasser und 300 Teilen Eis innerhalb von etwa 15 Minuten unter gutem Rühren und unter Einhaltung eines pH-Wertes zwischen 6,5 und 7 mittels einer wäßrigen Natriumcarbonatlösung mit einer Lösung von 10 Teilen Cyanurchlorid in 100 Teilen Aceton versetzt. Man rührt danach noch 15 Minuten weiter und gibt sodann 19,5 Teile N-Phenyl-N-[γ-(β'-sulfatoethylsulfonyl)-propyl]-amin in 270 Teilen Wasser hinzu und führt die Umsetzung bei einem pH-Wert von 4 und einer Temperatur von 30 bis 35°C innerhalb von etwa 3 Stunden zu Ende.

Der erfindungsgemäße Kupferformazanfarbstoff der Formel (in Form der freien Säure geschrieben) 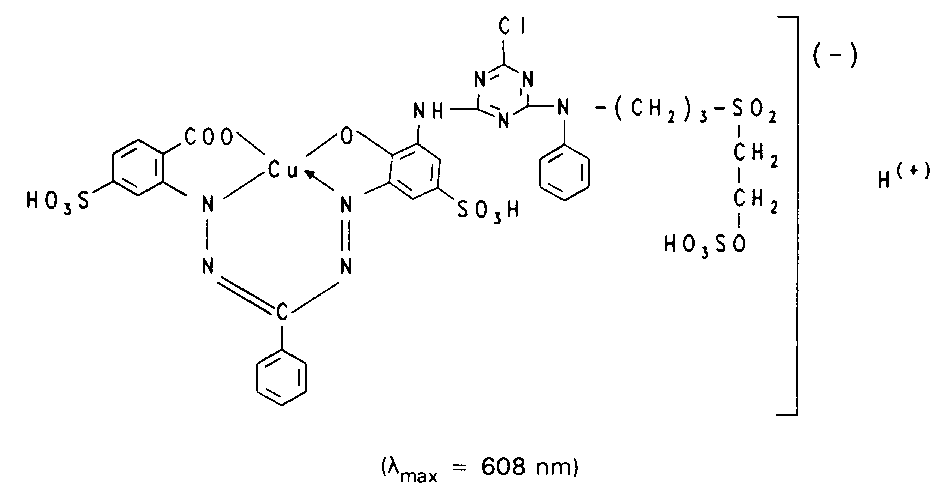 wird aus der Syntheselösung durch Natriumchlorid ausgesalzen. Er färbt beispielsweise Baumwolle nach den in der Technik für faserreaktive Farbstoffe üblichen Färbeweisen in farbstarken, blauen Tönen mit guten Echtheiten.

### Beispiel 179

Eine Lösung von 48,4 Teilen des Natriumsalzes von N-Phenyl-N-[γ-(β'-sulfatoethylsulfonyl)-propyl]-amin in 1300 Teilen eines Wasser/Eis-Gemisches wird innerhalb kurzer Zeit unter intensivem Rühren mit 12 Vol.-Teilen Cyanurfluorid versetzt. Man rührt noch kurze Zeit nach und gibt sodann zu dieser Lösung 124,6 Teile der Ausgangsverbindung N-(2-Carboxy-5-sulfophenyl)-N'-(2'-hydroxy-3'-amino-5'-sulfo-phenyl)-ms-phenyl-Kupferformazan hinzu und führt die Umsetzung bei einem pH-Wert von 9,5 und einer Temperatur von 0 bis 3°C durch. Man rührt noch einige Zeit nach, stellt den pH-Wert auf 7 und erwärmt den Ansatz auf 20°C.

Der erfindungsgemäße Kupferformazanfarbstoff der Formel (in Form der freien Säure geschrieben) 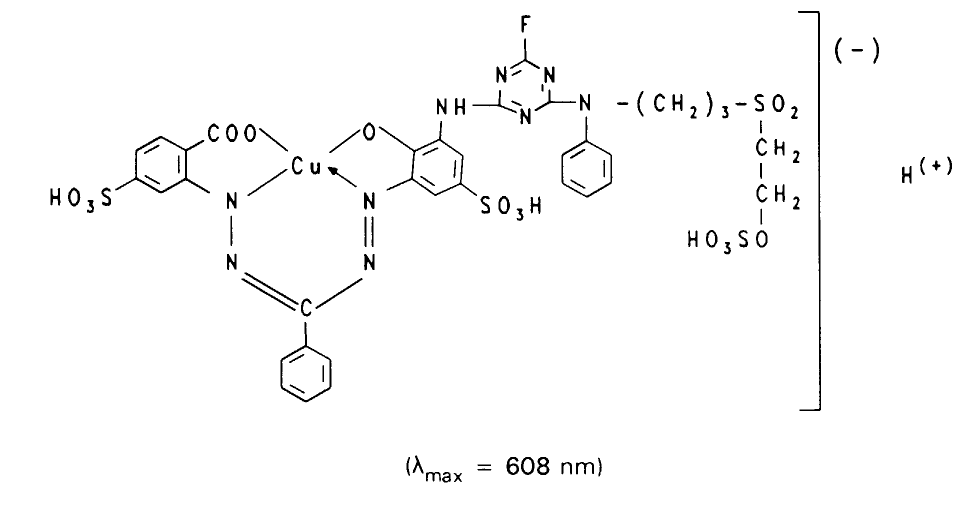 wird aus der Syntheselösung durch Natriumchlorid ausgesalzen. Er färbt beispielsweise Baumwolle nach den in der Technik für faserreaktive Farbstoffe üblichen Färbeweisen in farbstarken, blauen Tönen mit guten Echtheiten.

### Beispiel 180

Eine neutrale Lösung von 26,5 Teilen 1-Amino-4-(2',4',6'-trimethyl-3'-aminophenylamino)-anthrachinon-2,5'-disulfonsäure wird unter gutem Rühren bei 0°C und unter Einhaltung eines pH-Wertes von 5 innerhalb von 30 Minuten mit 7,1 Teilen Cyanurfluorid versetzt. Man rührt noch einige Zeit bis zur Beendigung der Umsetzung nach, gibt sodann 16,85 Teile N-Phenyl-N-[γ-(β'-sulfatoethylsulfonyl)-propyl]-amin hinzu, führt die Umsetzung bei einem pH-Wert von 8,5 und bei einer Temperatur von 5°C zu Ende und rührt anschließend noch einige Zeit bei einem pH-Wert von 7 und bei 20°C nach.

Der erfindungsgemäße Anthrachinonfarbstoff der Formel (in Form der freien Säure geschrieben) 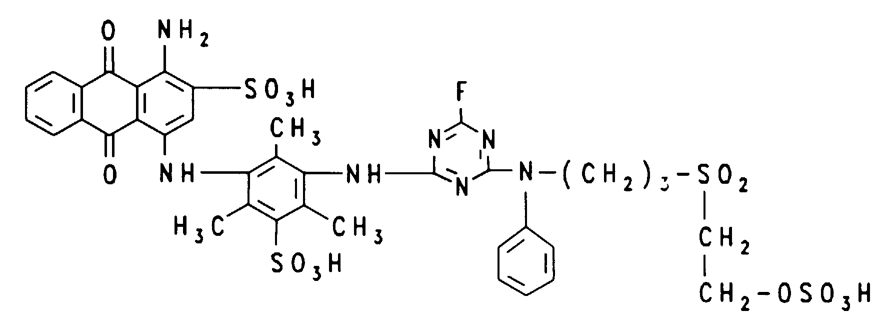 wird durch Aussalzen aus der Syntheselösung mittels Kaliumchlorid isoliert. Er zeigt sehr gute faserreaktive Farbstoffeigenschaften und färbt die in der Beschreibung genannten Materialien, wie beispielsweise Cellulosefasermaterialien, in brillanten blauen Tönen mit guten Echtheitseigenschaften.

### Beispiel 181

Man verfährt zur Herstellung des erfindungsgemäßen Anthrachinonfarbstoffes der Formel (in Form der freien Säure geschrieben) 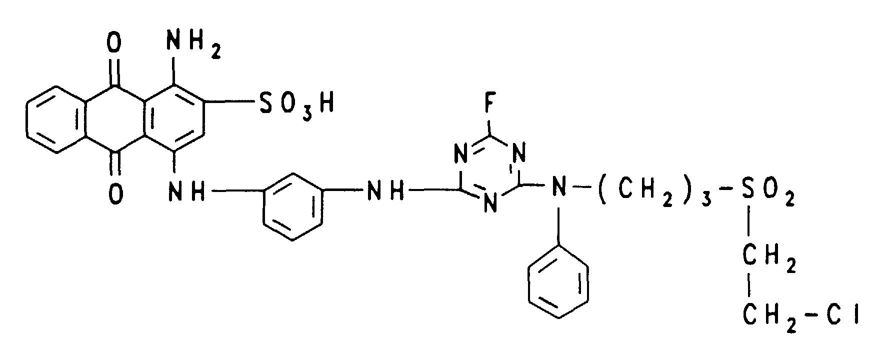 gemäß der Verfahrensweise des Beispieles 180 mit der Ausnahme, daß man anstelle der dort angegebenen Amino-anthrachinon-Ausgangsverbindung von 19,8 Teilen 1-Amino-4-(3'-aminophenyl-amino)-anthrachinon-2-sulfonsäure in 500 Teilen einer neutralen wäßrigen Lösung von etwa 0°C ausgeht. Der erfindungsgemäße Anthrachinonfarbstoff zeigt ebenfalls sehr gute faserreaktive Farbstoffeigenschaften und färbt beispielsweise Baumwolle nach den in der Technik für faserreaktive Farbstoffe üblichen Färbe- und Druckverfahren in echten, farbstarken blauen Tönen.

### Beispiel 182

Man verfährt zur Herstellung des erfindungsgemäßen Anthrachinonfarbstoffes gemäß der Verfahrensweise des Beispieles 180 mit der Ausnahme, daß man anstelle der dort angegebenen Amino-anthrachinon-Ausgangsverbindung von 19,8 Teilen 1-Amino-4-(4'-aminophenyl-amino)-anthrachinon-2-sulfonsäure in 500 Teilen einer neutralen wäßrigen Lösung von etwa 0°C ausgeht. Der erfindungsgemäße Anthrachinonfarbstoff zeigt ebenfalls sehr gute faserreaktive Farbstoffeigenschaften und färbt beispielsweise Baumwolle nach den in der Technik für faserreaktive Farbstoffe üblichen Färbe- und Druckverfahren in echten, farbstarken blauen Tönen.

### Beispiel 183

Eine neutrale Lösung von 55 Teilen der Triphendioxazinverbindung der Formel 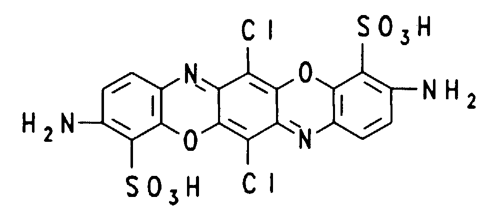 in 500 Teilen Wasser wird bei 0°C und unter Einhaltung eines pH-Wertes zwischen 5 und 6 unter gutem Rühren langsam mit 29 Teilen Cyanurfluorid versetzt. Man rührt noch kurze Zeit nach und gibt sodann 68 Teile N-Phenyl-N-[γ-(β'-sulfatoethylsulfonyl)-propyl]-amin hinzu und führt die Umsetzung bei 0 bis 10°C unter Einhaltung eines pH-Wertes von 8 bis 9 zu Ende.

Der erfindungsgemäße Triphendioxazinfarbstoff, der, in Form der freien Säure geschrieben, die Formel 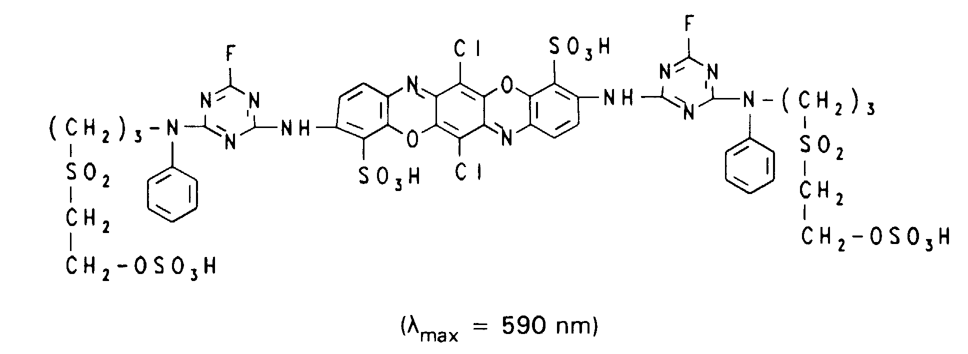 besitzt, wird durch Aussalzen mit Natriumchlorid isoliert. Er zeigt gute faserreaktive Farbstoffeigenschaften und färbt nach den in der Technik für faserreaktive Farbstoffe üblichen Applikations- und Fixierverfahren auf den in der Beschreibung genannten Materialien, wie insbesondere Cellulosefasermaterialien, in farbstarken blauen Tönen mit guten Echtheitseigenschaften.

### Beispiel 184

Unter gutem Rühren gibt man in eine neutrale Lösung von 65 Teilen der Ausgangsverbindung 1,6-Disulfo-2,7-di-(γ-amino-propylamino)-5,10-dichlortriphendioxazin in 500 Teilen Wasser bei 0°C 25 Teile Cyanurfluorid und hält hierbei den pH-Wert bei etwa 6 mittels einer wäßrigen 2N Natriumhydroxidlösung aufrecht. Man rührt noch einige Zeit nach, versetzt den Ansatz mit 68 Teilen N-Phenyl-N-[γ-(β'-sulfatoethylsulfonyl)-propyl]-amin und führt die Umsetzung bei einer Temperatur von 0 bis 10°C unter Einhaltung eines pH-Wertes von 8 bis 9 zu Ende.

Der erfindungsgemäße Triphendioxazinfarbstoff wird in üblicher Weise durch Aussalzen mit Natriumchlorid aus der Syntheselösung isoliert. Er besitzt, in Form der freien Säure geschrieben, die Formel 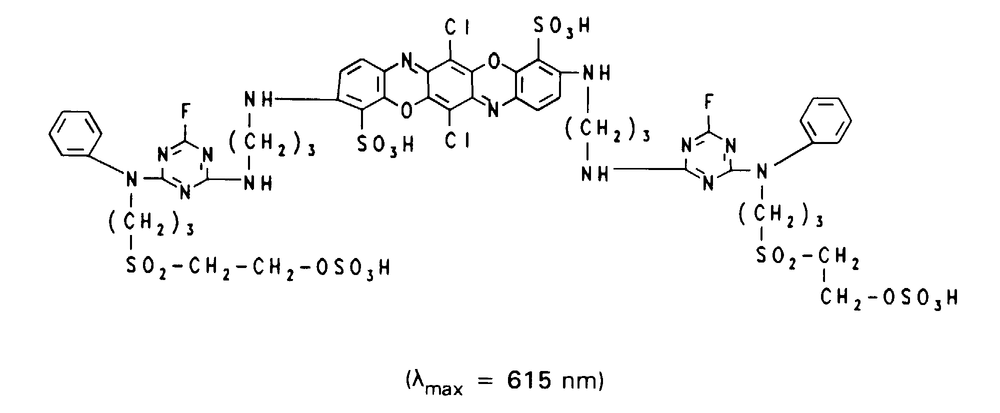 und färbt die in der Beschreibung genannten Fasermaterialien, wie insbesondere Cellulosefasermaterialien, nach den in der Technik für faserreaktive Farbstoffe üblichen Applikations- und Fixierverfahren in farbstarken, blauen Tönen mit guten Echtheitseigenschaften.

### Beispiel 185

58,9 Teile 2,9-Diamino-6,13-dichlor-1,8-disulfo-triphendioxazin werden in 1500 Teilen Wasser angerührt und mit einer wäßrigen Lithiumhydroxidlösung auf einen pH-Wert von 7 eingestellt. Man gibt bei 20 bis 25°C 60 Teile Cyanurchlorid hinzu, wobei man den pH-Wert mittels einer wäßrigen Lithiumhydroxidlösung zwischen 4 und 5 hält. Man rührt den Ansatz noch eine Weile weiter, bis die Umsetzung abgeschlossen ist, saugt die ausgefallene Dichlortriazinylamino-Verbindung ab, rührt den feuchten Filterkuchen in 1000 Teile Wasser ein, gibt sodann 66 Teile N-Phenyl-N-[γ-(β'-sulfatoethylsulfonyl)-propyl]-amin, gelöst in 200 Teilen Wasser, hinzu, erhöht die Temperatur auf 60°C und rührt bei einem pH-Wert zwischen 4 und 5 bis zur vollständigen Umsetzung weiter.

Man isoliert den erfindungsgemäßen Triphendioxazinfarbstoff der Formel (in Form der freien Säure geschrieben) 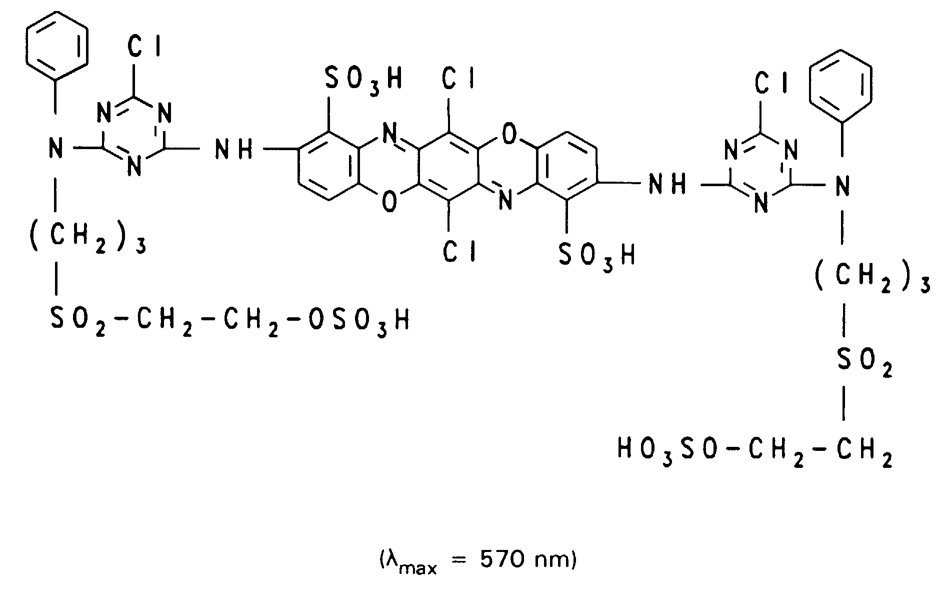 durch Aussalzen mittels Natriumchlorid als Alkalimetallsalz (Natriumsalz). Der erfindunsgemäße Farbstoff zeigt sehr gute faserreaktive Eigenschaften und liefert auf den in der Beschreibung genannten Materialien, insbesondere Cellulosefasermaterialien, nach den in der Technik für faserreaktive Farbstoffe üblichen Anwendungsverfahren farbstarke, brillante rotstichig blaue Färbungen und Drucke in hohen Fixierquoten und guten Echtheitseigenschaften.

### Beispiele 186 bis 202

In den nachfolgenden Tabellenbeispielen sind weitere erfindungsgemäße Triphendioxazinfarbstoffe entsprechend der allgemeinen Formel (C) 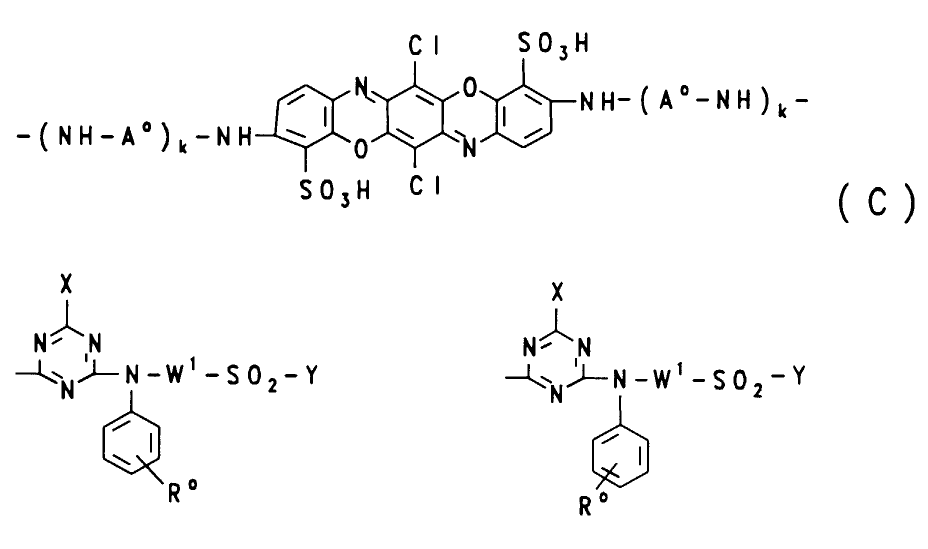 mit Hilfe der daraus ersichtlichen Komponenten beschrieben. Sie lassen sich in erfindungsgemäßer Weise, beispielsweise analog den Angaben der Beispiele 183 bis 185, durch Umsetzung der aus den Formelresten von Formel (C) ersichtlichen Ausgangsverbindungen herstellen. Sie besitzen sehr gute faserreaktive Farbstoffeigenschaften und liefern auf den in der Beschreibung genannten Fasermaterialien, wie insbesondere Baumwolle, nach den für faserreaktive Farbstoffe üblichen Applikations- und Fixierverfahren farbstarke Färbungen und Drucke mit dem in dem jeweiligen Tabellenbeispiel für Baumwolle angegebenen Farbton.

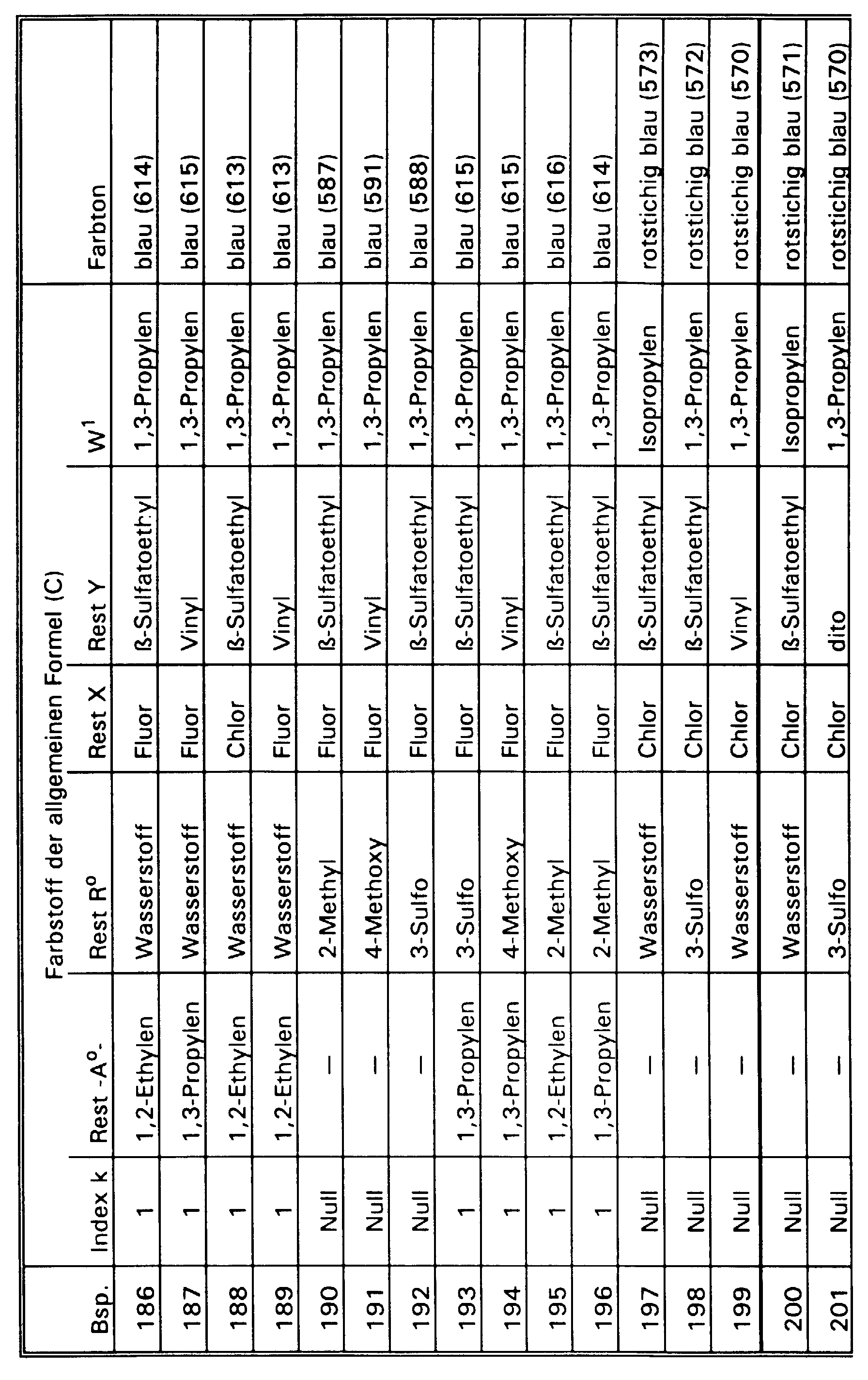

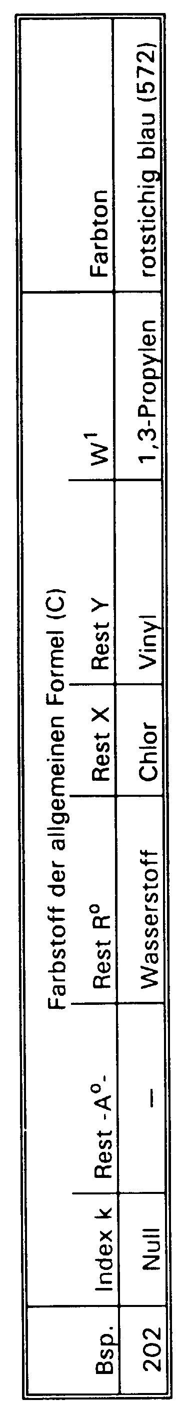

### Beispiel 203

Eine neutrale Lösung von 50,3 Teilen der Verbindung 3-(m-Amino-p-sulfophenyl-aminosulfonyl)-kupferphthalocyanin-3',3",3''-trisulfonsäure in 500 Teilen Wasser wird bei 0 bis 3°C unter Einhaltung eines pH-Wertes von 6 bis 7 innerhalb von etwa 30 Minuten mit 7,1 Teilen Cyanurchlorid versetzt. Man rührt unter Einhaltung dieser Verfahrensbedingungen noch einige Zeit nach, setzt 16,8 Teile N-Phenyl-N-[γ-(β'-sulfatoethylsulfonyl)-propyl]-amin hinzu und führt die Umsetzung bei einem pH-Wert von etwa 5 und einer Temperatur von etwa 35°C durch.

Der erhaltene erfindungsgemäße Kupferphthalocyaninfarbstoff der Formel (in Form der freien Säure geschrieben) 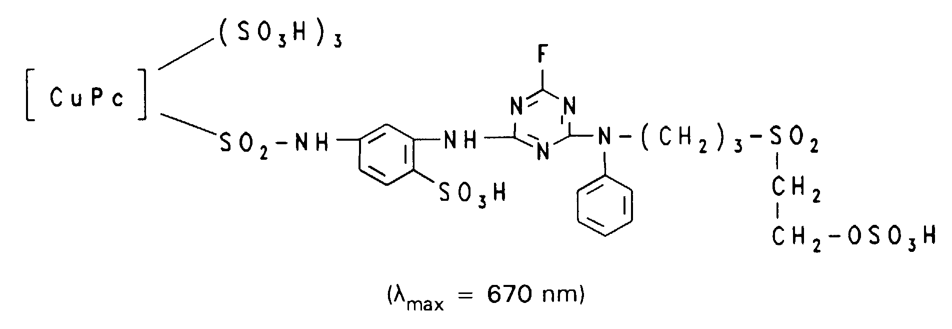 wird durch Natriumchlorid aus der Syntheselösung ausgesalzen. Er färbt beispielsweise Baumwolle, Zellwolle und Regeneratcellulose in farbstarken, türkisblauen, echten Tönen.

### Beispiel 204

Zur Herstellung eines erfindungsgemäßen Kupferphthalocyaninfarbstoffes verfährt man gemäß der Verfahrensweise des Beispieles 203, setzt jedoch anstelle der dort verwendeten Kupferphthalocyanin-Ausgangsverbindung die 3-(p-Amino-m-sulfo-phenyl-aminosulfonyl)-kupferphthalocyanin-3',3',3"'-trisulfonsäure.

Der erfindungsgemäße Kupferphtha!ocyaninfarbstoff zeigt in wäßriger Lösung im sichtbaren Bereich ein Absorptionsmaximum bei 670 nm und liefert als Farbstoff mit guten faserreaktiven Eigenschaften beispielsweise auf Baumwolle farbstarke türkisblaue, echte Färbungen und Drucke.

### Beispiel 205

Eine unter Zusatz eines Dispergiermittels hergestellte Suspension von 18 Teilen Cyanurchlorid in 500 Teilen Wasser von 0°C wird bei einem pH-Wert zwischen 2 und 2,5 und einer Temperatur zwischen 0 bis 5°C innerhalb von 20 Minuten mit 17,5 Teilen 1,3-Diaminobenzol-5-sulfonsäure versetzt. Man rührt noch eine Stunde nach und gibt sodann eine neutrale Lösung von 36 Teilen N-Phenyl-N-[γ-(β'-sulfatoethylsulfonyl)-propyl]-amin in 200 Teilen Wasser hinzu, erwärmt den Ansatz auf 30°C und führt die Umsetzung bei einem pH-Wert von 5 während zwei Stunden zu Ende. Sodann kühlt man den Ansatz auf 0°C ab, gibt 20 Teile 31 %ige wäßrige Salzsäure hinzu, diazotiert mittels 6,9 Teilen Natriumnitrit, rührt noch zwei Stunden bei 0°C nach, gibt die erhaltene Suspension zu einer Lösung von 19,6 Teilen N-Ethyl-4-methyl-3-aminocarbonyl-2-hydroxy-pyrid-6-on in 300 Teilen Wasser und führt die Kupplungsreaktion bei 20°C und einem pH-Wert von 5 während drei Stunden zu Ende.

Der erfindungsgemäße Azofarbstoff, der, in Form der freien Säure geschrieben, die Formel 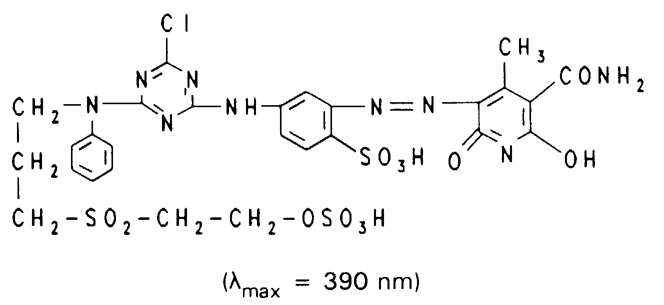 besitzt, wird durch Eindampfen aus der wäßrigen Syntheselösung isoliert. Er färbt beispielsweise Baumwolle in brillanten gelben, echten Farbtönen bei hohem Fixiergrad.

## Patentansprüche

1. Farbstoff der allgemeinen Formel (1)
F - Zₙ (1)
in welcher bedeuten:
F ist der Rest eines sulfogruppenhaltigen Mono-, Dis- oder Polyazofarbstoffes oder eines Schwermetallkomplex-Mono-, -Dis- oder -Trisazofarbstoffes oder eines Anthrachinon-, Azomethin-, Phenazin-, Stilben-, Triphenylmethan-, Xanthen-, Thioxanthen-, Nitroaryl-, Naphthochinon-, Pyrenchinon-, Perylentetracarbimid-, Formazan-, Kupferformazan-, Phthalocyanin-, Kupferphthalocyanin-, Nickelphthalocyanin- oder Kobaltphthalocyanin-Farbstoffes oder eines Triphendioxazin-Farbstoffes;
Z ist eine Gruppe der allgemeinen Formel (2) 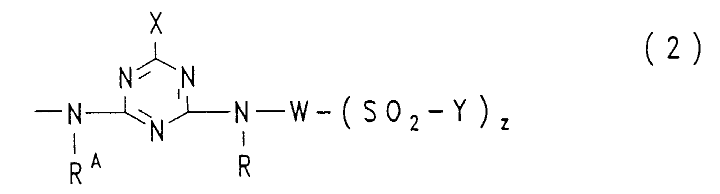 in welcher
R^{A} Wasserstoff oder Alkyl von 1 bis 4 C-Atomen ist, das durch Halogen, Hydroxy, Cyano, Alkoxy von 1 bis 4 C-Atomen, Alkoxycarbonyl von 2 bis 5 C-Atomen, Carboxy, Sulfamoyl, Sulfo, Sulfato oder Phosphato substituiert sein kann,
X Halogen, Cyanoamino, Methylsulfonylamino, Ethylsulfonylamino, Carboxy-pyridinyl, Aminocarbonyl-pyridinyl oder ein Rest der Formel -N(R)-W-(SO₂-Y)_{z} mit R, W, Y und z einer der nachstehend angegebenen Bedeutungen ist,
R Phenyl ist, das durch 1, 2 oder 3 Substituenten aus der Gruppe Halogen, Alkyl von 1 bis 4 C-Atomen, Alkoxy von 1 bis 4 C-Atomen, Sulfo und Carboxy substituiert sein kann,
W geradkettiges oder verzweigtes Alkylen von 3 bis 8 C-Atomen ist, das durch 1 oder 2 Substituenten aus der Gruppe Alkoxy von 1 bis 6 C-Atomen, Halogen, Alkoxycarbonyl von 2 bis 5 C-Atomen, Carboxy, Sulfo und Sulfato oder durch einen Phenylrest oder einen durch 1 oder 2 Substituenten aus der Gruppe Sulfo, Carboxy, Methoxy, Ethoxy und Methyl substituierten Phenylrest oder durch solch einen gegebenenfalls substituierten Phenylrest und eine Sulfo-, Sulfato- oder Carboxygruppe oder durch einen heterocyclischen Rest substituiert sein kann,
Y Vinyl, β-Sulfatoethyl, β-Thiosulfatoethyl, β-Phosphatoethyl, β-(C₂-C₅ Alkanoyloxy)-ethyl, β-Benzoyloxyethyl, β-(Sulfobenzoyloxy)-ethyl oder β-(p-Toluolsulfonyloxy)-ethyl ist, und
z die Zahl 1 oder 2 ist, und
n die Zahl 1 oder 2 ist.

2. Farbstoff nach Anspruch 1, dadurch gekennzeichnet, daß F der Rest eines sulfogruppenhaltigen Mono- oder Disazofarbstoffes ist.

3. Farbstoff nach Anspruch 1, dadurch gekennzeichnet, daß F der Rest eines 1:1-Kupferkomplex-Mono- oder -Disazofarbstoffes ist.

4. Farbstoff nach Anspruch 1, dadurch gekennzeichnet, daß F der Rest eines Anthrachinonfarbstoffes ist.

5. Farbstoff nach Anspruch 1, dadurch gekennzeichnet, daß F der Rest eines Kupferformazan-Farbstoffes ist.

6. Farbstoff nach Anspruch 1, dadurch gekennzeichnet, daß F der Rest eines Kupferphthalocyanin- oder Nickelphthalocyanin-Farbstoffes ist.

7. Farbstoff nach Anspruch 1, dadurch gekennzeichnet, daß F der Rest eines Triphendioxazinfarbstoffes ist.

8. Farbstoff nach mindestens einem der Ansprüche 1 bis 7, dadurch gekennzeichnet, daß X Chlor oder Fluor ist.

9. Farbstoff nach mindestens einem der Ansprüche 1 bis 7, dadurch gekennzeichnet, daß X Cyanoamino ist.

10. Farbstoff nach mindestens einem der Ansprüche 1 bis 9, dadurch gekennzeichnet, daß W n-Propylen ist.

11. Farbstoff nach mindestens einem der Ansprüche 1 bis 10, dadurch gekennzeichnet, daß R Phenyl ist, das durch 1 oder 2 Substituenten aus der Gruppe Methyl, Methoxy und Sulfo substituiert sein kann.

12. Farbstoff nach mindestens einem der Ansprüche 1 bis 11, dadurch gekennzeichnet, daß R^{A} Wasserstoff ist.

13. Verfahren zur Herstellung eines Farbstoffes entsprechend der allgemeinen Formel (1) von Anspruch 1, dadurch gekennzeichnet, daß man für den jeweiligen Farbstoff typische Vorprodukte, von denen mindestens eines eine Gruppe der allgemeinen Formel (2) enthält, miteinander umsetzt oder daß man eine Halogen-triazin-Verbindung der allgemeinen Formel (21) 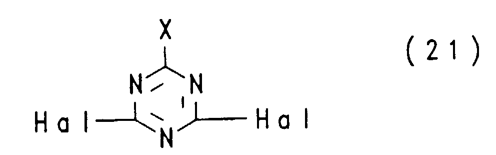 in welcher X eine der in Anspruch 1 genannten Bedeutungen hat und Hal für Halogen steht, mit einer aminogruppenhaltigen Verbindung der allgemeinen Formel (20) 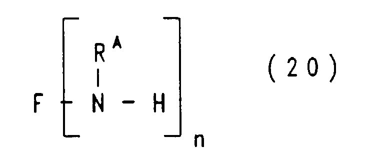 mit F, R^{A} und n der in Anspruch 1 genannten Bedeutungen und einer Aminoverbindung der allgemeinen Formel (22) 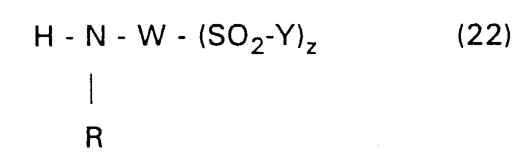 mit R, W, Y und z der in Anspruch 1 genannten Bedeutungen in beliebiger Reihenfolge umsetzt.

14. Verwendung eines Farbstoffes von Anspruch 1 oder eines nach Anspruch 13 hergestellten Farbstoffes zum Färben (einschließlich Bedrucken) von hydroxy- und/oder carbonamidgruppenhaltigem Material, insbesondere Fasermaterial.

15. Verfahren zum Färben (einschließlich Bedrucken) von hydroxy- und/oder carbonamidgruppenhaltigem Material, insbesondere Fasermaterial, bei welchem man einen Farbstoff auf das Material aufbringt und den Farbstoff auf dem Material mittels Wärme oder mit Hilfe eines alkalisch wirkenden Mittels oder mittels beider Maßnahmen fixiert, dadurch gekennzeichnet, daß man als Farbstoff einen Farbstoff von Anspruch 1 oder einen nach Anspruch 13 hergestellten Farbstoff einsetzt.

16. Verbindung entsprechend der allgemeinen Formel (22A) 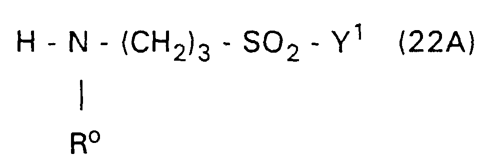 in welcher Y¹ Vinyl ist oder β-Sulfatoethyl, β-Thiosulfatoethyl, β-Phosphatoethyl, β-(C₂-C₅)-Alkanoyloxy-ethyl, β-Benzoyloxyethyl, β-(Sulfobenzoyloxy)-ethyl, β-(p-Toluolsulfonyloxy)-ethyl, oder β-Hydroxyethyl und R^{o} Phenyl ist, das durch 1, 2 oder 3 Substituenten aus der Gruppe Halogen, Alkyl von 1 bis 4 C-Atomen, Alkoxy von 1 bis 4 C-Atomen, Sulfo und Carboxy substituiert sein kann.

17. Verwendung einer Verbindung von Anspruch 16 als Ausgangsverbindung zur Synthese von faserreaktiven Verbindungen, insbesondere Farbstoffen.

## Claims

1. A dye of the formula (1)
F-Zₙ (1)
in which:
F is the radical of a sulfo-containing mono-, dis- or polyazo dye or of a heavy metal complex monoazo, heavy metal complex disazo or heavy metal complex trisazo dye or of an anthraquinone, azomethine, phenazine, stilbene, triphenylmethane, xanthene, thioxanthene, nitroaryl, naphthoquinone, pyrenequinone, perylenetetracarbimide, formazan, copper formazan, phthalocyanine, copper phthalocyanine, nickel phthalocyanine or cobalt phthalocyanine dye or of a triphendioxazine dye;
z is a group of the formula (2) 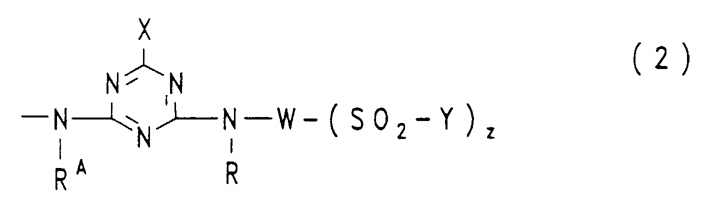 in which
R^{A} is hydrogen or alkyl of 1 to 4 carbon atoms, which may be substituted by halogen, hydroxyl, cyano, alkoxy of 1 to 4 carbon atoms, alkoxycarbonyl of 2 to 5 carbon atoms, carboxyl, sulfamoyl, sulfo, sulfato or phosphato,
X is halogen, cyanoamino, methylsulfonylamino, ethylsulfonylamino, carboxypyridinyl, aminocarbonylpyridinyl or a radical of the formula -N(R)-W-(SO₂-Y)_{z} where R, W, Y and z have one of the meanings given below,
R is phenyl which may be substituted by 1, 2 or 3 substituents from the group comprising halogen, alkyl of 1 to 4 carbon atoms, alkoxy of 1 to 4 carbon atoms, sulfo and carboxyl,
W is straight-chain or branched alkylene of 3 to 8 carbon atoms, which may be substituted by 1 or 2 substituents from the group comprising alkoxy of 1 to 6 carbon atoms, halogen, alkoxycarbonyl of 2 to 5 carbon atoms, carboxyl, sulfo and sulfato, or by a phenyl radical or a phenyl radical which is substituted by 1 or 2 substituents from the group comprising sulfo, carboxyl, methoxy, ethoxy and methyl, or by such a substituted or unsubstituted phenyl radical and a sulfo, sulfato or carboxyl group, or by a heterocyclic radical,
Y is vinyl or β-sulfatoethyl, β-thiosulfatoethyl, β-phosphatoethyl, β-(C₂-C₅-alkanoyloxy)ethyl, β-benzoyloxyethyl, β-(sulfobenzoyloxy)ethyl or β-(p-toluene-sulfonyloxy)ethyl, and
z is 1 or 2, and
n is 1 or 2.

2. A dye as claimed in claim 1, wherein F is the radical of a sulfo-containing mono- or disazo dye.

3. A dye as claimed in claim 1, wherein F is the radical of a 1:1 copper complex monoazo or 1:1 copper complex disazo dye.

4. A dye as claimed in claim 1, wherein F is the radical of an anthraquinone dye.

5. A dye as claimed in claim 1, wherein F is the radical of a copper formazan dye.

6. A dye as claimed in claim 1, wherein F is the radical of a copper phthalocyanine or nickel phthalocyanine dye.

7. A dye as claimed in claim 1, wherein F is the radical of a triphendioxazine dye.

8. A dye as claimed in at least one of claims 1 to 7, wherein X is chlorine or fluorine.

9. A dye as claimed in at least one of claims 1 to 7, wherein X is cyanoamino.

10. A dye as claimed in at least one of claims 1 to 9, wherein W is n-propylene.

11. A dye as claimed in at least one of claims 1 to 10, wherein R is phenyl which may be substituted by 1 or 2 substituents from the group comprising methyl, methoxy and sulfo.

12. A dye as claimed in at least one of claims 1 to 11, wherein R^{A} is hydrogen.

13. A process for preparing a dye of the formula (1) of claim 1, which comprises reacting precursors typical of the particular dye, at least one of which contains a group of the formula (2), with one another or reacting a halotriazine compound of the formula (21) 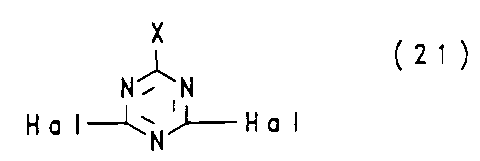 in which X has one of the meanings given in claim 1 and Hal is halogen, with an amino-containing compound of the formula (20) 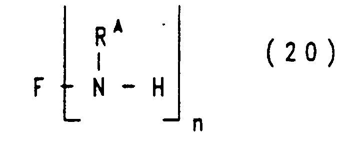 where F, R^{A} and n have the meanings given in claim 1 and an amino compound of the formula (22) 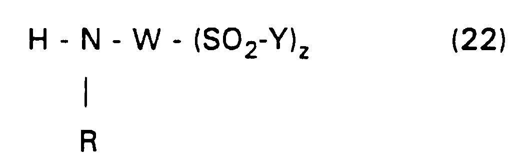 where R, W, Y and z have the meanings given in claim 1 in any desired order.

14. Use of a dye of claim 1 or of a dye prepared as claimed in claim 13 for dyeing (including printing) hydroxyl- and/or carboxamido-containing material, in particular fiber material.

15. A process for dyeing (including printing) hydroxyl- and/or carboxamido-containing material, in particular fiber material, in which a dye is applied to the material and the dye is fixed on the material by means of heat or by means of an alkaline agent or by means of both measures, wherein the dye used is a dye of claim 1 or a dye prepared as claimed in claim 13.

16. A compound of the formula (22A) 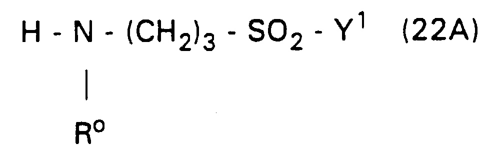 in which Y¹ is vinyl or β-sulfatoethyl, β-thiosulfatoethyl, β-phosphatoethyl, β-(C₂-C₅)-alkanoyloxyethyl, β-benzoyloxyethyl, β-(sulfobenzoyloxy)ethyl, β-(p-toluenesulfonyloxy)ethyl or β-hydroxyethyl and R^{o} is phenyl which may be substituted by 1, 2 or 3 substituents from the group comprising halogen, alkyl of 1 to 4 carbon atoms, alkoxy of 1 to 4 carbon atoms, sulfo and carboxyl.

17. Use of a compound of claim 16 as starting compound for synthesizing fiber-reactive compounds, in particular dyes.

## Revendications

1. Colorant de formule générale (1)
F - Zₙ (1)
dans laquelle
F représente le reste, contenant des groupes sulfo, d'un colorant mono-, dis- ou polyazoïque, ou d'un complexe de métal lourd-colorant mono-, dis- ou -trisazoïque, ou d'un colorant d'anthraquinone, d'azométhine, de phénazine, de stilbène, de triphénylméthane, de xanthène, de thioxanthène, de nitroaryle, de naphtoquinone, de pyrènequinone, de pérylène-tétracarbimide, de formazane, de formazane de cuivre, de phtalocyanine, de phtalocyanine de cuivre, de phtalocyanine de nickel ou de phtalocyanine de cobalt ou d'une triphéndioxazine ;
Z représente un groupe de formule générale (2) 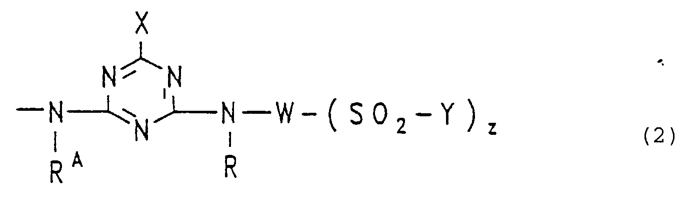 dans laquelle
R^{A} représente un atome d'hydrogène ou un groupe alkyle avec 1 à 4 atomes de carbone, qui peut être substitué par un atome d'halogène, des groupes hydroxy, cyano, alcoxy avec 1 à 4 atomes de carbone, alcoxycarbonyle avec 2 à 5 atomes de carbone, carboxy, sulfamoyle, sulfo, sulfato ou phosphato,
X représente un atome d'halogène, des groupes cyanamino, méthylsulfonylamino, éthylsulfonylamino, carboxypyridinyle, aminocarbonylpyridinyle ou un reste de formule -N(R)-W-(SO₂-Y)_{z}, avec R, W, Y et z ayant les significations données ci-après,
R représente phényle, qui peut être substitué par 1, 2 ou 3 substituants pris dans le groupe comportant des halogènes, des groupes alkyle avec 1 à 4 atomes de carbone, des groupes alcoxy avec 1 à 4 atomes de carbone, sulfo et carboxy,
W représente alkylène linéaire ou ramifié avec 3 à 8 atomes de carbone, qui peut être substitué par 1 ou 2 substituants pris dans le groupe comportant des groupes alcoxy avec 1 à 6 atomes de carbone, des atomes d'halogènes, des groupes alcoxycarbonyle avec 2 à 5 atomes de carbone, des groupes carboxy, sulfo et sulfato, ou par un reste phényle, ou par un reste phényle substitué par 1 ou 2 substituants pris dans le groupe comportant des groupes sulfo, carboxy, méthoxy, éthoxy et méthyle ou par un tel reste phényle éventuellement substitué et un groupe sulfo, sulfato ou carboxy, ou par un reste hétérocyclique,
Y représente un groupe vinyle ou β-sulfatoéthyle, β-thiosulfatoéthyle, β-phosphatoéthyle, β-(alcanoyl en C₂-C₅)oxyéthyle, β-benzoyloxyéthyle, β-(sulfobenzoyloxy)-éthyle ou β-(p-toluènesulfonyloxy)éthyle et
z vaut 1 ou 2, et
n vaut 1 ou 2.

2. Colorant selon la revendication 1, caractérisé en ce que F représente le reste d'un colorant mono- ou disazoïque contenant des groupes sulfo.

3. Colorant selon la revendication 1, caractérisé en ce que F représente le reste d'un complexe 1:1 de cuivre-colorant mono- ou disazoïque.

4. Colorant selon la revendication 1, caractérisé en ce que F est le reste d'un colorant d'anthraquinone.

5. Colorant selon la revendication 1, caractérisé en ce que F est le reste d'un colorant de formazane de cuivre.

6. Colorant selon la revendication 1, caractérisé en ce que F est le reste d'un colorant de phtalocyanine de cuivre ou de phtalocyanine de nickel.

7. Colorant selon la revendication 1, caractérisé en ce que F est reste d'un colorant de triphèndioxazine.

8. Colorant selon au moins l'une des revendications 1 à 7, caractérisé en ce que X représente le chlore ou le fluor.

9. Colorant selon au moins l'une des revendications 1 à 7, caractérisé en ce que X est le groupe cyanamino.

10. Colorant selon au moins l'une des revendications 1 à 9, caractérisé en ce que W est le n-propylène.

11. Colorant selon au moins l'une des revendications 1 à 10, caractérisé en ce que R représente un groupe phényle qui peut être substitué par un ou deux substituants pris dans le groupe comportant les groupes méthyle, méthoxy et sulfo.

12. Colorant selon au moins l'une des revendications 1 à 11, caractérisé en ce que R^{A} représente un atome d'hydrogène.

13. Procédé pour la préparation d'un colorant répondant à la formule générale (1) de la revendication 1, caractérisé en ce que l'on fait réagir, dans un ordre facultatif, les uns avec les autres, des précurseurs caractéristiques des colorants respectifs, dont au moins un comporte un groupe de formule générale (2), ou en ce que l'on fait réagir un composé d'halogeno-triazine de formule générale (21) 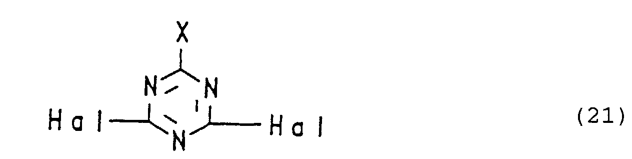 dans laquelle X a l'une des significations données dans la revendication 1 et Hal représente un atome d'halogène avec un composé contenant des groupes amino de formule générale (20) 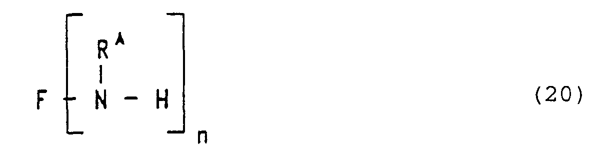 dans laquelle F, R^{A} et n ont les significations données dans la revendication 1, et avec un composé aminé de formule générale (22) 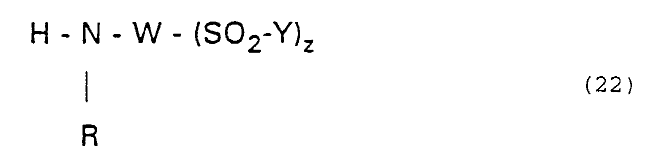 avec R, W, Y et z ayant les significations données dans la revendication 1.

14. Utilisation d'un colorant de la revendication 1 ou préparé selon la revendication 13, pour la teinture (y compris l'impression) de matière contenant des groupes hydroxy- et/ou carboxamido, plus particulièrement des matières fibreuses.

15. Procédé pour la teinture (y compris l'impression) de matière contenant des groupes hydroxy- et/ou carboxamido, plus particulièrement de matière fibreuse, dans lequel on dépose un colorant sur la matière et on fixe le colorant sur la matière par la chaleur ou à l'aide d'un agent à effet alcalin, ou à l'aide des deux mesures, caractérisé en ce que l'on utilise en tant que colorant un colorant de la revendication 1 ou un colorant préparé selon la revendication 13.

16. Composé répondant à la formule générale (22A) 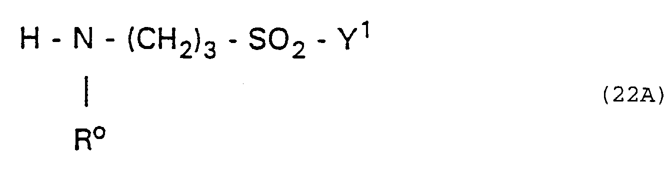 dans laquelle Y¹ représente un groupe vinyle ou β-sulfatoéthyle, β-thiosulfatoéthyle, β-phosphatoéthyle, β-(alcanoyl en C₁-C₅)oxy-éthyle, β-benzoyloxyéthyle, β-(sulfobenzoyloxy)-éthyle, β-(p-toluènesulfonyloxy)-éthyle, ou β-hydroxyéthyle et R^{o} est un groupe phényle, qui peut être substitué par 1, 2 ou 3 substituants pris dans le groupe comportant des atomes d'halogène, des groupes alkyle avec 1 à 4 atomes de carbone, alcoxy avec 1 à 4 atomes de carbone, sulfo et carboxy.

17. Utilisation d'un composé de la revendication 16, en tant que composé de départ pour la synthèse de composés réactifs sur les fibres, plus particulièrement de colorants.
